(19) Europäisches Patentamt / European Patent Office / Office européen des brevets

(11) **EP 4 202 388 A1**

(12) **EUROPEAN PATENT APPLICATION**
published in accordance with Art. 153(4) EPC

(43) Date of publication:
**28.06.2023 Bulletin 2023/26**

(21) Application number: **21872583.6**

(22) Date of filing: **24.09.2021**

(51) International Patent Classification (IPC):
***G01L 1/16*** *(2006.01)* ***H01L 41/087*** *(2006.01)*
***H01L 41/113*** *(2006.01)* ***H01L 41/193*** *(2006.01)*

(52) Cooperative Patent Classification (CPC):
**G01L 1/16; H10N 30/30; H10N 30/60; H10N 30/857**

(86) International application number:
**PCT/JP2021/035189**

(87) International publication number:
**WO 2022/065454 (31.03.2022 Gazette 2022/13)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **25.09.2020 JP 2020161350**

(71) Applicant: **Mitsui Chemicals, Inc.**
**Tokyo 104-0028 (JP)**

(72) Inventors:
- **MARUKO, Nobuhiro**
  **Nagoya-shi, Aichi 457-8522 (JP)**
- **YOSHIDA, Mitsunobu**
  **Nagoya-shi, Aichi 457-8522 (JP)**
- **OZAKI, Katsutoshi**
  **Nagoya-shi, Aichi 457-8522 (JP)**

(74) Representative: **Hoffmann Eitle**
**Patent- und Rechtsanwälte PartmbB**
**Arabellastraße 30**
**81925 München (DE)**

(54) **PIEZOELECTRIC DEVICE, FORCE SENSOR, AND BIOLOGICAL INFORMATION ACQUISITION DEVICE**

(57) A piezoelectric device includes a first piezoelectric sensor having a first inner conductor, a first piezoelectric body, and a first outer conductor, a second piezoelectric sensor having a second inner conductor, a second piezoelectric body, and a second outer conductor, and a differential signal forming unit having a pair of differential input terminals. When an external force acts on the first piezoelectric body, the first piezoelectric sensor generates a first voltage in the first inner conductor with respect to the first outer conductor according to displacement of the first piezoelectric body due to the external force. When the external force acts on the second piezoelectric body, the second piezoelectric sensor generates, in the second inner conductor with respect to the second outer conductor, a second voltage having a voltage different in polarity from the first voltage according to displacement of the second piezoelectric body due to the external force. The first inner conductor is electrically connected to one differential input terminal. The second inner conductor is electrically connected to the other differential input terminal. The differential signal forming unit forms a differential signal based on a first signal input to the one differential input terminal and a second signal input to the other differential input terminal.

FIG.1

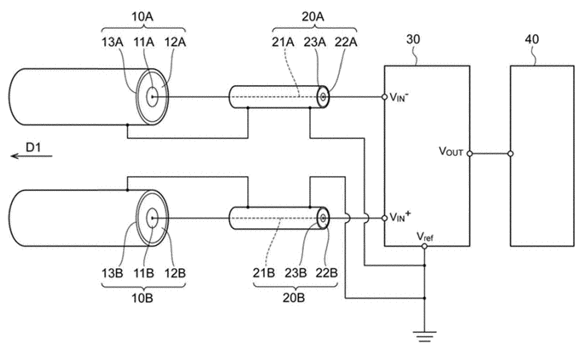


EP 4 202 388 A1

## Description

Technical Field

The present disclosure relates to a piezoelectric device, a force sensor, and a biological information acquisition device.

Background Art

In recent years, a material having piezoelectricity is used by coating a conductor.

Patent Document 1 discloses a wire harness. The wire harness disclosed in Patent Document 1 includes a piezo cable and an electrical connector. The piezo cable includes a central conductor, a piezoelectric material layer, an outer conductor, and a sheath. The central conductor, the piezoelectric material layer, the outer conductor, and the sheath are coaxially arranged in this order from a center toward the outside. As the piezoelectric material layer, a copolymer of polyvinylidene fluoride (PVDF) is used. The electrical connector incorporates an A/D conversion circuit.

Patent Document 1: Japanese Patent Application Laid-Open (JP-A) No. H10-132669

SUMMARY OF INVENTION

Technical Problem

However, in the piezo cable disclosed in Patent Document 1, noise may be superimposed on the outer conductor due to external electromagnetic noise or the like. Therefore, noise may also be superimposed on a voltage generated between the central conductor and the outer conductor. As a result, the piezo cable disclosed in Patent Document 1 may not be able to detect an external force such as fine vibration with high sensitivity.

In view of the above circumstances, an object of the present disclosure is to provide a piezoelectric device, a force sensor, and a biological information acquisition device capable of detecting an external force with high sensitivity.

Solution to Problem

Means for solving the above problems include the following embodiments.

< 1 > A piezoelectric device including:

a first piezoelectric sensor including a first inner conductor extending in a first direction, a first piezoelectric body covering at least a part of the first inner conductor, and a first outer conductor disposed at an outer periphery of the first piezoelectric body, the first piezoelectric sensor generating a first voltage in the first inner conductor with respect to the first outer conductor according to displacement of the first piezoelectric body due to an external force when the external force acts on the first piezoelectric body;
a second piezoelectric sensor including a second inner conductor extending in a second direction, a second piezoelectric body covering at least a part of the second inner conductor, and a second outer conductor disposed at an outer periphery of the second piezoelectric body, the second piezoelectric sensor generating a second voltage having a voltage different in polarity from the first voltage in the second inner conductor with respect to the second outer conductor according to displacement of the second piezoelectric body due to the external force when the external force acts on the second piezoelectric body; and
a differential signal forming unit including one differential input terminal to which the first inner conductor is electrically connected and another differential input terminal to which the second inner conductor is electrically connected, the differential signal forming unit forming a differential signal based on a first signal input to the one differential input terminal and a second signal input to the other differential input terminal.

< 2 > The piezoelectric device according to < 1 >, in which the differential signal forming unit includes a differential amplifier circuit.
< 3 > The piezoelectric device according to < 1 > or < 2 >, in which the first direction and the second direction are substantially parallel.
< 4 > The piezoelectric device according to < 3 >, in which the first outer conductor and the second outer conductor are in physical contact with each other.
< 5 > The piezoelectric device according to any one of < 1 > to < 4 >, in which

the first piezoelectric body is formed by spirally winding an elongated organic piezoelectric body including an organic piezoelectric material having a piezoelectric constant $d_{14}$ in a first spiral direction toward the first direction, and

the second piezoelectric body is formed by spirally winding the elongated organic piezoelectric body in a second spiral direction different from the first spiral direction toward the second direction.

< 6 > The piezoelectric device according to < 5 >, in which the organic piezoelectric material contains a helical chiral polymer having optical activity.

< 7 > The piezoelectric device according to < 6 >, in which the helical chiral polymer contains polylactic acid.

< 8 > The piezoelectric device according to < 5 >, in which the organic piezoelectric material contains long fibers composed of an optically active polypeptide.

< 9 > The piezoelectric device according to any one of < 1 > to < 4 >, in which

the first piezoelectric body is formed by winding a sheet-shaped piezoelectric body containing a piezoelectric material having a piezoelectric constant $d_{33}$ and a piezoelectric constant $d_{31}$ and not having a piezoelectric constant $d_{14}$ such that one surface of the sheet-shaped piezoelectric body is on a side of the first inner conductor, and

the second piezoelectric body is formed by winding the sheet-shaped piezoelectric body such that another surface of the sheet-shaped piezoelectric body is on a side of the second inner conductor.

< 10 > The piezoelectric device according to < 9 >, in which the sheet-shaped piezoelectric body contains polyvinylidene fluoride.

< 11 > A piezoelectric device including:

a first piezoelectric sensor including a first inner conductor extending in a first direction, a first piezoelectric body covering at least a part of the first inner conductor, and a first outer conductor disposed at an outer periphery of the first piezoelectric body, the first piezoelectric sensor generating a first voltage in the first inner conductor with respect to the first outer conductor according to displacement of the first piezoelectric body due to an external force when the external force acts on the first piezoelectric body;

a second piezoelectric sensor including a second inner conductor extending in a second direction, a second piezoelectric body covering at least a part of the second inner conductor, and a second outer conductor disposed at an outer periphery of the second piezoelectric body, the second piezoelectric sensor generating a second voltage having a voltage different in polarity from the first voltage in the second inner conductor with respect to the second outer conductor according to displacement of the second piezoelectric body due to the external force when the external force acts on the second piezoelectric body; and

an instrumentation amplifier including one differential input terminal to which the first inner conductor is electrically connected, another differential input terminal to which the second inner conductor is electrically connected, and a reference terminal electrically connected to each of the first outer conductor and the second outer conductor.

< 12 > A force sensor including the piezoelectric device according to any one of < 1 > to < 11 >.

< 13 > A biological information acquisition device including the piezoelectric device according to any one of < 1 > to < 11 >.

Advantageous Effects of Invention

According to the present disclosure, a piezoelectric device, a force sensor, and a biological information acquisition device capable of detecting an external force with high sensitivity are provided.

BRIEF DESCRIPTION OF DRAWINGS

Fig. 1 is a schematic configuration diagram of a piezoelectric device according to the present embodiment.

Fig. 2 is a block circuit diagram of an instrumentation amplifier according to the present embodiment.

Fig. 3 is a block diagram illustrating a hardware configuration of an information processing unit according to the present embodiment.

Fig. 4 is a block diagram illustrating an example of a functional configuration of a CPU according to the present embodiment.

Fig. 5 is a front view illustrating one aspect of a first piezoelectric sensor according to the present embodiment.
Fig. 6 is a cross-sectional view taken along line VI-VI of Fig. 5.
Fig. 7 is a front view illustrating another aspect of the first piezoelectric sensor according to the present embodiment.
Fig. 8 is a front view illustrating still another aspect of the first piezoelectric sensor according to the present embodiment.
Fig. 9 is a partially cutaway perspective view of one aspect of the first piezoelectric sensor and a second piezoelectric sensor according to the present embodiment.
Fig. 10 is a partially cutaway perspective view of another aspect of the first piezoelectric sensor 10A and the second piezoelectric sensor 10B according to the present embodiment.
Fig. 11 is a conceptual diagram of a force sensor according to the present embodiment.
Fig. 12 is a top view of a measurement device and a vibration generator of Example 1.
Fig. 13 is a cross-sectional view taken along section line XIII in Fig. 12.
Fig. 14 is a cross-sectional view taken along section line XIV in Fig. 12.
Fig. 15 is a side view of the measurement device 100 and the vibration generator 200 of Example 1 as viewed from a direction D4 in Fig. 12.
Fig. 16 is a measurement result of fast Fourier transform (FFT) analysis of Example 1.
Fig. 17 is a measurement result of FFT analysis of Comparative Example 1.
Fig. 18 is a measurement result of FFT analysis of Comparative Example 2.

DESCRIPTION OF EMBODIMENTS

An example of a preferred embodiment of the present disclosure will be described in detail. These descriptions and examples illustrate the embodiments and do not limit the scope of the embodiments.

In the present disclosure, a combination of two or more preferred aspects is a more preferred aspect.

In the present specification, a numerical range represented using "to" means a range including numerical values described before and after "to" as a lower limit value and an upper limit value.

In a numerical range described in stages in the present disclosure, an upper limit value or a lower limit value described in a certain numerical range may be replaced with an upper limit value or a lower limit value of another described numerical range in stages. Furthermore, in the numerical range described in the present disclosure, an upper limit value or a lower limit value described in a certain numerical range may be replaced with a value shown in Examples.

A piezoelectric device according to a first aspect of the present disclosure includes a first piezoelectric sensor, a second piezoelectric sensor, and a differential signal forming unit.

The first piezoelectric sensor includes a first inner conductor extending in a first direction, a first piezoelectric body covering at least a part of the first inner conductor, and a first outer conductor disposed at an outer periphery of the first piezoelectric body. When an external force acts on the first piezoelectric body, a first voltage is generated in the first inner conductor with respect to the first outer conductor according to displacement of the first piezoelectric body by the external force.

The second piezoelectric sensor includes a second inner conductor extending in a second direction, a second piezoelectric body covering at least a part of the second inner conductor, and a second outer conductor disposed at an outer periphery of the second piezoelectric body. When the external force acts on the second piezoelectric body, a second voltage having a voltage different in polarity from the first voltage is generated in the second inner conductor with respect to the second outer conductor according to displacement of the second piezoelectric body by the external force.

The differential signal forming unit includes one differential input terminal to which the first inner conductor is electrically connected and the other differential input terminal to which the second inner conductor is electrically connected. The differential signal forming unit forms a differential signal based on a first signal input to the one differential input terminal and a second signal input to the other differential input terminal.

In the first aspect, the types of the first signal, the second signal, and the differential signal are the same, and may be, for example, a voltage, a current, or a charge amount.

Each of the first voltage and the second voltage is likely to include common mode noise due to external electromagnetic noise or the like. The noise in the common mode may be a factor that lowers the sensitivity of the external force detected by the piezoelectric device according to the first aspect.

The differential signal forming unit forms a differential signal obtained by differentiating the first signal and the second signal. That is, in the differential signal, noise in the common mode of the first signal and the second signal is reduced. As a result, the piezoelectric device according to the first aspect can detect the external force with high sensitivity based on the differential signal.

In the first aspect, examples of the first piezoelectric sensor include the same ones as those exemplified as a first piezoelectric sensor 10A described later. Examples of the second piezoelectric sensor include the same ones as

those exemplified as a second piezoelectric sensor 10B described later.

Hereinafter, detailed description of constituent members of the first piezoelectric sensor, constituent members of the second piezoelectric sensor, and the like in the first aspect will be omitted.

The differential signal forming unit forms a differential signal based on the first signal and the second signal. The differential signal may be formed by at least one of a differential circuit (hardware) and software of digital calculation processing. The differential signal forming unit has a known configuration.

In a case in which the differential signal is formed by the differential circuit, the differential signal forming unit may include, for example, an operational amplifier in addition to a pair of differential input terminals. Specific examples of the differential signal forming unit include a differential amplifier circuit using an operational amplifier, a differential amplifier circuit using a plurality of operational amplifiers and a buffer amplifier, and an instrumentation amplifier.

In a case in which the differential signal is formed by software of digital calculation processing, the differential signal forming unit includes, for example, a digital to analog (AD) converter and an information processing device in addition to the pair of differential input terminals. The information processing device is disposed at a subsequent stage of the AD converter.

The AD converter converts each of the input first signal and second signal into a digital signal and outputs the digital signal to the information processing device. The information processing device performs digital calculation processing on the input pair of digital signals to form a differential signal.

The information processing device executes a command of a program which is software for realizing each function. The information processing device includes a storage unit and a control unit.

The storage unit stores various data. The storage unit includes a main storage unit such as a read only memory (ROM) and a random access memory (RAM), and an auxiliary storage unit. The main storage unit includes a semiconductor memory. The auxiliary storage unit includes a solid state drive (SSD). The storage unit stores various programs executed by the control unit. The program includes a program for performing digital calculation processing on the pair of digital signals, firmware, and a control program.

The control unit is a hardware circuit including a processor such as a central processing unit (CPU). The control unit executes digital calculation processing or the like by executing a program stored in the storage unit.

In the first aspect, the differential signal forming unit preferably includes a differential amplifier circuit. In this case, each of the first signal and the second signal is an analog signal. As a result, the differential amplifier circuit can amplify an output from each of the first piezoelectric sensor and the second piezoelectric sensor as an analog signal and can form a differential signal. Therefore, the external noise is hardly affected by circuit noise or the like. As a result, the differential amplifier circuit can more accurately signal-process signals detected by the first piezoelectric sensor and the second piezoelectric sensor.

In the first aspect, the first direction and the second direction are preferably substantially parallel. In other words, the first piezoelectric sensor and the second piezoelectric sensor are preferably disposed substantially in parallel.

In the present disclosure, "substantially parallel" means that the first piezoelectric sensor and the second piezoelectric sensor can be regarded as being parallel at a glance. Specifically, "substantially parallel" means that an angle formed by the first piezoelectric sensor and the second piezoelectric sensor is less than 10 degrees.

As a result, when an external force acts on the piezoelectric device, substantially the same external force is more likely to act on each of the first piezoelectric body and the second piezoelectric body than in a case in which the first piezoelectric sensor and the second piezoelectric sensor are not disposed substantially in parallel. As a result, the piezoelectric device according to the first aspect can detect the external force with higher sensitivity.

In the first aspect, it is preferable that the first outer conductor and the second outer conductor are in physical contact with each other.

As a result, when an external force acts on the piezoelectric device, substantially the same external force is more likely to act on each of the first piezoelectric body and the second piezoelectric body than in a case in which the first outer conductor and the second outer conductor are not in physical contact with each other. The first outer conductor and the second outer conductor are short-circuited. As a result, the piezoelectric device according to the first aspect can detect the external force with higher sensitivity.

In a first aspect, it is preferable that the first piezoelectric body is formed by spirally winding an elongated organic piezoelectric body containing an organic piezoelectric material having a piezoelectric constant $d_{14}$ in a first spiral direction toward the first direction, and the second piezoelectric body is formed by spirally winding the elongated organic piezoelectric body in a second spiral direction different from the first spiral direction toward the second direction.

As a result, an optically active polymer (A) having excellent piezoelectricity and the like can be used as a material of each of the first piezoelectric body of the first piezoelectric sensor and the second piezoelectric body of the second piezoelectric sensor.

The piezoelectricity (piezo) indicates a property of generating a charge when stress is applied.

Details of the optically active polymer (A) will be described later.

In the first aspect, the organic piezoelectric material preferably contains a helical chiral polymer having optical

activity.

As a result, workability, availability, and the like of the organic piezoelectric material are more excellent, and the piezoelectricity of each of the first piezoelectric sensor and the second piezoelectric sensor is more excellent.

In the first aspect, the helical chiral polymer preferably contains polylactic acid.

As a result, workability, availability, and the like of the organic piezoelectric material are further excellent, and the piezoelectricity of each of the first piezoelectric sensor and the second piezoelectric sensor is further excellent.

In the first aspect, the organic piezoelectric material preferably contains long fibers composed of an optically active polypeptide.

As a result, the piezoelectricity of each of the first piezoelectric sensor and the second piezoelectric sensor is particularly excellent.

In the first aspect, it is preferable that the first piezoelectric body is formed by winding a sheet-shaped piezoelectric body including a piezoelectric material having a piezoelectric constant $d_{33}$ and a piezoelectric constant $d_{31}$ and not having a piezoelectric constant $d_{14}$ such that one surface of the sheet-shaped piezoelectric body is on a side of the first inner conductor, and the second piezoelectric body is formed by winding the sheet-shaped piezoelectric body such that the other surface of the sheet-shaped piezoelectric body is on a side of the second inner conductor.

Accordingly, as a material of each of the first piezoelectric body of the first piezoelectric sensor and the second piezoelectric body of the second piezoelectric sensor, an organic piezoelectric material such as polyvinylidene fluoride (PVDF) and an inorganic piezoelectric material such as lead zirconate titanate (PZT), which are excellent in piezoelectricity and the like, can be used.

Details of the organic piezoelectric material such as polyvinylidene fluoride (PVDF) and the inorganic piezoelectric material such as lead zirconate titanate (PZT) will be described later.

In the first aspect, the sheet-shaped piezoelectric body preferably contains polyvinylidene fluoride. As a result, the sheet-shaped piezoelectric body is lightweight, rich in flexibility, and excellent in workability and the like.

A piezoelectric device according to a second aspect of the present disclosure may include a first piezoelectric sensor, a second piezoelectric sensor, and an instrumentation amplifier.

The first piezoelectric sensor includes a first inner conductor extending in a first direction, a first piezoelectric body covering at least a part of the first inner conductor, and a first outer conductor disposed at an outer periphery of the first piezoelectric body. When an external force acts on the first piezoelectric body, a first voltage is generated in the first inner conductor with respect to the first outer conductor according to displacement of the first piezoelectric body by the external force.

The second piezoelectric sensor includes a second inner conductor extending in a second direction, a second piezoelectric body covering at least a part of the second inner conductor, and a second outer conductor disposed at an outer periphery of the second piezoelectric body. When the external force acts on the second piezoelectric body, a second voltage having a voltage different in polarity from the first voltage is generated in the second inner conductor with respect to the second outer conductor according to displacement of the second piezoelectric body by the external force.

The instrumentation amplifier includes one differential input terminal to which the first inner conductor is electrically connected, the other differential input terminal to which the second inner conductor is electrically connected, and a reference terminal electrically connected to each of the first outer conductor and the second outer conductor.

Each of the first voltage and the second voltage easily includes a common mode voltage due to external electromagnetic noise or the like. The common mode voltage may be a factor that lowers the sensitivity of the external force detected by the piezoelectric device according to the second aspect. The instrumentation amplifier outputs a differential voltage obtained by differentially amplifying the first voltage and the second voltage with reference to a voltage of the reference terminal. That is, in the differential voltage output from the instrumentation amplifier, the common mode voltage of the first voltage and the second voltage is reduced and amplified. In other words, the differential voltage has an excellent SN ratio. As a result, the piezoelectric device according to the second aspect can detect the external force with high sensitivity based on the differential voltage.

Details of the piezoelectric device according to the second aspect will be described later with reference to Figs. 1 to 10.

A force sensor of the present disclosure includes the piezoelectric device according to the first aspect or the piezoelectric device according to the second aspect.

An example of the force sensor of the present disclosure will be described later with reference to Figs. 1 to 11.

A biological information acquisition device of the present disclosure includes the piezoelectric device according to the first aspect or the piezoelectric device according to the second aspect.

An example of the biological information acquisition device of the present disclosure will be described later with reference to Figs. 1 to 10.

Hereinafter, embodiments of a piezoelectric device, a force sensor, and a biological information acquisition device according to embodiments of the present disclosure using an instrumentation amplifier will be described with

reference to the drawings. In the drawings, the same or corresponding parts are denoted by the same reference signs, and the description thereof will not be repeated.

[Piezoelectric Device]

A piezoelectric device 1 according to an embodiment of the present disclosure will be described with reference to Fig. 1. Fig. 1 is a schematic configuration diagram of the piezoelectric device 1 according to an embodiment of the present disclosure.

As illustrated in Fig. 1, the piezoelectric device 1 includes a first piezoelectric sensor 10A, a second piezoelectric sensor 10B, a first coaxial cable 20A, a second coaxial cable 20B, an instrumentation amplifier 30, and an information processing unit 40. The first piezoelectric sensor 10A is electrically connected to the instrumentation amplifier 30 via the first coaxial cable 20A. The second piezoelectric sensor 10B is electrically connected to the instrumentation amplifier 30 via the second coaxial cable 20B. The instrumentation amplifier 30 is electrically connected to the information processing unit 40.

The first piezoelectric sensor 10A includes a first inner conductor 11A, a first piezoelectric body 12A, and a first outer conductor 13A. The first piezoelectric body 12A is located between the first inner conductor 11A and the first outer conductor 13A. The first inner conductor 11A and the first outer conductor 13A are not in physical contact with each other. The configuration of the first piezoelectric sensor 10A will be described later in detail with reference to Figs. 5 to 8.

When an external force acts on the first piezoelectric body 12A, the first piezoelectric sensor 10A generates a first voltage between the first inner conductor 11A and the first outer conductor 13A by displacement of the first piezoelectric body 12A due to the external force. The first voltage indicates a potential difference of the first inner conductor 1 1A with respect to the first outer conductor 13A.

The external force includes tension, pressurization, and bending. The displacement of the first piezoelectric body 12A includes restorable deformation (Hereinafter, the deformation is referred to as "non-plastic deformation".) of the first piezoelectric body 12A according to the external force. The non-plastic deformation of the first piezoelectric body 12A includes partial or total elongation and compression of the first piezoelectric body 12A.

The second piezoelectric sensor 10B includes a second inner conductor 11B, a second piezoelectric body 12B, and a second outer conductor 13B. The second piezoelectric body 12B is located between the second inner conductor 11B and the second outer conductor 13B. The second inner conductor 11B and the second outer conductor 13B are not in physical contact with each other. Details of the configuration of the second piezoelectric sensor 10B will be described later.

When an external force acts on the second piezoelectric body 12B, the second piezoelectric sensor 10B generates a second voltage between the second inner conductor 11B and the second outer conductor 13B by displacement of the second piezoelectric body 12B due to the external force. The second voltage indicates a potential difference of the second inner conductor 11B with respect to the second outer conductor 13B. The second voltage and the first voltage are different in polarity.

The displacement of the second piezoelectric body 12B includes non-plastic deformation of the second piezoelectric body 12B according to the external force. The non-plastic deformation of the second piezoelectric body 12B includes partial or total elongation and compression of the second piezoelectric body 12B.

In the present embodiment, each of the first inner conductor 11A and the second inner conductor 11B extends in a direction D1. That is, the first inner conductor 11A and the second inner conductor 11B are substantially parallel to each other. In other words, the first piezoelectric sensor 10A and the second piezoelectric sensor 10B are disposed substantially in parallel. The first piezoelectric sensor 10A and the second piezoelectric sensor 10B are preferably in physical contact with each other. The direction D1 is an example of a first direction and a second direction.

Substantially parallel is a relationship in which the first piezoelectric sensor 10A and the second piezoelectric sensor 10B can be regarded as being parallel at a glance. Specifically, "substantially parallel" means that an angle formed by the first piezoelectric sensor 10A and the second piezoelectric sensor 10B is less than 10 degrees.

The instrumentation amplifier 30 amplifies a potential difference between the first voltage and the second voltage to be input, and outputs the obtained differential voltage as a single output. The instrumentation amplifier 30 includes a first differential input terminal $V_{IN}^-$, a second differential input terminal $V_{IN}^+$, a reference terminal $V_{ref}$, and an output terminal $V_{out}$. Details of the configuration of the instrumentation amplifier 30 will be described later with reference to Fig. 2. The first differential input terminal $V_{IN}$- is an example of one differential input terminal. The second differential input terminal $V_{IN}^+$ is an example of the other differential input terminal.

The first coaxial cable 20A includes a first conductive wire 21A, a first insulating layer 22A, and a first conductor layer 23A. The first conductive wire 21A electrically connects the first inner conductor 11A of the first piezoelectric sensor 10A and the first differential input terminal $V_{IN}^-$ of the instrumentation amplifier 30. The first insulating layer 22A covers the first conductive wire 21A. That is, the first conductive wire 21A and the first conductor layer 23A are not in physical contact with each other. The first conductor layer 23A electrically connects the first outer conductor 13A of the first

piezoelectric sensor 10A and the reference terminal $V_{ref}$ of the instrumentation amplifier 30. The first conductor layer 23A covers the first insulating layer 22A.

Examples of a material of the first conductive wire 21A include copper and the like. A material of the first insulating layer 22A may be any material having electrical insulation properties, and examples thereof include a fluororesin, polyethylene, polypropylene, an amorphous polyolefin resin, and polyethylene naphthalate. Examples of a material of the first conductor layer 23A include copper, aluminum, and the like. The first conductor layer 23A may be a braided wire. The first conductor layer 23A may be covered with a protective film. Examples of a material of the protective film include vinyl chloride resin, polyethylene terephthalate, and polyimide.

The second coaxial cable 20B includes a second conductive wire 21B, a second insulating layer 22B, and a second conductor layer 23B. The second conductive wire 21B electrically connects the second inner conductor 11B of the second piezoelectric sensor 10B and the second differential input terminal $V_{IN}^+$ of the instrumentation amplifier 30. The second insulating layer 22B covers the second conductive wire 21B. That is, the second conductive wire 21B and the second conductor layer 23B are not in physical contact with each other. The second conductor layer 23B electrically connects the second outer conductor 13B of the second piezoelectric sensor 10B and the reference terminal $V_{ref}$ of the instrumentation amplifier 30. The second conductor layer 23B covers the second insulating layer 22B.

Examples of a material of the second conductive wire 21B include the same material as the material exemplified as the material of the first conductive wire 21B. Examples of a material of the second insulating layer 22B include the same material as the material exemplified as the material of the first insulating layer 22A. A material of the second conductor layer 23B is the same as the material exemplified as the material of the first conductor layer 23A. The second conductor layer 23B may be covered with a protective film. The configuration of the second coaxial cable 20B may be the same as or different from that of the first coaxial cable 20A.

The information processing unit 40 converts the differential voltage output from the instrumentation amplifier 30 into a digital signal and performs data processing. The data processing includes display of the differential voltage, detection of the external force, recording of the differential voltage, and the like. Details of the information processing unit 40 will be described later with reference to Figs. 3 and 4.

In the present embodiment, the first inner conductor 11A and the second inner conductor 11B are substantially parallel, but the present disclosure is not limited thereto, and the first inner conductor 11A and the second inner conductor 11B may not be substantially parallel. In the present embodiment, the piezoelectric device 1 includes the first coaxial cable 20A and the second coaxial cable 20B, but the present disclosure is not limited thereto, and the piezoelectric device 1 may not include the first coaxial cable 20A and the second coaxial cable 20B. In the present embodiment, the piezoelectric device 1 includes the information processing unit 40, but the present disclosure is not limited thereto, and the piezoelectric device 1 may not include the information processing unit 40.

(Instrumentation Amplifier)

Next, the instrumentation amplifier 30 will be further described with reference to Fig. 2. Fig. 2 is a block circuit diagram of the instrumentation amplifier 30 according to the present embodiment.

As illustrated in Fig. 2, the instrumentation amplifier 30 includes a first amplifier 31, a second amplifier 32, a third amplifier 33, and resistors $R_1$ to $R_7$. The first amplifier 31 and the second amplifier 32 constitute a pair of input stages. The third amplifier 33 amplifies a difference in voltage output from each of the first amplifier 31 and the second amplifier 32.

The first amplifier 31 includes a first non-inverting differential input terminal $I_1$, a first inverting differential input terminal $I_2$, and a first output terminal $O_1$. The first non-inverting differential input terminal $I_1$ is connected to the first differential input terminal $V_{IN}^-$ of the instrumentation amplifier 30. Therefore, the first inner conductor 11A of the first piezoelectric sensor 10A is electrically connected to the first non-inverting differential input terminal $I_1$. Therefore, the first voltage is input to the first non-inverting differential input terminal $I_1$.

The second amplifier 32 includes a second non-inverting differential input terminal $I_3$, a second inverting differential input terminal $I_4$, and a second output terminal $O_2$. The second non-inverting differential input terminal $I_3$ is connected to the second differential input terminal $V_{IN}^+$ of the instrumentation amplifier 30. Therefore, the second inner conductor 11B of the second piezoelectric sensor 10B is electrically connected to the second non-inverting differential input terminal $I_3$. Therefore, the second voltage is input to the second non-inverting differential input terminal $I_3$.

The output terminal $O_1$ of the first amplifier 31 and the output terminal $O_2$ of the second amplifier 32 are connected via each of the resistors $R_2$, $R_1$, and $R_3$. The inverting differential input terminal $I_2$ of the first amplifier 31 is connected between the resistor $R_2$ and the resistor $R_1$. The inverting differential input terminal $I_4$ of the second amplifier 32 is connected between the resistor $R_1$ and the resistor $R_3$.

The third amplifier 33 includes a non-inverting differential input terminal $I_5$, an inverting differential input terminal $I_6$, and an output terminal $O_3$. The non-inverting differential input terminal $I_5$ is connected to the output terminal $O_1$ of the first amplifier 31 via the resistor $R_4$, and is connected to the output terminal $O_3$ via the resistor $R_6$. The inverting

differential input terminal $I_6$ is connected to the output terminal $O_2$ of the second amplifier 32 via the resistor $R_5$, and is connected to the reference terminal $V_{ref}$ of the instrumentation amplifier 30 via the resistor $R_7$. That is, the reference terminal $V_{ref}$ is electrically connected to each of the first outer conductor 13A of the first piezoelectric sensor 10A and the second outer conductor 13B of the second piezoelectric sensor 10B, and is grounded. A voltage of the reference terminal $V_{ref}$ is preferably 0 V The output terminal $O_3$ is connected to the output terminal $V_{OUT}$ of the instrumentation amplifier 30.

In Fig. 2, a voltage of each of the first differential input terminal $V_{IN}^-$, the second differential input terminal $V_{IN}^+$, and the output terminal $V_{OUT}$ is based on the voltage of the reference terminal $V_{ref}$.

In this manner, in the instrumentation amplifier 30, the first amplifier 31 and the second amplifier 32 take a voltage difference between the first voltage and the second voltage. Next, the third amplifier 33 amplifies the voltage difference between the first voltage and the second voltage, and outputs the obtained differential voltage.

(Information Processing Unit)

Next, the information processing unit 40 will be described with reference to Fig. 3. Fig. 3 is a block diagram illustrating a hardware configuration of the information processing unit 40 according to the present embodiment. Fig. 4 is a block diagram illustrating an example of a functional configuration of a CPU 421 according to the present embodiment.

As illustrated in Fig. 3, the information processing unit 40 includes an AD converter 41, a PC 42, a monitor 43, and a speaker 44. Each of the AD converter 41, the monitor 43, and the speaker 44 is electrically connected to the PC 42. The AD converter 41 converts a differential voltage, which is an analog signal, into a digital signal. The PC 42 detects the digital signal converted by the AD converter 41.

The PC 42 includes a central processing unit (CPU) 421, a read only memory (ROM) 422, a random access memory (RAM) 423, a storage 424, a communication interface (I/F) 425, and an input/output I/F 426. The CPU 421, the ROM 422, the RAM 423, the storage 424, the communication I/F 425, and the input/output I/F 426 are communicably connected to each other via a bus 427.

The CPU 421 is a central processing unit. The CPU 421 executes various programs. The CPU 421 controls each unit. That is, the CPU 421 reads a program from the ROM 422 or the storage 424, and executes the program using the RAM 423 as a work area. In the present embodiment, an execution program for executing various processing is stored in the storage 424. The CPU 421 functions as a detection unit 4211, a determination unit 4212, and a notification unit 4213 illustrated in Fig. 4 by executing the execution program.

The ROM 422 stores various programs and various data. The RAM 422 temporarily stores programs or data as a work area. The storage 424 as a storage unit includes a hard disk drive (HDD), a solid state drive (SSD), or the like, and stores various programs including an operating system and various data.

The communication I/F 425 is an interface for communicating with a mobile terminal such as a smartphone. As the communication I/F 425, for example, standards such as ETHERNET (registered trademark), a fiber-distributed data interface (FDDI), and WI-FI (registered trademark) are used.

The input/output I/F 426 is an interface for communicating with each device constituting the information processing unit 40. The AD converter 41, the monitor 43, and the speaker 44 are connected to the PC 42 of the present embodiment via the input/output I/F 426.

As illustrated in Fig. 4, the CPU 421 includes the detection unit 4211, the determination unit 4212, and the notification unit 4213. Each functional configuration is realized by the CPU 421 reading the execution program stored in the storage 424 and executing the execution program.

The detection unit 4211 has a function of detecting a digital signal output from the AD converter 41 via the communication I/F 425. The detection unit 4211 digitizes the detected digital signal. As a result, a user can know how much external force has acted on the first piezoelectric sensor 10A and the second piezoelectric sensor 10B.

The determination unit 4212 has a function of determining whether or not a predetermined external force has acted on the first piezoelectric sensor 10A and the second piezoelectric sensor 10B by comparing a predetermined value with the digitized digital signal. The predetermined value is stored in the ROM 422. For example, the determination unit 4212 determines whether or not the digitized digital signal is greater than or equal to the predetermined value. In the case of determining that the quantified digital signal is greater than or equal to the predetermined value, the determination unit 4212 determines that the predetermined external force has acted on the first piezoelectric sensor 10A and the second piezoelectric sensor 10B. In the case of determining that the digitized digital signal is not greater than or equal to the predetermined value, the determination unit 4212 determines that the predetermined external force does not act on the first piezoelectric sensor 10A and the second piezoelectric sensor 10B.

The notification unit 4213 has a function of notifying the detected digital signal. For example, the notification unit 57 outputs character information related to a determination result to the monitor 43 via the communication I/F 425. The notification unit 4213 outputs voice information related to the determination result to the speaker 44.

(Operation of Piezoelectric Device)

As described with reference to Figs. 1 to 4, the piezoelectric device 1 according to the present embodiment includes the first piezoelectric sensor 10A, the second piezoelectric sensor 10B, and the instrumentation amplifier 30. The first piezoelectric sensor 10A includes a first inner conductor 11A, a first piezoelectric body 12A, and a first outer conductor 13A. The first piezoelectric sensor 10A generates the first voltage when the external force acts on the first piezoelectric body 12A. The second piezoelectric sensor 10B includes a second inner conductor 11B, a second piezoelectric body 12B, and a second outer conductor 13B. When the external force acts on the second piezoelectric body 12B, the second piezoelectric sensor 10B generates the second voltage with different polarity voltage from first voltage. The instrumentation amplifier 30 has the pair of differential input terminals $V_{IN}^-$ and $V_{IN}^+$ and the reference terminal $V_{REF}$. The first inner conductor 11A is electrically connected to one differential input terminal $V_{IN}^-$. The second inner conductor 11B is electrically connected to the other differential input terminal $V_{IN}^+$. The reference terminal $V_{REF}$ is electrically connected to each of the first outer conductor 13A and the second outer conductor 13B.

Each of the first voltage and the second voltage easily includes a common mode voltage due to external electromagnetic noise or the like. The common mode voltage may be a factor of lowering the sensitivity of the external force detected by the piezoelectric device 1. The instrumentation amplifier 30 outputs a differential voltage obtained by differentially amplifying the first voltage and the second voltage with reference to the voltage of the reference terminal $V_{REF}$. That is, in the differential voltage output from the instrumentation amplifier 30, the common mode voltage of the first voltage and the second voltage is reduced and amplified. In other words, the differential voltage has an excellent SN ratio. As a result, the piezoelectric device 1 can detect the external force with high sensitivity based on the differential voltage.

As described with reference to Figs. 1 to 4, in the present embodiment, the first inner conductor 11A extends in the direction D1, and the second inner conductor 11B extends in the direction D1. Therefore, the first inner conductor 11A and the second inner conductor 11B are substantially parallel.

In other words, the first piezoelectric sensor 10A and the second piezoelectric sensor 10B are disposed substantially in parallel. As a result, when an external force acts on the piezoelectric device 1, substantially the same external force is more likely to act on each of the first piezoelectric body 12A and the second piezoelectric body 12B than in a case in which the first piezoelectric sensor 10A and the second piezoelectric sensor 10B are not disposed substantially in parallel. As a result, the piezoelectric device 1 can detect the external force with high sensitivity.

As described with reference to Figs. 1 to 4, in the present embodiment, the first outer conductor 13A and the second outer conductor 13B are preferably in physical contact with each other.

As a result, when an external force acts on the piezoelectric device 1, substantially the same external force is more likely to act on each of the first piezoelectric body 12A and the second piezoelectric body 12B than in a case in which the first outer conductor 13A and the second outer conductor 13B are not in physical contact with each other. The first outer conductor 13A and the second outer conductor 13B are short-circuited. As a result, the piezoelectric device 1 can detect the external force with higher sensitivity.

(First Piezoelectric Sensor)

Next, details of the first piezoelectric sensor 10A will be described with reference to Figs. 1 and 5 to 8. Fig. 5 is a front view illustrating an aspect of the first piezoelectric sensor 10A according to the present embodiment. Fig. 6 is a cross-sectional view taken along line VI-VI of Fig. 5. Fig. 7 is a front view illustrating another aspect of the first piezoelectric sensor 10A according to the present embodiment. Fig. 8 is a front view illustrating still another aspect of the first piezoelectric sensor 10A according to the present embodiment. In Figs. 5, 7, and 8, the first outer conductor 13A is omitted.

As illustrated in Fig. 1, the first piezoelectric sensor 10A includes the first inner conductor 11A, the first piezoelectric body 12A, and the first outer conductor 13A. The first inner conductor 11A extends in the direction D1. The first piezoelectric body 12A covers at least a part of the first inner conductor 11A. The first outer conductor 13A is disposed at an outer periphery of the first piezoelectric body 12A.

The first piezoelectric sensor 10A is a linear object. A cross-sectional shape of the first piezoelectric sensor 10A in a plane orthogonal to the direction D1 is appropriately adjusted according to the application of the piezoelectric device 1 and the like, and examples thereof include a circular shape, an elliptical shape, a rectangular shape, a cocoon shape, a four-leaf shape, a star shape, and an irregular shape. In a case in which the cross-sectional shape of the first piezoelectric sensor 10A is a circular shape, a diameter of the first piezoelectric sensor 10A is preferably from 0.1 mm to 10 mm. A length of the first piezoelectric sensor 10A in the direction D1 is appropriately adjusted according to the application of the piezoelectric device 1 or the like, and is, for example, from 1 mm to 100 mm.

< First Inner Conductor >

The first inner conductor 11A is a conductor for efficiently detecting an electrical signal from the first piezoelectric sensor 10A.

The first inner conductor 11A is preferably an electrically good conductor, and examples thereof include a copper wire, an aluminum wire, a steel use stainless (SUS) wire, a metal wire coated with an insulating film, carbon fiber, a resin fiber integrated with carbon fiber, a tinsel wire, and an organic conductive material. The tinsel wire is formed by spirally winding a copper foil around a fiber. An outer diameter of the fiber is appropriately adjusted according to desired characteristics of the first piezoelectric sensor 10A, and is preferably from 0.1 mm to 10 mm. In particular, the first inner conductor 11A is preferably a tinsel wire or carbon fiber from the viewpoint of improving the piezoelectric sensitivity and the stability of the piezoelectric output and imparting high flexibility, and more preferably a tinsel wire from the viewpoint of low electrical resistance.

< First Outer Conductor >

The first outer conductor 13A is a conductor paired with the first inner conductor 11A in order to detect an electrical signal from the first piezoelectric sensor 10A.

The first outer conductor 13A may be disposed at an outer periphery of the first piezoelectric body 12A, and may cover at least a part of the first piezoelectric body 12A. Specifically, the first outer conductor 13A may cover a part of an outer peripheral surface of the first piezoelectric body 12A, or may cover the entire outer peripheral surface of the first piezoelectric body 12A.

The first outer conductor 13A is formed by winding an elongated conductor, for example.

Examples of a cross-sectional shape of the elongated conductor include a circular shape, an elliptical shape, a rectangular shape, and an irregular shape. In particular, the cross-sectional shape of the elongated conductor is preferably a rectangular shape from the viewpoint of being in close contact with the first piezoelectric body 12A in a plane and efficiently generating the first voltage.

A material of the elongated conductor is not particularly limited, and the following materials are mainly exemplified depending on the cross-sectional shape.

Examples of the elongated conductor having a rectangular cross section include a copper foil ribbon obtained by rolling a copper wire having a circular cross section and processing the copper wire into a flat plate shape, and an aluminum foil ribbon.

Examples of the elongated conductor having a circular cross section include a copper wire, an aluminum wire, an SUS wire, a metal wire coated with an insulating film, a carbon fiber, a resin fiber integrated with a carbon fiber, and a tinsel wire in which a copper foil is spirally wound around a fiber.

As the elongated conductor, a conductor obtained by coating an organic conductive material with an insulating material may be used.

Examples of a method of winding the elongated conductor include a method in which a copper foil or the like is spirally wound around the first piezoelectric body 12A, a method in which a copper wire or the like is formed into a tubular braid and the first piezoelectric body 12Ais wrapped, and a method in which the first piezoelectric body 12Ais cylindrically wrapped.

< First Piezoelectric Body >

In the first piezoelectric body 12A, when an external force acts, the first voltage is generated between the first inner conductor 11A and the first outer conductor 13A.

The first piezoelectric body 12A may cover at least a part of the first inner conductor 11A, may cover a part of the outer peripheral surface of the first inner conductor 11A, or may cover the entire outer peripheral surface of the first inner conductor 11A.

A configuration of the first piezoelectric body 12A may be, for example, the following first configuration A, second configuration A, third configuration A, or fourth configuration A.

In the first configuration A, as illustrated in Fig. 5, the first piezoelectric body 12A is formed by winding a first elongated organic piezoelectric body 121. The first elongated organic piezoelectric body 121 will be described later.

In the second configuration A, as illustrated in Fig. 7, the first piezoelectric body 12A is formed by winding the first elongated organic piezoelectric body 121 and a second elongated organic piezoelectric body 122. The second elongated organic piezoelectric body 122 will be described later.

In the third configuration A, the first piezoelectric body 12A is formed by winding a braid structure as illustrated in Fig. 8. The braid structure is formed by alternately crossing the first elongated organic piezoelectric body 121 and the second elongated organic piezoelectric body 122.

In the fourth configuration A, the sheet-shaped piezoelectric body is wound. The sheet-shaped piezoelectric body will be described later.
Hereinafter, the first elongated organic piezoelectric body 121, the first configuration A, the second elongated organic piezoelectric body 122, the second configuration A, the third configuration A, the sheet-shaped piezoelectric body, and the fourth configuration A will be described in this order.

(First Elongated Organic Piezoelectric Body)

The first elongated organic piezoelectric body 121 is made of an organic piezoelectric material and has a piezoelectric constant $d_{14}$.
Whether or not "the first elongated organic piezoelectric body 121 has the piezoelectric constant $d_{14}$" is determined by the following first determination method.
In the first determination method, the first piezoelectric sensor 10A and the first piezoelectric sensor 10B manufactured in accordance with Example 1 described later, and a voltmeter are used.
Specifically, in the first determination method, the first piezoelectric sensor 10A and the first piezoelectric sensor 10B are manufactured in accordance with Example 1 described later.
A rear end part (see Fig. 12) of the first inner conductor 11A of the first piezoelectric sensor 10A and a rear end part (see Fig. 12) of the first outer conductor 13A of the first piezoelectric sensor 10A are electrically connected to the voltmeter. Next, a predetermined external force is applied to the first piezoelectric sensor 10A to detect a determination voltage.
Similarly, a rear end part (see Fig. 12) of the first inner conductor 11A of the second piezoelectric sensor 10B and a rear end part (see Fig. 12) of the first outer conductor 13A of the second piezoelectric sensor 10B are electrically connected to the voltmeter. Next, the same external force as the external force applied to the first piezoelectric sensor 10A is applied to the second piezoelectric sensor 10B, and a determination voltage is detected.
In the first determination method, in a case in which the following condition (Y1) is satisfied, it is determined that "the first elongated organic piezoelectric body 121 has the piezoelectric constant $d_{14}$". On the other hand, when the following condition (Y1) is not satisfied, it is determined that "the first elongated organic piezoelectric body 121 has the piezoelectric constant $d_{14}$" is not satisfied.
(Y1) A polarity of the determination voltage of the first piezoelectric sensor 10A is opposite to a polarity of the determination voltage of the second piezoelectric sensor 10B.
The first elongated organic piezoelectric body 121 is an elongated object.
Examples of a shape of the first elongated organic piezoelectric body 121 include a ribbon shape, a fiber shape, and the like. The ribbon shape is a flat elongated shape. A structure of the fiber may be monofilaments or multifilaments.
In a case in which the first elongated organic piezoelectric body 121 has a ribbon shape, a width of the first elongated organic piezoelectric body 121 is preferably from 0.1 mm to 30 mm. When the width is 0.1 mm or more, the strength of the first elongated organic piezoelectric body 121 is secured. Further, the first elongated organic piezoelectric body 121 is excellent in manufacturing suitability (for example, manufacturing suitability in a slitting step to be described later). When the width is 30 mm or less, the degree of freedom (flexibility) of non-plastic deformation of the first elongated organic piezoelectric body 121 is improved.
In a case in which the first elongated organic piezoelectric body 121 has a ribbon shape, a thickness of the first elongated organic piezoelectric body 121 is preferably from 0.001 mm to 0.2 mm. When the thickness is 0.001 mm or more, the strength of the first elongated organic piezoelectric body 121 is secured. Further, the first elongated organic piezoelectric body 121 is excellent in manufacturing suitability. When the thickness is 0.2 mm or less, the degree of freedom (flexibility) of non-plastic deformation in the thickness direction of the first elongated organic piezoelectric body 121 is improved.
In a case in which the first elongated organic piezoelectric body 121 has a ribbon shape, a ratio of the width of the first elongated organic piezoelectric body 121 to the thickness of the first elongated organic piezoelectric body 121 (Hereinafter, the ratio is referred to as "ratio [width/thickness]".) is preferably 2 or more. When the ratio [width/thickness] is 2 or more, a principal surface of the first elongated organic piezoelectric body 121 becomes clear. Therefore, the first elongated organic piezoelectric body 121 is easily wound around the first outer conductor 13A in the same direction over a length direction of the first elongated organic piezoelectric body 121. Therefore, the first piezoelectric sensor 10A has excellent piezoelectric sensitivity and excellent stability of piezoelectric sensitivity.
In a case in which the first elongated organic piezoelectric body 121 has a fiber shape, examples of the cross-sectional shape of the first elongated organic piezoelectric body 121 include a circular shape, an elliptical shape, a rectangular shape, a cocoon shape, a four-leaf shape, a star shape, and an irregular shape. A major axis diameter of the cross section of the first elongated organic piezoelectric body 121, a major axis diameter of the cross section of the first elongated organic piezoelectric body 121 is preferably from 0.0001 mm to 10 mm, more preferably from 0.001 mm to 5 mm, and still more preferably from 0.002 mm to 1 mm.

The "major axis diameter of the cross section" corresponds to a "diameter" in a case in which the cross-sectional shape of the first elongated organic piezoelectric body 121 is a circular shape, and is a longest width among widths of the cross section in a case in which the cross-sectional shape of the first elongated organic piezoelectric body 121 is not a circular shape.
In a case in which the fiber shape is made of multifilaments, the "major axis diameter of the cross section" is a major axis diameter of the cross section of the multifilaments.
The organic piezoelectric material may be any material as long as the first elongated organic piezoelectric body 121 has the piezoelectric constant $d_{14}$, and examples thereof include an optically active polymer (A).
Examples of the optically active polymer (A) include a helical chiral polymer (A1) (Hereinafter, sometimes referred to as "helical chiral polymer (A1)".) having optical activity, a polypeptide (A2) (Hereinafter, the "optically active polypeptide (A2)" may be referred to.) having optical activity, and the like.
The "helical chiral polymer (A1) having optical activity" refers to a polymer having a helical molecular structure and molecular optical activity. Examples of the helical chiral polymer (A1) include a polylactic acid-based polymer, a synthetic polypeptide, a cellulose derivative, polypropylene oxide, poly ($\beta$-hydroxybutyric acid).
Examples of the polylactic acid-based polymer include a homopolymer of L-lactic acid (Hereinafter referred to as "PLLA".) and a homopolymer of D-lactic acid (Hereinafter referred to as "PDLA".). The molecular structure of PLLA consists of a left-handed helical structure. The molecular structure of PDLA consists of a right-handed helical structure.
Examples of the synthetic polypeptide include poly ($\gamma$-benzyl glutarate) and poly ($\gamma$-methyl glutarate).
Examples of the cellulose derivative include cellulose acetate and cyanoethyl cellulose.
Details of each of the polylactic acid-based polymer and the helical chiral polymer (A1) will be described later.
The "optically active polypeptide (A2)" refers to a polypeptide having asymmetric carbon atoms and having a bias in the abundance of optical isomers. The optically active polypeptide (A2) preferably has a $\beta$-sheet structure from the viewpoint of piezoelectricity and strength. Examples of the optically active polypeptide (A2) include animal proteins having optical activity. Examples of the animal proteins include fibroin and spider silk protein. Examples of the fiber composed of the animal proteins include silk and spider silk.
Details of the animal proteins will be described later.
Among them, the organic piezoelectric material preferably contains the optically active polymer (A), particularly the helical chiral polymer (A1) or the optically active polypeptide (A2) from the viewpoint of good piezoelectricity, processability, availability, and the like. The helical chiral polymer (A1) preferably contains a polylactic acid-based polymer. The optically active polypeptide (A2) preferably contains an animal protein.
Each of the polylactic acid-based polymer and the optically active polypeptide (A2) is non-pyroelectric. Since the organic piezoelectric material contains the polylactic acid-based polymer or the optically active polypeptide (A2), the first piezoelectric sensor 10A is further improved in stability of piezoelectric sensitivity and stability of piezoelectric output (stability with time or temperature change) as compared with a piezoelectric sensor using pyroelectric PVDF.
The optically active polypeptide (A2) is excellent in hydrolysis resistance in a high-temperature and high-humidity environment. In the first piezoelectric sensor 10A containing the optically active polypeptide (A2), for example, a decrease in the first voltage is suppressed particularly under a high temperature and high humidity environment as compared with the first piezoelectric sensor 10A containing a polylactic acid-based polymer.
Details of the helical chiral polymer (A) will be described later.
In a case in which the optically active polymer (A) has a fiber shape, the shape of the first elongated organic piezoelectric body 121 may be a fiber shape or a ribbon shape.
In a case in which the optically active polymer (A) has a fiber shape and the first elongated organic piezoelectric body 121 has a fiber shape, the organic piezoelectric material may be composed of only the optically active polymer (A).
In a case in which the optically active polymer (A) has a fiber shape and the first elongated organic piezoelectric body 121 has a ribbon shape, the organic piezoelectric material may contain the optically active polymer (A) and a resin. In this case, the organic piezoelectric material can be molded into a ribbon shape by a resin. In a case in which the organic piezoelectric material contains a plurality of fiber-shaped polymer materials (A), each of a plurality of the optically active polymers (A) may be bonded by a resin.
The resin includes at least one of a thermoplastic resin and a thermosetting resin.
Examples of the thermoplastic resin include a polymethacrylic resin, a polyacrylic resin, an aromatic polyether ketone, and a polyarylene-based resin. Examples of the polymethacrylic resin include a polymethacrylic resin, a polyolefin resin, and a polymethyl methacrylate resin. Examples of the polyacrylic resin include polymethyl acrylate resins. Examples of the aromatic polyether ketone include a polystyrene resin, a polyvinyl acetal resin, a polycarbonate resin, and a polyphenylene ether resin. Examples of the polyarylene-based resin include a polyphenylene oxide resin and a polyphenylene sulfide (PPS) resin. These thermoplastic resins may be used singly or in combination of two or more kinds thereof.
Examples of the thermosetting resin include an epoxy resin, a phenol resin, an unsaturated polyester resin, a thermosetting polyimide resin, a bismaleimide triazine resin, and a benzoxazine resin. These thermosetting resins may be used alone, or may be used in combination of two or more thereof.

The first elongated organic piezoelectric body 121 preferably has a first composition.

In the first composition,

the organic piezoelectric material includes an optically active polymer (A),
a length direction of the first elongated organic piezoelectric body 121 and a main orientation direction of the optically active polymer (A) contained in the first elongated organic piezoelectric body 121 are substantially parallel (a direction parallel to a double-headed arrow D2 in Fig. 5), and
an orientation degree F of the first elongated organic piezoelectric body 121 determined from X-ray diffraction measurement by the following Formula (a) is from 0.5 to less than 1.0.

$$\text{Orientation degree F} = (180° - \alpha)/180° \cdots \text{(a)}$$

where $\alpha$ represents a half-value width of a peak derived from orientation. The unit of $\alpha$ is °.

The orientation degree F of the first elongated organic piezoelectric body 121 is an index indicating the degree of orientation of the optically active polymer (A) contained in the first elongated organic piezoelectric body 121.

The orientation degree F of the first elongated organic piezoelectric body 121 is, for example, a c-axis orientation degree measured by a wide-angle X-ray diffractometer (RINT 2550 manufactured by Rigaku Corporation, attached device: rotating sample stage, X-ray source: CuK$\alpha$, output: 40 kV 370 mA, detector: scintillation counter).

The orientation degree F of the first elongated organic piezoelectric body 121 is preferably from 0.50 to 0.99, more preferably from 0.70 to 0.98, and particularly preferably from 0.80 to 0.97.

In the first elongated organic piezoelectric body 121, the fact that the length direction of the first elongated organic piezoelectric body 121 and the main orientation direction of the optically active polymer (A) contained in the first elongated organic piezoelectric body 121 are substantially parallel also contributes to the development of piezoelectricity.

The fact that the length direction of the first elongated organic piezoelectric body 121 is substantially parallel to the main orientation direction of the optically active polymer (A) contained in the first elongated organic piezoelectric body 121 also has an advantage that the first elongated organic piezoelectric body 121 is excellent in tensile strength in the length direction. Therefore, when the first elongated organic piezoelectric body 121 is spirally wound around the first inner conductor 11A, the first elongated organic piezoelectric body 121 is hardly broken.

"Substantially parallel" indicates that an angle formed by two line segments is from 0° to less than 30°. The angle formed by the two line segments is preferably from 0° to 22.5°, more preferably from 0° to 10°, still more preferably from 0° to 5°, and particularly preferably from 0° to 3°.

For example, in a case in which the organic piezoelectric material contains silk or spider silk as an example of fibers made of an animal protein, a length direction of the silk or spider silk and a main orientation direction of the optically active polypeptide (A2) (for example, fibroin or spider silk protein which is an example of an animal protein) are substantially parallel in the process of producing the silk or spider silk.

The main orientation direction of the optically active polymer (A) means a main orientation direction of the optically active polymer (A). The main orientation direction of the optically active polymer (A) can be confirmed, for example, by measuring the orientation degree F of the first elongated organic piezoelectric body 121.

In a case in which the first elongated organic piezoelectric body 121 is produced by stretching a film and forming a slit of the stretched film, the main orientation direction of the optically active polymer (A) in the first elongated organic piezoelectric body 121 means a main stretching direction. Here, the main stretching direction refers to a stretching direction in the case of uniaxial stretching, and refers to a stretching direction with a higher stretching ratio in the case of biaxial stretching.

Hereinafter, a case in which the first elongated organic piezoelectric body 121 has the first composition and the optically active polymer (A) is the helical chiral polymer (A1) will be described.

Examples of a method of manufacturing the first elongated organic piezoelectric body 121 include a method in which a raw material (for example, the optically active polymer (A)) is molded into a film shape to obtain an unstretched film, the obtained unstretched film is stretched and crystallized, and the obtained organic piezoelectric film is slit.

Here, "slitting" means cutting the organic piezoelectric film into an elongated shape.

Note that either stretching or crystallization may be performed first. Pre-crystallization, stretching, and crystallization (annealing) may be performed on the unstretched film in this order. The stretching may be uniaxial stretching or biaxial stretching. In a case in which the stretching is biaxial stretching, the stretch ratio in one direction (main stretching direction) is preferably increased.

For a method of manufacturing an organic piezoelectric film, known documents such as Japanese Patent No. 4934235, WO 2010/104196 A, WO 2013/054918 A, and WO 2013/089148 A can be appropriately referred to.

The first elongated organic piezoelectric body 121 will be described later.

[First Configuration of First Piezoelectric Body]

Next, the first configuration A of the first piezoelectric body 12A will be described with reference to Figs. 5 and 6.
In the first configuration A, as illustrated in Fig. 5, the first elongated organic piezoelectric body 121 is wound in a left direction (left turn, i.e. counterclockwise) toward the direction D1. Specifically, the first elongated organic piezoelectric body 121 is spirally wound in the first spiral direction D2 at a first spiral angle β1 toward the direction D1 along the outer peripheral surface of the first inner conductor 11A so as not to have a gap. The wound first elongated organic piezoelectric body 121 constitutes the first piezoelectric body 12A.
The "spiral angle β1" means an angle formed by an axial direction AX of the first inner conductor 11A and an arrangement direction of the first elongated organic piezoelectric body 121 with respect to the axial direction AX of the first inner conductor 11A.
The spiral angle β1 is preferably from 15° to 75° (45° ± 30°), and more preferably from 35° to 55° (45° ± 10°).
The "first spiral direction D2" refers to a direction in which the first elongated organic piezoelectric body 121 is wound in the direction D1.
Next, an operation of the first configuration A will be described.
For example, in the first piezoelectric sensor 10A, in a case in which a tensile force (stress) is applied in a direction parallel to the axial direction AX, when shear strain is applied to the helical chiral polymer (A1) contained in the first elongated organic piezoelectric body 121, polarization of the helical chiral polymer (A1) occurs in a radial direction of the first piezoelectric sensor 10A. A direction of this polarization substantially coincides with a direction of an electric field generated due to the piezoelectric constant $d_{14}$. Specifically, in a case where the first piezoelectric body 12A of the first configuration A in which the first elongated organic piezoelectric body 121 is spirally wound is regarded as an aggregate of minute regions that can be regarded as a plane with respect to the axial direction AX, the direction of the electric field generated due to the piezoelectric constant $d_{14}$ and the polarization direction substantially coincide with each other when the shear force due to the tension (stress) is applied to the helical chiral polymer (A1) on the plane of the minute regions that constitute it. Specifically, it is considered that the polarization of the helical chiral polymer (A1) occurs in the radial direction of the first piezoelectric sensor 10A as indicated by arrows in Fig. 6, and the polarization directions thereof are generated with phases aligned. As a result, the first piezoelectric sensor 10A is likely to effectively generate the first voltage proportional to the external force.
From the above, in a case in which the first piezoelectric body 12A has the first configuration A, the piezoelectric sensitivity and the stability of the piezoelectric output of the first piezoelectric sensor 10A are excellent.
In the first configuration A, in a case in which the helical chiral polymer (A1) is PLLA, when tension acts on the first piezoelectric sensor 10A in the direction parallel to the axial direction AX, an electric field (polarization) is generated in parallel to the radial direction from a center of a circle of a circular cross section perpendicular to the tension toward an outward direction. That is, the polarity of the first voltage is positive.
In the first configuration A, in a case in which the helical chiral polymer (A1) is PLLA, when pressurization acts on the first piezoelectric sensor 10A in the direction parallel to the axial direction AX, an electric field (polarization) is generated in parallel to the radial direction from the outside of the circle of the circular cross section perpendicular to the pressurization toward a central direction. That is, the polarity of the first voltage is negative.
In the first configuration A, in a case in which the helical chiral polymer (A1) is PDLA, the polarity of the first voltage are opposite to those in a case in which the helical chiral polymer (A1) is PLLA.
Specifically, in the first configuration A, in a case in which the helical chiral polymer (A1) is PDLA, when tension acts on the first piezoelectric sensor 10A in a direction parallel to the axial direction AX, an electric field (polarization) is generated in parallel to the radial direction from the outside of a circle of a circular cross section perpendicular to the tension toward the central direction. That is, the polarity of the first voltage is negative.
In the first configuration A, in a case in which the helical chiral polymer (A1) is PDLA, when pressurization acts on the first piezoelectric sensor 10A in the direction parallel to the axial direction AX, an electric field (polarization) is generated in parallel to the radial direction from a center of the circle of the circular cross section perpendicular to the pressurization toward the outward direction. That is, the polarity of the first voltage is positive.
In the first configuration A, the first elongated organic piezoelectric body 121 is wound left-handed in the direction D1, but the configuration of the first piezoelectric body 12A may be a configuration in which the first elongated organic piezoelectric body 121 is wound right-handed in the direction D1 (hereinafter, referred to as "first configuration A'").
In the first configuration A', in a case in which the helical chiral polymer (A1) is PLLA, the polarity of the first voltage are opposite to those in a case in which the helical chiral polymer (A1) of the first configuration A is PLLA.
Specifically, in the first configuration A', in a case in which the helical chiral polymer (A1) is PLLA, when tension acts on the first piezoelectric sensor 10A in the direction parallel to the axial direction AX, an electric field (polarization) is generated in parallel to the radial direction from the outside of the circle of the circular cross section perpendicular to the tension toward the central direction. That is, the polarity of the first voltage is negative.
In the first configuration A', in a case in which the helical chiral polymer (A1) is PLLA, when pressurization acts

on the first piezoelectric sensor 10A in the direction parallel to the axial direction AX, an electric field (polarization) is generated in parallel to the radial direction from the center of the circle of the circular cross section perpendicular to the pressurization toward the outward direction. That is, the polarity of the first voltage is positive.

In the first configuration A', in a case in which the helical chiral polymer (A1) is PDLA, the polarity of the first voltage are opposite to those in a case in which the helical chiral polymer (A1) of the first configuration A is PDLA.

Specifically, in the first configuration A', in a case in which the helical chiral polymer (A1) is PDLA, when tension acts on the first piezoelectric sensor 10A, an electric field (polarization) is generated in parallel to the radial direction from the center of the circle of the circular cross section perpendicular to the tension toward the outward direction. That is, the polarity of the first voltage is positive.

In the first configuration A', in a case in which the helical chiral polymer (A1) is PDLA, when pressurization acts on the first piezoelectric sensor 10A, an electric field (polarization) is generated in parallel to the radial direction from the outside of the circle of the circular cross section perpendicular to the pressurization toward the central direction. That is, the polarity of the first voltage is negative.

(Second Elongated Organic Piezoelectric Body)

The second elongated organic piezoelectric body 122 is made of an organic piezoelectric material and has the piezoelectric constant $d_{14}$.

Whether or not "the second elongated organic piezoelectric body 122 has the piezoelectric constant $d_{14}$" is determined by, for example, the first determination method described above.

The second elongated organic piezoelectric body 122 is an elongated object.

Each of the shape, width, thickness, ratio [width/thickness], cross-sectional shape, and major axis diameter of the second elongated organic piezoelectric body 122 is the same as those exemplified for the first elongated organic piezoelectric body 121. Each of the width, thickness, and ratio [width/thickness] of the second elongated organic piezoelectric body 122 may be the same as or different from each of the width, thickness, and ratio [width/thickness] of the first elongated organic piezoelectric body 121.

Examples of the organic piezoelectric material of the second elongated organic piezoelectric body 122 include materials similar to the materials exemplified as the organic piezoelectric material of the first elongated organic piezoelectric body 121. The organic piezoelectric material of the second elongated organic piezoelectric body 122 may be the same as or different from the organic piezoelectric material of the first elongated organic piezoelectric body 121.

The second elongated organic piezoelectric body 122 preferably has the following second composition.

In the second composition,

the organic piezoelectric material includes an optically active polymer (A),
a length direction of the second elongated organic piezoelectric body 122 and a main orientation direction of the optically active polymer (A) contained in the second elongated organic piezoelectric body 122 are substantially parallel to each other, and
an orientation degree F of the second elongated organic piezoelectric body 122 obtained by the above Formula (a) from the X-ray diffraction measurement is from 0.5 to less than 1.0.

The orientation degree F of the second elongated organic piezoelectric body 122 is an index indicating the degree of orientation of the optically active polymer (A) contained in the second elongated organic piezoelectric body 122.

"Substantially parallel" indicates that an angle formed by two line segments is from 0° to less than 30°. The angle formed by the two line segments is preferably from 0° to 22.5°, more preferably from 0° to 10°, still more preferably from 0° to 5°, and particularly preferably from 0° to 3°.

Hereinafter, a case in which the organic piezoelectric material of the second elongated organic piezoelectric body 122 has the second composition and the optically active polymer (A) is the helical chiral polymer (A1) will be described.

Examples of a method of manufacturing the second elongated organic piezoelectric body 122 include a manufacturing method similar to the manufacturing method exemplified as the method of manufacturing the first elongated organic piezoelectric body 121.

The second elongated organic piezoelectric body 122 will be described later.

[Second Configuration of First Piezoelectric Body]

Next, the second configuration A of the first piezoelectric body 12A will be described with reference to Fig. 7.

The second configuration A of the first piezoelectric body 12A is different from the first configuration A of the first piezoelectric body 12A in including the second elongated organic piezoelectric body 122.

Specifically, as illustrated in Fig. 7, the second configuration A of the first piezoelectric body 12A includes the first elongated organic piezoelectric body 121 and the second elongated organic piezoelectric body 122.
In the second configuration A, similarly to the first configuration A, the first elongated organic piezoelectric body 121 is spirally wound in the first spiral direction D2 toward the direction D1 at the spiral angle β1 along the outer peripheral surface of the first inner conductor 11A without a gap. That is, the first elongated organic piezoelectric body 121 is wound in the left direction (left-handed, i.e. counterclockwise) toward the direction D1.
In the second configuration A, as illustrated in Fig. 7, the second elongated organic piezoelectric body 122 is spirally wound along an outer peripheral surface of the first elongated organic piezoelectric body 121 at a spiral angle β2 in a direction opposite to a winding direction of the first elongated organic piezoelectric body 121.
The "spiral angle β2" is synonymous with the above-described spiral angle β1. The spiral angle β2 is substantially the same as the spiral angle β1.
Here, the "direction opposite to the winding direction of the first elongated organic piezoelectric body 121" in the second configuration A means right winding. That is, the second elongated organic piezoelectric body 122 is wound right-handed in the direction D1.
In Fig. 7, a main orientation direction of the helical chiral polymer (A1) contained in the second elongated organic piezoelectric body 122 is indicated by a double-headed arrow D3. That is, the main orientation direction of the helical chiral polymer (A1) contained in the second elongated organic piezoelectric body 122 is substantially parallel to an arrangement direction of the second elongated organic piezoelectric body 122 (a length direction of the second elongated organic piezoelectric body 122).
The chirality of the helical chiral polymer (A1) contained in the first elongated organic piezoelectric body 121 and the chirality of the helical chiral polymer (A1) contained in the second elongated organic piezoelectric body 122 are preferably different from each other. As a result, for example, when tension in the axial direction AX is applied to the first piezoelectric sensor 10A, polarization occurs in both the helical chiral polymer (A1) contained in the first elongated organic piezoelectric body 121 and the helical chiral polymer (A1) contained in the second elongated organic piezoelectric body 122. Both polarization directions are the radial direction of the first piezoelectric sensor 10A. As a result, a voltage signal (charge signal) proportional to the tension is more effectively detected. Therefore, the piezoelectric sensitivity and the stability of the piezoelectric output are further improved.
Hereinafter, an operation of the first piezoelectric sensor 10A of the second configuration A will be described.
For example, when tension is applied in a direction parallel to the direction D1 of the first piezoelectric sensor 10A, shear stress is applied to both the helical chiral polymer (A1) contained in the first elongated organic piezoelectric body 121 and the helical chiral polymer (A1) contained in the second elongated organic piezoelectric body 122, and polarization occurs. Both polarization directions are the radial direction of the first piezoelectric sensor 10A. As a result, a voltage signal proportional to the tension is detected effectively.
As described above, according to the first piezoelectric sensor 10A of the second configuration A, the piezoelectric sensitivity and the stability of the piezoelectric output are improved as compared with the first piezoelectric sensor 10A of the first configuration A.
In particular, the first piezoelectric body 12A includes the first elongated organic piezoelectric body 121 and the second elongated organic piezoelectric body 122, and has a two-layer structure. Therefore, each of the first elongated organic piezoelectric body 121 and the second elongated organic piezoelectric body 122 can be in close contact with the first inner conductor 11A and the first outer conductor 13A without an air gap. As a result, the electric field generated by the tension is likely to be efficiently transmitted to the first inner conductor 11A.
Therefore, the first piezoelectric sensor 10A of the second configuration A can detect the external force with higher sensitivity.

[Third Configuration of First Piezoelectric Body]

Next, the third configuration A of the first piezoelectric body 12A will be described with reference to Fig. 8.
The third configuration A of the first piezoelectric body 12A is different from the first piezoelectric sensor 10A of the second configuration A of the first piezoelectric body 12A in forming a braid structure.
Specifically, as illustrated in Fig. 8, the third configuration A of the first piezoelectric body 12A includes the first elongated organic piezoelectric body 121 and the second elongated organic piezoelectric body 122.
In the third configuration A, the first elongated organic piezoelectric body 121 is spirally wound left-handed at the spiral angle β1 with respect to the axial direction AX of the first inner conductor 11A, the second elongated organic piezoelectric body 122 is spirally wound right-handed at the spiral angle β2, and the first elongated organic piezoelectric body 121 and the second elongated organic piezoelectric body 122 are alternately crossed. The axial direction AX of the first inner conductor 11A is substantially parallel to the direction D1.
"Substantially parallel" indicates that an angle formed by two line segments is from 0° to less than 30°. The angle formed by the two line segments is preferably from 0° to 22.5°, more preferably from 0° to 10°, still more preferably

from 0° to 5°, and particularly preferably from 0° to 3°.

In the braid structure illustrated in Fig. 8, a main orientation direction (double-headed arrow D2) of the helical chiral polymer (A1) contained in the first elongated organic piezoelectric body 121 is substantially parallel to an arrangement direction of the first elongated organic piezoelectric body 121. Similarly, a main orientation direction (double-headed arrow D2) of the helical chiral polymer (A1) contained in the second elongated organic piezoelectric body 122 is substantially parallel to an arrangement direction of the second elongated organic piezoelectric body 122.

The chirality of the helical chiral polymer (A1) contained in the first elongated organic piezoelectric body 121 and the chirality of the helical chiral polymer (A1) contained in the second elongated organic piezoelectric body 122 are preferably different from each other. As a result, for example, when tension in the axial direction AX is applied to the first piezoelectric sensor 10A, polarization occurs in both the helical chiral polymer (A1) contained in the first elongated organic piezoelectric body 121 and the helical chiral polymer (A1) contained in the second elongated organic piezoelectric body 122. Both polarization directions are the radial direction of the first piezoelectric sensor 10A. As a result, a voltage signal proportional to the tension is more effectively detected. As a result, the piezoelectric sensitivity and the stability of the piezoelectric output are further improved.

Hereinafter, an operation of the first piezoelectric sensor 10A of the third configuration A will be described.

For example, when tension in the axial direction AX is applied to the first piezoelectric sensor 10A, polarization occurs in both the helical chiral polymer (A1) contained in the first elongated organic piezoelectric body 121 and the helical chiral polymer (A1) contained in the second elongated organic piezoelectric body 122. Both polarization directions are the radial direction of the first piezoelectric sensor 10A. As a result, a voltage signal proportional to the tension is detected effectively.

As described above, the first piezoelectric sensor 10A of the third configuration A can detect the external force applied to the first piezoelectric body 12A with higher sensitivity.

In particular, when tension is applied in the length direction of the first piezoelectric sensor 10A, a shear stress acts on the left-handed first elongated organic piezoelectric body 121 and the right-handed second elongated organic piezoelectric body 122 forming the braid structure. These polarization directions coincide with each other, and a volume fraction contributing to the piezoelectric performance in an insulator (that is, the first piezoelectric body 12A and the second piezoelectric body 12B) between the first inner conductor 11A and the first outer conductor 13A increases. Therefore, the piezoelectric performance is further improved.

(Sheet-shaped Piezoelectric Body)

The sheet-shaped piezoelectric body is made of a piezoelectric material, has a piezoelectric constant $d_{33}$ and a piezoelectric constant dsi, and does not have the piezoelectric constant $d_{14}$.

Whether or not "the sheet-shaped piezoelectric body has the piezoelectric constant $d_{33}$ and the piezoelectric constant dsi, and does not have the piezoelectric constant $d_{14}$" is determined by the following second determination method.

In the second determination method, the first piezoelectric sensor 10A (Hereinafter, the "first piezoelectric sensor 10A of the fourth configuration A" may be referred to.) in which the first piezoelectric body 12A is the fourth configuration A described later, the second piezoelectric sensor 10B (Hereinafter, the "second piezoelectric sensor 10B of the fourth configuration B" may be referred to.) in which the second piezoelectric body 12B described later is the fourth configuration B described later, and a voltmeter are used.

Specifically, in the second determination method, the first piezoelectric sensor 10A of the fourth configuration A and the second piezoelectric sensor 10B of the fourth configuration B are manufactured.

The first inner conductor 11A of the first piezoelectric sensor 10A of the fourth configuration A and the first outer conductor 13A of the first piezoelectric sensor 10A of the fourth configuration A are electrically connected to the voltmeter. Next, a predetermined external force is applied to the first piezoelectric sensor 10A of the fourth configuration A to detect a determination voltage.

Similarly, the first inner conductor 11A of the second piezoelectric sensor 10B in the fourth configuration B and the first outer conductor 13A of the second piezoelectric sensor 10B in the fourth configuration B are electrically connected to the voltmeter. Next, the same external force as the external force applied to the first piezoelectric sensor 10A of the fourth configuration A is applied to the second piezoelectric sensor 10B of the fourth configuration B, and a determination voltage is detected.

In the second determination method, in a case in which the following condition (Y2) is satisfied, it is determined that "the sheet-shaped piezoelectric body has the piezoelectric constant $d_{33}$ and the piezoelectric constant dsi, and does not have the piezoelectric constant $d_{14}$" is satisfied. On the other hand, in a case in which the following condition (Y2) is not satisfied, it is determined that "the sheet-shaped piezoelectric body has the piezoelectric constant $d_{33}$ and the piezoelectric constant dsi, and does not have the piezoelectric constant $d_{14}$" is not satisfied.

(Y2) A polarity of the determination voltage of the first piezoelectric sensor 10A of the fourth configuration A is opposite

to a polarity of the determination voltage of the second piezoelectric sensor 10B of the fourth configuration B.

The sheet-shaped piezoelectric body has a sheet shape.

A thickness of the sheet-shaped piezoelectric body is preferably from 0.001 mm to 0.2 mm. When the thickness is 0.001 mm or more, the strength of the sheet-shaped piezoelectric body is secured. Further, when the thickness is 0.2 mm or less, the degree of freedom (flexibility) of non-plastic deformation in a thickness direction of the sheet-shaped piezoelectric body is improved.

The piezoelectric material may be any material as long as the sheet-shaped piezoelectric body has the piezoelectric constant $d_{33}$ and the piezoelectric constant $d_{31}$ and does not have the piezoelectric constant $d_{14}$, and examples thereof include an organic piezoelectric material and an inorganic piezoelectric material. The organic piezoelectric material may be a low molecular weight material or a polymer material.

The organic piezoelectric material is preferably at least one selected from the group consisting of, for example, polyvinylidene fluoride (PVDF), polytetrafluoroethylene (PTFE), polyvinyl fluoride (PVF), polychlorotrifluoroethylene (PCTFE), ethyl trifluoroacetate (EFA), tetrafluoroethylene-perfluoroalkyl vinyl ether copolymer (PFA), tetrafluoroethylene-hexafluoropropylene copolymer (FEP), chlorotrifluoroethylene-ethylene copolymer (ETFE), chlorotrifluoroethylene-ethylene copolymer (ECTFE), poly (vinylidene fluoride/hexafluoropropylene) copolymer (PVDF-HFP), and poly (vinylidene fluoride/trifluoroethylene).

Examples of the inorganic piezoelectric material include a material obtained by molding powder of an inorganic ceramic piezoelectric material such as lead zirconate titanate (PZT) into a sheet shape using a polymer.

Among them, the piezoelectric material preferably contains PVDF from the viewpoint of being lightweight, being rich in flexibility, and being excellent in workability and the like.

Hereinafter, a case where the piezoelectric material is made of PVDF will be described.

Examples of a method of manufacturing the sheet-shaped piezoelectric body include a method in which a raw material (PVDF) is molded into a sheet, subjected to a stretching treatment to obtain a stretched sheet, and the obtained stretched sheet is subjected to a poling treatment.

In the poling treatment, for example, while the stretched sheet is heated, a positive voltage is applied to one surface of the stretched sheet, and a negative voltage is applied to the other surface of the stretched sheet. As a result, in a film thickness direction of the stretched sheet, a dipole moment due to a difference in electric attraction degree between a fluorine atom and a carbon atom is formed from one surface to the other surface of the stretched sheet. That is, the sheet-shaped piezoelectric body has spontaneous polarization.

[Fourth Configuration of First Piezoelectric Body]

Next, the fourth configuration A of the first piezoelectric body 12A will be described.

In the fourth configuration A, the sheet-shaped piezoelectric body is wound in a cylindrical shape along the outer peripheral surface of the first inner conductor 11A such that polymer chains of PVDF are oriented along the axial direction AX. One surface of the sheet-shaped piezoelectric body is on a side of the first inner conductor 11A. The wound sheet-shaped piezoelectric body constitutes the first piezoelectric body 12A.

The phrase "polymer chains of PVDF are oriented along the axial direction AX" means that a stretching direction in the stretching treatment in the manufacturing process of the sheet-shaped piezoelectric body and the axial direction AX have a parallel relationship.

Next, an operation of the fourth configuration A will be described.

For example, in the first piezoelectric sensor 10A, in a case in which the first piezoelectric body 12A contracts when an external force is applied, an electric field (polarization) is generated in parallel with the radial direction from the center of the circle of the circular cross section toward the outward direction. That is, the polarity of the first voltage is positive.

For example, in the first piezoelectric sensor 10A, in a case in which the first piezoelectric body 12A extends when the external force is applied, an electric field (polarization) is generated in parallel with the radial direction from the outside of the circle of the circular cross section toward the central direction. That is, the polarity of the first voltage is negative.

< Adhesive Layer >

The first piezoelectric sensor 10A may have an adhesive layer. The adhesive layer is disposed, for example, between the first inner conductor 11A and the first piezoelectric body 12A. As a result, in the axial direction AX of the inner conductor 11A, for example, even when tension caused by an external force acts on the first piezoelectric sensor 10A, occurrence of deviation in relative positions between the first piezoelectric body 12A and the first inner conductor 11A can be suppressed. The axial direction AX and the direction D1 are parallel. Further, the tension caused by the external force is more easily applied to the first elongated organic piezoelectric body 121.

Examples of a material of the adhesive that forms the adhesive layer include epoxy-based adhesives, urethane-based adhesives, vinyl acetate resin-based emulsion type adhesives, ethylene vinyl acetate (EVA)-based emulsion type adhesives, acrylic resin-based emulsion type adhesives, styrene-butadiene rubber-based latex type adhesives, silicone resin-based adhesives, $\alpha$-olefin (isobutene-maleic anhydride resin)-based adhesives, vinyl chloride resin-based solvent type adhesives, rubber-based adhesives, elastic adhesives, chloroprene rubber-based solvent type adhesives, nitrile rubber-based solvent type adhesives, and cyanoacrylate-based adhesives.

< First Insulator >

The first piezoelectric sensor 10A may have a first insulator. The first insulator is disposed, for example, between the first piezoelectric body 12A and the first inner conductor 11A and/or between the first piezoelectric body 12A and the first outer conductor 13A. Thus, the occurrence of a short circuit between the first inner conductor 11A and the first outer conductor 13A can be further suppressed.

The first insulator is formed by, for example, spirally winding an elongated object along the outer peripheral surface of the first inner conductor 11A.

A material of the first insulator may be any material having electrical insulation properties, and examples thereof include vinyl chloride resin, polyethylene resin, polypropylene resin, ethylene-tetrafluoroethylene copolymer (ETFE), tetrafluoroethylene-hexafluoropropylene copolymer (FEP), tetrafluoroethylene resin (PTFE), tetrafluoroethylene-perfluoropropyl vinyl ether copolymer (PFA), fluororubber, polyester resin, polyimide resin, polyamide resin, polyethylene terephthalate resin (PET), and rubber (including elastomer).

< Second Insulator >

The first piezoelectric sensor 10A may have a second insulator. The second insulator is disposed at the outer periphery of the first outer conductor 13A. The second insulator may cover the entire outer periphery of the first outer conductor 13A. As a result, the first inner conductor 11A can be electrostatically shielded, and a change in the voltage of the first voltage due to the influence of external static electricity can be suppressed.

A material of the second insulator may be any material having electrical insulation properties, and examples of the material of the second insulator include the same materials as those exemplified as the materials of the first insulator.

< Functional Layer >

The first piezoelectric sensor 10A may have a functional layer. The functional layer is disposed, for example, between the first piezoelectric body 12A and the first inner conductor 11A and/or between the first piezoelectric body 12A and the first outer conductor 13A.

Examples of a layer constituting the functional layer (Hereinafter, the layer is referred to as a "constituent layer".) include an easy adhesive layer, a hard coat layer, a refractive index adjusting layer, an anti-reflection layer, an anti-glare layer, an easily slipping layer, an antiblock layer, a protective layer, an adhesive layer, an antistatic layer, a heat dissipation layer, an ultraviolet absorbing layer, an anti-Newton ring layer, a light scattering layer, a polarizing layer, a gas barrier layer, a hue adjusting layer, and an electrode layer.

The functional layer may have a single-layer structure including a single layer of constituent layers, or may have a plurality of structures including two or more constituent layers. In a case in which the functional layer has the plurality of structures, each of the plurality of constituent layers may be the same constituent layer or different constituent layers. In a case in which the first piezoelectric sensor 10A has functional layers between the first piezoelectric body 12A and the first inner conductor 11A and between the first piezoelectric body 12A and the first outer conductor 13A, the functional layer between the first piezoelectric body 12A and the first inner conductor 11A and the functional layer between the first piezoelectric body 12A and the first outer conductor 13A may be the same or different.

A film thickness of the functional layer is not particularly limited, and is preferably from 0.01 $\mu$m to 10 $\mu$m.

A material of the functional layer is appropriately selected according to a function required for the functional layer, and is, for example, an inorganic substance such as a metal or a metal oxide. an organic substance such as a resin, a composite composition containing a resin and microparticles, and the like. Examples of the resin include a cured product obtained by curing the resin at a temperature or with an active energy ray.

(Second Piezoelectric Sensor)

Next, details of the second piezoelectric sensor 10B will be described.

As illustrated in Fig. 1, the second piezoelectric sensor 10B includes the second inner conductor 11B, the second piezoelectric body 12B, and the second outer conductor 13B. The second inner conductor 11B extends in the

direction D1. The second piezoelectric body 12B covers at least a part of the second inner conductor 11B. The second outer conductor 13B is disposed at the outer periphery of the second piezoelectric body 12B.

A configuration of the second piezoelectric sensor 10B may be the same as the configuration of the first piezoelectric sensor 10A except that the second piezoelectric body 12B and the first piezoelectric body 12A are different. From the viewpoint that the piezoelectric device detects the external force with higher sensitivity, in the second piezoelectric sensor 10B, the other configuration of the second piezoelectric body 12B is preferably the same as the configuration of the first piezoelectric sensor 10A.

The second piezoelectric sensor 10B is a linear object. A cross-sectional shape of a surface orthogonal to the direction D1 of the second piezoelectric sensor 10B is similar to that exemplified as the cross-sectional shape of the first piezoelectric sensor 10A. The cross-sectional shape and the like of the second piezoelectric sensor 10B may be the same as or different from the cross-sectional shape and the like of the first piezoelectric sensor 10A.

< Second Inner Conductor >

Examples of the second inner conductor 11B include those similar to those exemplified as the first inner conductor 11A of the first piezoelectric sensor 10A. The second inner conductor 11B may be the same as or different from the first inner conductor 11A.

< Second Outer Conductor >

Examples of the second outer conductor 13B include the same conductors as those exemplified as the second outer conductor 13A of the first piezoelectric sensor 10A. The second outer conductor 13B may be the same as or different from the second outer conductor 13A.

(Second Piezoelectric Body)

When an external force acts, the second piezoelectric body 12B generates the second voltage between the second inner conductor 11B and the second outer conductor 13B.

The second piezoelectric body 12B may cover at least a part of the second inner conductor 11B, may cover a part of the outer peripheral surface of the second inner conductor 11B, or may cover the entire outer peripheral surface of the second inner conductor 11B.

A configuration of the second piezoelectric body 12B only needs to be a configuration that generates the second voltage whose voltage is different in polarity from the first voltage, and is appropriately adjusted according to the configuration of the first piezoelectric body 12A.

In a case in which the configuration of the first piezoelectric body 12A is the first configuration A, the configuration of the second piezoelectric body 12B is preferably a first configuration B. In the first configuration B, the first elongated organic piezoelectric body 121 is wound. Details of the first configuration B of the second piezoelectric body 12B will be described later.

In a case in which the configuration of the first piezoelectric body 12A is the second configuration A, the configuration of the second piezoelectric body 12B is preferably a second configuration B. In the second configuration B, the first elongated organic piezoelectric body 121 and the second elongated organic piezoelectric body 122 are wound. Details of the second configuration B of the second piezoelectric body 12B will be described later.

In a case in which the configuration of the first piezoelectric body 12A is the third configuration A, the configuration of the second piezoelectric body 12B is preferably a third configuration B. In the third configuration B, the braid structure is wound. The braid structure is formed by alternately crossing the first elongated organic piezoelectric body 121 and the second elongated organic piezoelectric body 122. Details of the third configuration B of the second piezoelectric body 12B will be described later.

In a case in which the configuration of the first piezoelectric body 12A is the fourth configuration A, the configuration of the second piezoelectric body 12B is preferably a fourth configuration B. In the fourth configuration B, the sheet-shaped piezoelectric body is wound. Details of the fourth configuration B of the second piezoelectric body 12B will be described later.

< First Configuration of Second Piezoelectric Body >

Similarly to the first configuration A, the first configuration B of the second piezoelectric body is formed by winding the first elongated organic piezoelectric body 121. At this time, in the first configuration B, the first elongated organic piezoelectric body 121 is spirally wound in a direction opposite to the winding direction of the first configuration A. Specifically, in the second piezoelectric body 12B, the first elongated organic piezoelectric body 121 is wound in the

second spiral direction D3 toward the direction D1 with respect to the first inner conductor 11A. That is, in the first configuration B, the first elongated organic piezoelectric body 121 is wound right-handed in the direction D1. As a result, when the same external force as that of the first piezoelectric body 12A acts on the second piezoelectric body B, the polarization direction of the helical chiral polymer (A1) contained in the second piezoelectric body 12B is different from the polarization direction of the helical chiral polymer (A1) contained in the first piezoelectric body 12A. That is, the second voltage having a polarity different from that of the first voltage is generated between the second inner conductor 11B and the second outer conductor 13B.

The first configuration A and the first configuration B will be further described with reference to Figs. 9 and 10. Fig. 9 is a partially cutaway perspective view of one aspect of the first piezoelectric sensor 10A and the second piezoelectric sensor 10B according to the present embodiment. Fig. 10 is a partially cutaway perspective view of another aspect of the first piezoelectric sensor 10A and the second piezoelectric sensor 10B according to the present embodiment.

The first piezoelectric body 12A of the first piezoelectric sensor 10A illustrated in Fig. 9 has the first configuration A. The second piezoelectric body 12B of the second piezoelectric sensor 10B illustrated in Fig. 9 has the first configuration B. The helical chiral polymer (A1) of each of the first configuration A and the first configuration B illustrated in Fig. 9 is PLLA.

A case in which an external force acts on the piezoelectric device 1, and tension F1 acts on the first piezoelectric sensor 10A and the second piezoelectric sensor 10B in the axial direction AX with respect to the first piezoelectric sensor 10A and the second piezoelectric sensor 10B, or a case in which a torsional force in a clockwise direction F2 acts on a spiral axis as a central axis will be described.

In this case, as illustrated in Fig. 9, a shear stress SF is applied to the first elongated organic piezoelectric body 121 of the first piezoelectric sensor 10A, and polarization of the PLLA occurs from the central direction of the circular cross section toward the outward direction. Therefore, the first voltage is generated in the first piezoelectric sensor 10A.

On the other hand, the shear stress SF is applied to the first elongated organic piezoelectric body 122 of the second piezoelectric sensor 10B, and the PLLA is polarized from the outward direction to the inward direction of the circular cross section. Therefore, the first voltage is generated in the first piezoelectric sensor 10A.

Therefore, in the first piezoelectric sensor 10A and the second piezoelectric sensor 10B, a charge (electric field) proportional to the tension F 1 and the torsional force is generated, and the generated charge is detected as a voltage signal (charge signal).

A case in which an external force acts on the piezoelectric device 1, pressurization F3 acts on the first piezoelectric sensor 10A and the second piezoelectric sensor 10B in the axial direction AX with respect to the first piezoelectric sensor 10A and the second piezoelectric sensor 10B, or a case in which a torsional force in a counterclockwise direction F4 acts on a spiral axis as a central axis will be described.

In this case, as illustrated in Fig. 10, a shear stress SF is applied to the first elongated organic piezoelectric body 121 of the first piezoelectric sensor 10A, and polarization of the PLLA occurs from the outward direction to the inward direction of the circular cross section. Therefore, the first voltage is generated in the first piezoelectric sensor 10A.

On the other hand, the shear stress SF is applied to the first elongated organic piezoelectric body 122 of the second piezoelectric sensor 10B, and the PLLA is polarized from the central direction of the circular cross section toward the outward direction. Therefore, the second voltage is generated in the first piezoelectric sensor 10A.

Therefore, in the first piezoelectric sensor 10A and the second piezoelectric sensor 10B, a charge (electric field) proportional to the pressurization F3 and the torsional force is generated, and the generated charge is detected as a voltage signal (charge signal).

In a case in which the first piezoelectric body 12A is the first configuration A, the second piezoelectric body 12A is the first configuration B, and the helical chiral polymer (A1) of each of the first configuration A and the first configuration B is PDLA, the polarization direction of the PDLA is opposite to the polarization direction of the PLLA.

In the first configuration B, the first elongated organic piezoelectric body 121 is spirally wound in a direction opposite to the winding direction of the first configuration A, but the present disclosure is not limited thereto. For example, the configuration of the second piezoelectric body 12B may be a first configuration B'.

In the first configuration B', the first elongated organic piezoelectric body 121 is spirally wound in the same direction as the winding direction of the first configuration A. For the helical chiral polymer (A1), in a case in which the first configuration A is PLLA, the first configuration B' is PDLA, and in a case in which the first configuration A is PDLA, the first configuration B' is PLLA. As a result, the second voltage having a polarity different from that of the first voltage is generated between the second inner conductor 11B and the second outer conductor 13B.

< Second Configuration of Second Piezoelectric Body >

Similarly to the second configuration A of the first piezoelectric body 12A, the second configuration B of the second piezoelectric body 12B is formed by winding the first elongated organic piezoelectric body 121 and the second elongated organic piezoelectric body 122. At this time, in the second configuration B, each of the first elongated organic piezoelectric body 121 and the second elongated organic piezoelectric body 122 is spirally wound in a direction opposite

to the winding direction of the second configuration A. As a result, the second voltage having a polarity different from that of the first voltage is generated between the second inner conductor 11B and the second outer conductor 13B.

In the second configuration B, each of the first elongated organic piezoelectric body 121 and the second elongated organic piezoelectric body 122 is spirally wound in a direction opposite to the winding direction of the second configuration A, but the present disclosure is not limited thereto. For example, the configuration of the second piezoelectric body 12B may be the second configuration B'.

In the second configuration B', each of the first elongated organic piezoelectric body 121 and the second elongated organic piezoelectric body 122 is spirally wound in the same direction as the winding direction of the second configuration A. For the helical chiral polymer (A1) of the first elongated organic piezoelectric body 121, in a case in which the second configuration A is PLLA, the second configuration B' is PDLA, and in a case in which the second configuration A is PDLA, the second configuration B' is PLLA. For the helical chiral polymer (A1) of the second elongated organic piezoelectric body 122, in a case in which the second configuration A is PLLA, the second configuration B' is PDLA, and in a case in which the second configuration A is PDLA, the second configuration B' is PLLA. As a result, the second voltage having a polarity different from that of the first voltage is generated between the second inner conductor 11B and the second outer conductor 13B.

< Third Configuration of Second Piezoelectric Body >

The third configuration B of the second piezoelectric body 12B is preferably formed by winding a braid structure similarly to the third configuration A of the first piezoelectric body 12A. At this time, in the third configuration B, each of the first elongated organic piezoelectric body 121 and the second elongated organic piezoelectric body 122 is spirally wound in a direction opposite to the winding direction of the third configuration A. As a result, the second voltage having a polarity different from that of the first voltage is generated between the second inner conductor 11B and the second outer conductor 13B.

In the third configuration B, each of the first elongated organic piezoelectric body 121 and the second elongated organic piezoelectric body 122 is spirally wound in a direction opposite to the winding direction of the third configuration A, but the present disclosure is not limited thereto. For example, the configuration of the third piezoelectric body 12B may be a third configuration B'.

In the third configuration B', each of the first elongated organic piezoelectric body 121 and the second elongated organic piezoelectric body 122 is spirally wound in the same direction as the winding direction of the third configuration A. For the helical chiral polymer (A1) of the first elongated organic piezoelectric body 121, in a case in which the second configuration A is PLLA, the second configuration B' is PDLA, and in a case in which the second configuration A is PDLA, the second configuration B' is PLLA. For the helical chiral polymer (A1) of the second elongated organic piezoelectric body 122, in a case in which the second configuration A is PLLA, the second configuration B' is PDLA, and in a case in which the second configuration A is PDLA, the second configuration B' is PLLA. As a result, the second voltage having a polarity different from that of the first voltage is generated between the second inner conductor 11B and the second outer conductor 13B.

< Fourth Configuration of Second Piezoelectric Body >

The fourth configuration B of the second piezoelectric body 12B is preferably formed by winding a sheet-shaped piezoelectric body similarly to the fourth configuration A of the first piezoelectric body 12A. At this time, in the fourth configuration B, a direction of a surface of the sheet-shaped piezoelectric body is opposite to that of the first piezoelectric body 12A. Specifically, since one surface of the sheet-shaped piezoelectric body in the first piezoelectric body 12A is on a side of the first inner conductor 11A, the other surface of the sheet-shaped piezoelectric body in the second piezoelectric body 12B is on a side of the second inner conductor 11B. As a result, the second voltage having a polarity different from that of the first voltage is generated between the second inner conductor 11B and the second outer conductor 13B.

< Adhesive Layer >

The second piezoelectric sensor 10B may have an adhesive layer. The adhesive layer of the second piezoelectric sensor 10B is disposed between the second inner conductor 11B and the second piezoelectric body 12B.

Examples of the adhesive layer of the second piezoelectric sensor 10B include the same layers as those exemplified as the adhesive layer of the first piezoelectric sensor 10A.

< First Insulator >

The second piezoelectric sensor 10B may have a first insulator. The first insulator is disposed, for example, between the second piezoelectric body 12B and the second inner conductor 11B and/or between the second piezoelectric body 12B and the second outer conductor 13B.
Examples of the first insulator of the second piezoelectric sensor 10B include those similar to those exemplified as the first insulator of the first piezoelectric sensor 10A.

< Second Insulator >

The second piezoelectric sensor 10B may have a second insulator. The second insulator is disposed at the outer periphery of the second outer conductor 13B. The second insulator may cover the entire outer periphery of the second outer conductor 13B.
Examples of the second insulator of the second piezoelectric sensor 10B include those similar to those exemplified as the second insulator of the first piezoelectric sensor 10A.

< Functional Layer >

The second piezoelectric sensor 10B may have a functional layer. The second piezoelectric sensor 10B is disposed, for example, between the second piezoelectric body 12B and the second inner conductor 11B and/or between the second piezoelectric body 12B and the second outer conductor 13B.
Examples of the functional layer of the second piezoelectric sensor 10B include the same layers as those exemplified as the functional layer of the first piezoelectric sensor 10A.

(Helical Chiral Polymer (A1))

Next, the helical chiral polymer (A1) will be described.
From the viewpoint of further improving the piezoelectricity, the helical chiral polymer (A1) preferably has an optical purity of 95.00%ee or more. The helical chiral polymer (A1) is preferably composed of a D-body or an L-body from the viewpoint of further improving piezoelectricity. From the viewpoint of further improving the piezoelectricity, the content of the helical chiral polymer (A1) is preferably 80 mass% or more with respect to the total amount of the first elongated organic piezoelectric body 121.
The optical purity of the helical chiral polymer (A1) is preferably 95.00%ee or more, more preferably 96.00%ee or more, still more preferably 99.00%ee or more, particularly preferably 99.99%ee or more, and desirably 100.00%ee from the viewpoint of improving the piezoelectricity of the first elongated organic piezoelectric body 121. It is considered that by setting the optical purity of the helical chiral polymer (A1) within the above range, the packing property of the polymer crystal exhibiting piezoelectricity is enhanced, and as a result, the piezoelectricity is enhanced.
The optical purity of the helical chiral polymer (A1) is a value calculated by the following Formula.

$$\text{Optical purity (\%ee)} = 100 \times |\text{L-body amount - D-body amount}|/(\text{L-body amount + D-body amount})$$

That is, the optical purity of the helical chiral polymer (A1) is
'numerical value obtained by dividing "amount difference (absolute value) between amount [% by mass] of L-body of helical chiral polymer (A1) and amount [% by mass] of D-body of helical chiral polymer (A1)" by "total amount of amount [mass%] of L-body of helical chiral polymer (A1) and amount [mass%] of D-body of helical chiral polymer (A1)"' is multiplied by "100".
Note that as the amount [mass%] of the L-body of the helical chiral polymer (A1) and the amount [mass%] of the D-body of the helical chiral polymer (A1), values obtained by a method using high performance liquid chromatography (HPLC) are used. Specific details of the measurement will be described later.

- Weight Average Molecular Weight -

The weight average molecular weight (Mw) of the helical chiral polymer (A1) is preferably from 50,000 to 1 million.
When the Mw of the helical chiral polymer (A1) is 50,000 or more, the mechanical strength of the first elongated organic piezoelectric body 121 and the second elongated organic piezoelectric body 122 (Hereinafter, the first elongated

organic piezoelectric body 121 and the like may be collectively referred to.) is improved. The Mw is more preferably 100,000 or more, still more preferably 200,000 or more.

When the Mw of the helical chiral polymer (A1) is 1 million or less, moldability when the first elongated organic piezoelectric body 121 and the like are obtained by molding (for example, extrusion molding and melt spinning) is improved. The Mw of the helical chiral polymer (A1) is more preferably 800,000 or less, still more preferably 300,000 or less.

The molecular weight distribution (Mw/Mn) of the helical chiral polymer (A1) is preferably from 1.1 to 5, more preferably from 1.2 to 4, and still more preferably from 1.4 to 3 from the viewpoint of the strength of the first elongated organic piezoelectric body 121 and the like.

The weight average molecular weight (Mw) and the molecular weight distribution (Mw/Mn) of the helical chiral polymer (A1) refer to values measured using a gel permeation chromatograph (GPC). Here, Mn is the number average molecular weight of the helical chiral polymer (A1).

Hereinafter, an example of a method of measuring Mw and Mw/Mn of the helical chiral polymer (A1) by GPC will be described.

- GPC Measurement Device -

GPC -100 manufactured by Waters Corporation

- Column -

Shodex LF-804 manufactured by Showa Denko K.K.

- Sample Preparation -

The first elongated organic piezoelectric body 121 and the like are dissolved in a solvent (for example, chloroform) at 40°C to prepare a sample solution having a concentration of 1 mg/ml.

- Measurement Conditions -

0.1 ml of the sample solution is introduced into the column at a solvent [chloroform], a temperature of 40°C, and a flow rate of 1 ml/min.

A sample concentration in the sample solution separated by the column is measured with a differential refractometer.

A universal calibration curve is prepared with a polystyrene standard sample, and the weight average molecular weight (Mw) and the molecular weight distribution (Mw/Mn) of the helical chiral polymer (A1) are calculated.

As the polylactic acid-based polymer which is an example of the helical chiral polymer (A1), commercially available polylactic acid can be used.

Examples of the commercially available product include PURASORB (PD, PL) manufactured by PURAC, LACEA (H-100, H-400) manufactured by Mitsui Chemicals, Inc., and Ingeo (trademark) biopolymer manufactured by NatureWorks LLC.

When a polylactic acid-based polymer is used as the helical chiral polymer (A1), in order to set the weight average molecular weight (Mw) of the polylactic acid-based polymer to 50,000 or more, it is preferable to manufacture the polylactic acid-based polymer by a lactide method or a direct polymerization method.

The first elongated organic piezoelectric body 121 and the like may contain only one kind or two or more kinds of the helical chiral polymers (A1).

The content (total content in the case of two or more kinds) of the helical chiral polymer (A1) in the first elongated organic piezoelectric body 121 and the like is preferably 80 mass% or more with respect to the total amount of the first elongated organic piezoelectric body 121.

(Polylactic Acid-based Polymer)

Next, the polylactic acid-based polymer will be described.

The polylactic acid-based polymer preferably has a main chain containing a repeating unit represented by the following Formula (1) from the viewpoint of increasing optical purity and improving piezoelectricity.

[Chemical Formula 1]

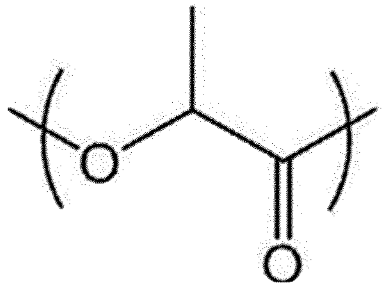

(1)

The polylactic acid-based polymer refers to "polylactic acid (polymer composed only of repeating unit derived from monomer selected from L-lactic acid and D-lactic acid)", "copolymer of L-lactic acid or D-lactic acid and compound copolymerizable with the L-lactic acid or D-lactic acid", or a mixture of both.

Among the polylactic acid-based polymers, polylactic acid is preferable, and PLLA or PDLA is most preferable.

Polylactic acid is a long-linked polymer obtained by polymerizing lactic acid through ester bonds.

A method of manufacturing polylactic acid includes a lactide method via lactide, and a direct polymerization method in which lactic acid is heated under reduced pressure in a solvent and polymerized while removing water.

Examples of the polylactic acid include a block copolymer and a graft copolymer. The block copolymer contains a homopolymer of L-lactic acid, a homopolymer of D-lactic acid, and a polymer of at least one of L-lactic acid and D-lactic acid. The graft copolymer contains a polymer of at least one of L-lactic acid and D-lactic acid.

Examples of the "compound copolymerizable with L-lactic acid or D-lactic acid" include hydroxycarboxylic acids such as glycolic acid, dimethyl glycolic acid, 3-hydroxybutyric acid, 4-hydroxybutyric acid, 2-hydroxypropanoic acid, 3-hydroxypropanoic acid, 2-hydroxyvaleric acid, 3-hydroxyvaleric acid, 4-hydroxyvaleric acid, 5-hydroxyvaleric acid, 2-hydroxycaproic acid, 3-hydroxycaproic acid, 4-hydroxycaproic acid, 5-hydroxycaproic acid, 6-hydroxycaproic acid, 6-hydroxymethylcaproic acid, and mandelic acid; cyclic esters such as glycolide, β-methyl-δ-valerolactone, γ-valerolactone, and ε-caprolactone; polyvalent carboxylic acids such as oxalic acid, malonic acid, succinic acid, glutaric acid, adipic acid, pimelic acid, azelaic acid, sebacic acid, undecanedioic acid, dodecanedioic acid, and terephthalic acid, and anhydrides thereof; polyhydric alcohol such as ethylene glycol, diethylene glycol, triethylene glycol, 1,2-propanediol, 1,3-propanediol, 1,3-butanediol, 1,4-butanediol, 2,3-butanediol, 1,5-pentanediol, 1,6-hexanediol, 1,9-nonanediol, 3-methyl-1, 5-pentanediol, neopentyl glycol, tetramethylene glycol, 1,4-hexanedimethanol; polysaccharides such as cellulose; aminocarboxylic acids such as α-amino acids; and the like.

"Copolymer of L-lactic acid or D-lactic acid and compound copolymerizable with L-lactic acid or D-lactic acid" include a block copolymer or a graft copolymer having a polylactic acid sequence capable of producing helical crystals.

The concentration of the structure derived from the copolymer component in the polylactic acid-based polymer is preferably 20 mol% or less.

For example, the concentration of the structure derived from the copolymer component is preferably 20 mol% or less with respect to the total number of moles of the structure derived from lactic acid and the structure derived from a compound copolymerizable with lactic acid (copolymer component) in the polylactic acid-based polymer.

Examples of a method of manufacturing a polylactic acid-based polymer include a method of directly dehydrating and condensing lactic acid described in Japanese Patent Application Laid-Open (JP-A) No. S59-096123 and Japanese Patent Application Laid-Open (JP-A) No. H07-033861, and a method of ring-opening polymerization using lactide which is a cyclic dimer of lactic acid described in U.S. Patent No. 2,668,182 and the like.

Further, in order to set the optical purity of the polylactic acid-based polymer obtained by each of the above manufacturing methods to 95.00%ee or more, for example, in a case in which polylactic acid is manufactured by the lactide method, it is preferable to polymerize lactide in which the optical purity is improved to 95.00%ee or more by a crystallization operation.

(Animal Protein)

Hereinafter, an animal protein which is an example of the optically active polypeptide (A2) will be described.

Examples of the animal protein include sericin, collagen, keratin, and elastin in addition to the fibroin and spider silk proteins described above.

Among them, the optically active polypeptide (A2) preferably contains at least one of fibroin and spider silk protein, and more particularly preferably consists of at least one of fibroin and spider silk protein.

The spider silk protein is not particularly limited as long as it is derived from or similar to (Hereinafter, these are collectively referred to as "derived".) the natural spider silk protein or the natural spider silk protein.

The "protein derived from the natural spider silk protein" is one having an amino acid sequence same as or similar to the repeated amino acid sequence of the natural spider silk protein. Examples of "those derived from natural spider silk proteins" include recombinant spider silk proteins, variants of natural spider silk proteins, analogs of natural spider silk proteins, and derivatives of natural spider silk proteins.

From the viewpoint of excellent toughness, the spider silk protein is preferably a major dragline silk protein produced in a bulky gland of a spider or a spider silk protein derived from a major dragline silk protein.

Examples of the major dragline silk proteins include MaSp1 or MaSp2, which is a major ampullate gland spidroin derived from Nephila clavipes, and ADF3 or ADF4 derived from Araneus diadematus.

The spider silk protein may be a small spigot dragline protein produced in a minor gland of a spider, or a spider silk protein derived from a small spigot dragline protein.

Examples of the small spigot dragline protein include MiSp1 or MiSp2, which is a minor ampullate gland spidroin derived from Nephila clavipes.

In addition, the spider silk protein may be a weft yarn protein produced in a flagelliform gland of a spider or a spider yarn protein derived from the weft yarn protein.

Examples of the weft yarn protein include flagelliform silk proteins derived from Nephila clavipes.

Examples of the spider silk protein derived from the major dragline silk protein described above include a recombinant spider silk protein containing units of an amino acid sequence represented by the following Formula (2).

The recombinant spider silk protein may contain two or more units of the amino acid sequence represented by the following Formula (2). The number of units of the amino acid sequence is preferably 4 or more, and more preferably 6 or more.

In a case in which the recombinant spider silk protein contains two or more units of the amino acid sequence represented by the following Formula (2), the two or more units of the amino acid sequence may be the same or different.

REP1 - REP2 ... Formula (2)

[In Formula (2), REP1 is a polyalanine region mainly composed of alanine and represented by (X1)p, and REP2 is an amino acid sequence composed of 10 to 200 amino acids.]

In Formula (2), REP1 is a polyalanine region mainly composed of alanine and represented by (X1)p. REP1 is preferably polyalanine.

In (X1)p, p is an integer, is not particularly limited, and is preferably 2 to 20, and more preferably 4 to 12.

In (X1)p, X1 represents alanine (Ala), serine (Ser), or glycine (Gly).

In the polyalanine region represented by (X1)p, the total number of residues of alanine is preferably 80% or more (more preferably 85% or more) of the total number of residues of amino acids in the polyalanine region.

In REP1 in Formula (2), the alanine that is continuously arranged is preferably 2 residues or more, more preferably 3 residues or more, still more preferably 4 residues or more, and particularly preferably 5 residues or more.

In the REP 1 in Formula (2), the alanine that is continuously arranged is preferably 20 residues or less, more preferably 16 residues or less, still more preferably 12 residues or less, and particularly preferably 10 residues or less.

In Formula (2), REP2 is an amino acid sequence consisting of 10 to 200 amino acids. The total number of residues of glycine, serine, glutamine, proline, and alanine contained in this amino acid sequence is preferably 40% or more, more preferably 50% or more, and particularly preferably 60% or more with respect to the total number of amino acid residues.

Examples of the spider silk protein derived from the small dragline silk protein include a recombinant spider silk protein containing an amino acid sequence represented by the following Formula (3).

REP3 - REP4 - REP5 ... Formula (3)

[In Formula (3), REP3 is an amino acid sequence represented by (Gly-Gly-Z)m, REP4 is an amino acid sequence represented by (Gly-Ala)1, and REP5 is an amino acid sequence represented by (Ala)r.]

In REP3, Z means any one amino acid.

In REP 3, m is 1 to 4, in REP 4, 1 is 0 to 4, and in REP 5, r is 1 to 6.]

In REP3, Z means any one amino acid, and is particularly preferably one amino acid selected from the group consisting of Ala, Tyr, and Gln.

The recombinant spider silk protein (for example, a recombinant spider silk protein containing a unit of the amino acid sequence represented by Formula (2), a recombinant spider silk protein containing the amino acid sequence

represented by Formula (3), and the like) described above can be manufactured using a host transformed with an expression vector containing a gene encoding a natural spider silk protein to be recombinant.

The first elongated organic piezoelectric body 121 and the like preferably contain fibers made of an optically active polypeptide (A2) from the viewpoint of piezoelectricity.

Examples of the fiber composed of the optically active polypeptide (A2) include a fiber composed of an animal protein having optical activity. Examples of the fiber made of an animal protein having optical activity include silk, wool, mohair, cashmere, camel, llama, alpaca, bicuna, angora, spider silk, and the like.

From the viewpoint of piezoelectricity, the fiber composed of the optically active polypeptide (A2) preferably contains at least one of silk and spider silk, and more preferably consists of at least one of silk and spider silk.

Examples of the silk include raw silk, refined silk, regenerated silk, and fluorescent silk.

As the silk, raw yarn or refined silk is preferable, and purified silk is particularly preferable.

The refined silk means silk obtained by removing sericin from raw yarn having a double structure of sericin and fibroin, and the refining means an operation of removing sericin from raw yarn. The color of the raw yarn is white without gloss, but by removing the sericin from the raw yarn (that is, refining), the color changes from white without gloss to white silver with gloss. By refining, a soft texture is increased.

From the viewpoint of piezoelectricity, the first elongated organic piezoelectric body 121 and the like preferably contain long fibers (Hereinafter, the "optically active polypeptide fiber" may be referred to.) composed of an optically active polypeptide (A2). This is considered to be because the stress applied to the first piezoelectric sensor 10A is more easily transmitted to the first piezoelectric body 12A in the long fibers than in the short fibers.

The "long fiber" means a fiber having a length that can be continuously wound from one end to the other end in a longitudinal direction of the first piezoelectric sensor 10A.

Silk, wool, mohair, cashmere, camel, llama, alpaca, vicuna, angora, and spider silk all correspond to long fibers. Among the long fibers, silk and spider silk are preferable from the viewpoint of piezoelectricity.

In a case in which the first elongated organic piezoelectric body 121 includes the fibers, the first elongated organic piezoelectric body 121 preferably includes at least one yarn including at least one of the fibers.

Examples of an aspect in which the first elongated organic piezoelectric body 121 includes the yarn include an aspect in which the first elongated organic piezoelectric body 121 includes one yarn, an aspect in which the first elongated organic piezoelectric body 121 is an aggregate of a plurality of the yarns, and the like.

The yarn may be a twisted yarn or a non-twisted yarn, but is preferably a yarn having a twist of 500 T/m or less (that is, a twisted yarn having a twist of 500 T/m or less or a non-twisted yarn (twist of 0 T/m)) from the viewpoint of piezoelectricity.

Examples of the non-twisted yarn include one original yarn and an aggregate of a plurality of the original yarns.

(First Elongated Organic Piezoelectric Body and Second Elongated Organic Piezoelectric Body)

Next, the first elongated organic piezoelectric body 121 and the like will be further described.

< Stabilizer >

The first elongated organic piezoelectric body 121 and the like preferably further contain a stabilizer (B) having one or more functional groups selected from the group consisting of a carbodiimide group, an epoxy group, and an isocyanate group in one molecule and having a weight average molecular weight of from 200 to 60,000. This makes it possible to further improve wet heat resistance.

As the stabilizer (B), the "stabilizer (B)" described in paragraphs 0039 to 0055 of WO 2013/054918 A can be used.

Examples of a compound containing a carbodiimide group in one molecule (carbodiimide compound) that can be used as the stabilizer (B) include a monocarbodiimide compound, a polycarbodiimide compound, and a cyclic carbodiimide compound.

As the monocarbodiimide compound, dicyclohexylcarbodiimide, bis-2,6-diisopropylphenylcarbodiimide, and the like are suitable.

As the polycarbodiimide compound, compounds manufactured by various methods can be used. A polycarbodiimide manufactured by a conventional method of manufacturing a polycarbodiimide (for example, U.S. Patent No. 2941956, Japanese Patent Publication (JP-B) No. S47-33279, J.0rg.Chem.28,2069-2075 (1963), Chemical Review 1981,Vol.81 No.4, p619-621) can be used. Specifically, a carbodiimide compound described in Japanese Patent No. 4084953 can also be used.

Examples of the polycarbodiimide compound include poly (4,4'-dicyclohexylmethanecarbodiimide), poly (N,N'-Di-2,6-diisopropylphenylcarbodiimide), and poly (1,3,5-triisopropylphenylene-2,4-carbodiimide.

The cyclic carbodiimide compound can be synthesized based on the method described in Japanese Patent Application Laid-Open (JP-A) No. 2011-256337 or the like.

As the carbodiimide compound, a commercially available product may be used, and examples thereof include B2756 (product name) manufactured by Tokyo Chemical Industry Co., Ltd., CARBODILITE (registered trademark) LA-1 (product name) manufactured by Nisshinbo Chemical Inc., and Stabaxol P, Stabaxol P400, and Stabaxol I (product names) manufactured by Rhein Chemie GmbH.

Examples of a compound containing an isocyanate group in one molecule (isocyanate compound) that can be used as the stabilizer (B) include 3-(triethoxysilyl) propyl isocyanate, 2,4-tolylene diisocyanate, 2,6-tolylene diisocyanate, m-phenylene diisocyanate, p-phenylene diisocyanate, 4,4'-diphenylmethane diisocyanate, 2,4'-diphenylmethane diisocyanate, 2,2'-diphenylmethane diisocyanate, xylylene diisocyanate, hydrogenated xylylene diisocyanate, and isophorone diisocyanate.

Examples of a compound containing an epoxy group in one molecule (epoxy compound) that can be used as the stabilizer (B) include phenyl glycidyl ether, diethylene glycol diglycidyl ether, bisphenol A-diglycidyl ether, hydrogenated bisphenol A-diglycidyl ether, a phenol novolac-type epoxy resin, a cresol novolac-type epoxy resin, and epoxidized polybutadiene.

The weight average molecular weight of the stabilizer (B) is from 200 to 60,000, preferably from 200 to 30,000, more preferably from 300 to 18000, and particularly preferably from 200 to 900.

When the molecular weight is within the above range, the stabilizer (B) more easily moves, and the wet heat resistance is improved.

Note that the weight average molecular weight of from 200 to 900 substantially coincides with the number average molecular weight of from 200 to 900. In a case in which the weight average molecular weight is from 200 to 900, the molecular weight distribution may be 1.0, and in this case, "the weight average molecular weight of from 200 to 900" can be simply paraphrased as "the molecular weight of from 200 to 900".

In a case in which the first elongated organic piezoelectric body 121 and the like contain the stabilizer (B), the first elongated organic piezoelectric body 121 and the like may contain only one kind of the stabilizer or two or more kinds thereof.

In a case in which the first elongated organic piezoelectric body 121 and the like contain the stabilizer (B), the content of the stabilizer (B) is preferably from 0.01 parts by mass to 10 parts by mass, more preferably from 0.01 parts by mass to 5 parts by mass, still more preferably from 0.1 parts by mass to 3 parts by mass, and particularly preferably from 0.5 parts by mass to 2 parts by mass with respect to 100 parts by mass of the helical chiral polymer (A1).

When the content of the stabilizer (B) is 0.01 parts by mass or more, the wet heat resistance is further improved.

When the content is 10 parts by mass or less, deterioration of transparency is further suppressed.

As a preferred aspect of the stabilizer (B), there is an aspect in which a stabilizer (B1) having one or more functional groups selected from the group consisting of a carbodiimide group, an epoxy group, and an isocyanate group and having a number average molecular weight of from 200 to 900 and a stabilizer (B2) having two or more functional groups in one molecule selected from the group consisting of a carbodiimide group, an epoxy group, and an isocyanate group and having a weight average molecular weight of from 1000 to 60,000 are used in combination. Note that the weight average molecular weight of the stabilizer (B 1) having a number average molecular weight of from 200 to 900 is approximately from 200 to 900, and the number average molecular weight and the weight average molecular weight of the stabilizer (B1) are substantially the same value.

In a case in which the stabilizer (B1) and the stabilizer (B2) are used in combination as the stabilizer, it is preferable to contain a large amount of the stabilizer (B 1) from the viewpoint of improving transparency.

Specifically, the amount of the stabilizer (B2) is preferably in a range of from 10 parts by mass to 150 parts by mass with respect to 100 parts by mass of the stabilizer (B1) from the viewpoint of achieving both transparency and wet heat resistance, and more preferably in a range of from 50 parts by mass to 100 parts by mass.

Hereinafter, specific examples (stabilizers (B-1) to (B-3)) of the stabilizer (B) will be described.

[Chemical Formula 2]

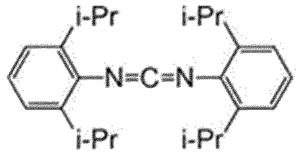

B-1

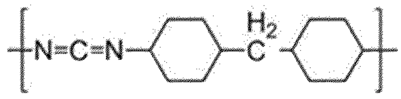

B-2

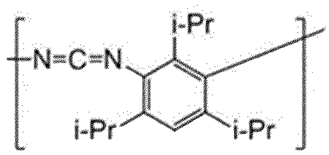

B-3

Hereinafter, compound names, commercially available products, and the like of the stabilizers (B-1) to (B-3) are shown.

· Stabilizer (B-1) ... Compound name is bis-2,6-diisopropylphenylcarbodiimide. The weight average molecular weight (In this example, it is simply equal to "molecular weight".) is 363. Examples of the commercially available products include "Stabaxol I" manufactured by Rhein Chemie GmbH and "B2756" manufactured by Tokyo Chemical Industry Co., Ltd.
· Stabilizer (B-2) ... Compound name is poly (4,4'-dicyclohexylmethanecarbodiimide). Examples of the commercially available product having a weight average molecular weight of about 2000 include "CARBODILITE (registered trademark) LA-1" manufactured by Nisshinbo Chemical Inc.
· Stabilizer (B-3) ... Compound name is poly (1,3,5-triisopropylphenylene-2,4-carbodiimide). Examples of the commercially available products having a weight average molecular weight of about 3000 include "Stabaxol P" manufactured by Rhein Chemie GmbH. Examples of those having a weight average molecular weight of 20,000 include "Stabaxol P400" manufactured by Rhein Chemie GmbH.

< Other Components >

The first elongated organic piezoelectric body 121 and the like may contain other components as necessary.
Examples of the other components include known resins such as polyvinylidene fluoride, polyethylene resin, and polystyrene resin, known inorganic fillers such as silica, hydroxyapatite, and montmorillonite, known crystal nucleating agents such as phthalocyanine; stabilizers other than the stabilizer (B); and the like.
Examples of the inorganic filler and the crystal nucleating agent can also include the components described in paragraphs 0057 and 0058 of WO 2013/054918 A.

(Orientation Degree F)

The orientation degree F of the first elongated organic piezoelectric body 121 or the like is preferably from 0.5 to less than 1.0, more preferably from 0.7 to less than 1.0, and still more preferably from 0.8 to less than 1.0.
When the orientation degree F of the first elongated organic piezoelectric body 121 or the like is 0.5 or more, there are many molecular chains (for example, polylactic acid molecular chains) of the helical chiral polymer (A1) arranged in the stretching direction. As a result, a rate of generation of oriented crystals is increased, and the piezoelectricity is improved.
When the orientation degree F of the first elongated organic piezoelectric body 121 or the like is less than 1.0, the longitudinal tear strength is further improved.

(Crystallinity)

The crystallinity of the first elongated organic piezoelectric body 121 or the like is a value measured by the above-described X-ray diffraction measurement (wide-angle X-ray diffraction measurement).

The crystallinity of the first elongated organic piezoelectric body 121 or the like is preferably from 20% to 80%, more preferably from 25% to 70%, and still more preferably from 30% to 60%.

When the crystallinity is 20% or more, high piezoelectricity is maintained. When the crystallinity is 80% or less, the transparency of the first elongated organic piezoelectric body 121 or the like is maintained high.

When the crystallinity is 80% or less, for example, whitening or breakage hardly occurs when an organic piezoelectric film to be a raw material of the first elongated organic piezoelectric body 121 or the like is manufactured by stretching. Therefore, the first elongated organic piezoelectric body 121 or the like can be easily manufactured. When the crystallinity is 80% or less, for example, when a raw material (for example, polylactic acid) such as the first elongated organic piezoelectric body 121 is manufactured by melt-spinning and then stretching, fibers having high flexibility and supple properties are obtained. Therefore, the first elongated organic piezoelectric body 121 and the like can be easily manufactured.

< Application of Piezoelectric Device >

The piezoelectric device 1 of the present embodiment can be used for, for example, a sensor application (a force sensor such as a seating sensor, a pressure sensor, a displacement sensor, a deformation sensor, a vibration sensor, an ultrasonic sensor, a biometric sensor, a racket, a golf club, an impact sensor, or the like at the time of striking various sports tools for ball games such as a bat, a touch/impact sensor of a stuffed toy, a watching sensor of a bed, a security sensor such as glass or a window frame, or the like), an actuator application (a sheet conveying device or the like), an energy harvesting application (power generation wear, power generation shoes, and the like), a healthcare related application (a wearable sensor or the like provided with the present sensor in various clothes such as T-shirts, sportswear, spats, and socks, supporters, plaster, diapers, a seat of a baby cart, a seat for a wheelchair, a mat of a medical incubator, shoes, an insole of shoes, a watch, and the like.), and the like.

Examples of the arrangement method include various methods such as sandwiching the first piezoelectric sensor 10A and the second piezoelectric sensor 10B with an object, and fixing the first piezoelectric sensor and the second piezoelectric sensor to the object with an adhesive.

Among the above applications, the piezoelectric device 1 of the present embodiment is preferably used as a sensor application.

Specifically, the piezoelectric device 1 of the present embodiment is preferably mounted on a force sensor and used.

The piezoelectric device 1 can switch a field effect transistor (FET) by applying a voltage generated by stress between a gate and a source of the FET, and can also be used as a switch that can be turned on and off by stress.

The first piezoelectric sensor 10A and the second piezoelectric sensor 10B of the present embodiment are, for example, suitable for automotive applications, and are particularly suitable for automobile handle grip detection using vibration/acoustic sensing, vehicle-mounted equipment operation system using resonance spectrum using vibration/acoustic sensing, touch sensor application of vehicle-mounted display, vibrating body application, sandwiching detection sensor application of automobile door and automobile window, vehicle body vibration sensor application, and the like.

Hereinafter, a configuration of a force sensor including the piezoelectric device 1 according to the present embodiment will be described with reference to the drawings.

Fig. 11 is a conceptual diagram of a force sensor according to the present embodiment. In Fig. 11, a second unit to be described later is omitted.

A force sensor 50 according to the present embodiment includes a first unit and a second unit. The first unit and the second unit are disposed in parallel. The first unit includes the first piezoelectric sensor 10A. The second unit includes the second piezoelectric sensor 10B.

The first unit includes a cylindrical rubber-based heat-shrinkable tube (Hereinafter, it is also simply referred to as a "shrinking tube".) 51 as a second insulator, the first piezoelectric sensor 10A disposed inside the shrinking tube 51, and a pair of crimp terminals (extraction electrodes) 52 disposed at both end parts of the shrinking tube 51. The pair of crimp terminals 52 includes a main body part 521 and a crimping part 522, and has a through hole 523 at a center. The first piezoelectric sensor 10A includes the first inner conductor 11A, the second piezoelectric body 12A spirally wound in one direction around the first inner conductor 11A, and a first outer conductor 13A spirally wound in one direction around the outer peripheral surface of the first piezoelectric body 12A.

In the first piezoelectric sensor 10A, one end (right end in Fig. 11) of the first inner conductor 11A extends to the outside of the shrinking tube 51, is crimped by the crimping part 522, and is electrically connected to the crimp terminal 52. On the other hand, the first outer conductor 13A is wound from one end side toward the other end side of the first inner conductor 11A, and then extends beyond the other end (left end in Fig. 11) of the first inner conductor 11A, and an extending part thereof forms a stress relieving part 53 in the shrinking tube 51.

After passing through the stress relieving part 53, the first outer conductor 13A extends further outside the

shrinking tube 51 (left end in Fig. 11) and is crimped by the crimping part 522 to be electrically connected to the crimp terminal 52.

As illustrated in Fig. 7, the stress relieving part 53 is made of the slackened first outer conductor 13A. In the stress relieving part 53, when tension (stress) is applied to the force sensor 50, an excessive force is suppressed from being loaded on the first piezoelectric body 12A due to extension of the slack part.

The first piezoelectric body 12A is made of the elongated organic piezoelectric body 121, and an aluminum vapor deposition film (not illustrated) is vapor-deposited as a functional layer on both surfaces. Note that the pair of crimp terminals 52 is connected to an external circuit or the like (not illustrated) that processes an output signal of the force sensor 50.

Note that, in the embodiment illustrated in Fig. 11, the slackened first outer conductor 13A is disposed as the stress relieving part 53, but the embodiment of the present disclosure is not limited thereto, and a function of relieving stress may be imparted to the force sensor 50 by disposing a linear stress relieving part at least one end part or both end parts of the first piezoelectric sensor 10A such that tension is transmitted by a method such as adhesion or a yarn knot.

At this time, the linear stress relieving part does not have a function of electrical connection, but the electrical connection function can detect a voltage signal of stress or distortion by connecting the first inner conductor 11A and the first outer conductor 13B to a coaxial cable or the like from the end part of the first piezoelectric sensor 10A independently of the stress relieving part.

At this time, the material and form of the stress relieving part are not particularly limited, and for example, a thread, a string, a tube, or the like made of a stretchable elastic material such as natural rubber, silicon rubber, or urethane rubber, and a spring made of metal material such as phosphor bronze, linear polymer, and the like. By independently disposing the stress relieving part and an electrical connection part at different locations, the restriction of the strain amount of the stress relieving part caused by the maximum elongation amount of the electrical connection part is eliminated, and the maximum strain amount as the tension sensor can be increased.

Hereinafter, an operation of the force sensor 50 of the present embodiment will be described.

When tension (stress) is applied to the force sensor 50, tension is applied to the first piezoelectric sensor 10A, a shear force is applied to the helical chiral polymer (A1) contained in the first piezoelectric body 12A of the first piezoelectric sensor 10A, and this shear force causes polarization of the helical chiral polymer (A1) in the radial direction of the first piezoelectric sensor 10A. The polarization direction is a radial direction of the first piezoelectric sensor 10A. As a result, a charge (electric field) proportional to the tension is generated, and the generated charge is detected as a voltage signal (charge signal). Note that the voltage signal is detected by an external circuit or the like (not illustrated) connected to the crimp terminal 52.

Since the force sensor 50 of the present embodiment includes the first piezoelectric sensor 10A having the same structure as the internal structure included in the coaxial cable, the force sensor has a high electromagnetic shielding property and can have a structure resistant to noise. In addition, since the structure is simple, it can be used by being worn on a part of the body, for example, as a wearable sensor.

The second unit has the same configuration as the first unit except that the second piezoelectric body 12A is used instead of the first piezoelectric body 12A.

The force sensor of the present embodiment is not limited to a configuration in which charges (electric fields) generated when tension is applied to the first piezoelectric sensor 10A and the second piezoelectric sensor 10B are extracted as a voltage signal, and for example, a configuration in which charges (electric fields) generated when torsional force is applied to the first piezoelectric sensor 10A and the second piezoelectric sensor 10B are extracted as a voltage signal may be adopted.

As an application of the piezoelectric device 1 of the present embodiment, a biological information acquisition device is also preferable.

That is, the biological information acquisition device of the present embodiment includes the piezoelectric device 1 of the present embodiment.

The biological information acquisition device of the present embodiment is a device for acquiring biological information of a subject by detecting a biological signal of the subject or a test animal (Hereinafter, these are also collectively referred to as a "subject".) by the piezoelectric device 1.

Examples of the biological signal here include a pulse wave signal (heart rate signal), a respiration signal, a body motion signal, a ballistocardiogram, and a biological tremor.

The biological tremors are rhythmic involuntary movements of body parts (fingers, hands, forearms, upper limbs, etc.).

The detection of the ballistocardiologic motion also includes detection of the effect of force by the heart function of the body.

That is, when the heart pumps blood into the aorta and pulmonary arteries, the body experiences a reaction in an opposite direction to a blood flow. The magnitude and direction of this reaction change with the functional phase of the heart. This reaction force is detected by sensing the ballistocardiologic motion outside the body.

The biological information acquisition device is used by being disposed at various articles such as various clothes (shirts, suits, blazers, blouses, coats, jackets, blousons, jumpers, vests, dresses, trousers, pants, underwear (slip, petticoat, camisole, brassiere), socks, gloves, Japanese clothing, obi fabric, gold brocade, cool clothing, ties, hangers, mufflers, scarves, stalls, eye masks), supporters (neck supporter, shoulder supporter, chest supporter, abdomen supporter, waist supporter, arm supporter, foot supporter, elbow supporter, knee supporter, wrist supporter, ankle supporter), footwear (sneakers, boots, sandals, pumps, mules, slippers, ballet shoes, Kung Fu shoes), insoles, towels, backpacks, hats (hat, cap, casket, hunting hat, tengalloon hat, tulip hat, sun visor, beret hat), helmets, helmet chin straps, hoods, belts, seat covers, sheets, cushions, cushions, futons, futon covers, blankets, pillows, pillow covers, sofas, chairs, desks, tables, seats, toilet seats, massage chairs, beds, bed pads, carpets, carpets, baskets, masks, bandages, ropes, various nets, bathtubs, floor materials, wall materials, personal computers, and mice.

As the article on which the biological information acquisition device is disposed, articles to which the weight of the subject is applied, such as footwear, insole, sheet, cushion, cushion, futon, futon cover, pillow, pillow cover, sofa, chair, seat, seat, toilet seat, bed, carpet, bathtub, and floor material, are preferable. More specifically, a seat, a seat part, a wheel, a stopper for preventing an infant from falling, and the like of a hand cart for infants, a seat, a seat part, and the like for a wheelchair, a mat of a medical incubator, and the like are preferable.

Hereinafter, an example of an operation of the biological information acquisition device will be described.

The biological information acquisition device is disposed at, for example, a bed or a seat surface of a chair. The subject lies down, sits down, or stands up on the biological information acquisition device. In this state, when tension is applied to the piezoelectric device 1 of the biological information acquisition device by a biological signal (heart rate or the like that has changed due to body motion, periodic vibration (pulse, respiration, etc.), or human sensitivity such as "cute" or "scaring") emitted from the subject, polarization occurs in the helical chiral polymer (A1) included in the piezoelectric device 1, and a potential proportional to the tension is generated. This potential changes over time according to the biological signal emitted from the subject. For example, in a case in which the biological signal emitted from the subject is periodic vibration such as pulse and respiration, the potential generated in the piezoelectric device 1 also periodically changes.

The temporal change of the potential generated with the application of the tension to the piezoelectric device 1 is acquired as a voltage signal by a measurement module. The temporal change of the acquired potential (piezoelectric signal) is a composite wave of a plurality of biological signals (pulse wave signal (heartbeat signal), respiration signal, body motion signal). The synthesized wave is separated for each frequency by Fourier transform to generate separation signals. By performing inverse Fourier transform on each of the generated separation signals, a biological signal corresponding to each of the separation signals is obtained.

For example, in a case in which the biological signal emitted from the subject is a composite wave of a heartbeat signal and a respiration signal, the potential generated with the application of the tension to the piezoelectric device 1 of the biological information acquisition device periodically changes over time.

In general, pulse of a person is from 50 times to 90 times per minute, and a cycle is from 0.6 Hz to 3 Hz. Generally, human respiration is from 16 times to 18 times per minute, and a cycle is from 0.1 Hz to 1 Hz. Generally, the body motion of a person is 10 Hz or more.

Based on these criteria, the composite wave of the plurality of biological signals can be separated into the respective biological signals. Further, a signal of a velocity pulse wave can be obtained from the heartbeat signal.

The separation of the composite wave of the plurality of biological signals into the respective biological signals is performed by the Fourier transform and the inverse Fourier transform using, for example, a biological signal notification program.

As described above, the composite wave of the plurality of biological signals can be separated into each of the plurality of biological signals.

Further, the biological signal data may be generated on the basis of at least one of the biological signals separated as described above.

The biological signal data is not particularly limited as long as the biological signal data is calculated on the basis of the biological signal. Examples of the biological signal data include the number of biological signals per unit time and an average value of the number of past biological signals.

[Examples]

Hereinafter, embodiments according to the present disclosure will be described in detail with reference to Examples. Note that the present disclosure is not limited to the descriptions of these examples at all.

[Example 1]

(Preparation of First Piezoelectric Sensor)

10 parts by mass of a stabilizer A described below, 70 parts by mass of a stabilizer B described below, and 20 parts by mass of a stabilizer C described below were mixed to obtain a stabilizer D. Next, 1.0 parts by mass of the stabilizer D was added to 100 parts by mass of the following polylactic acid, and dry-blended to prepare a raw material.

As the polylactic acid, the stabilizer A, the stabilizer B, and the stabilizer C, the following products were used.

Polylactic acid: "Ingeo (trademark) biopolymer 4032D" (helical chiral polymer (A1) manufactured by NatureWorks LLC)
Stabilizer A: "Stabaxol P400" (manufactured by Rhein Chemie GmbH, weight average molecular weight: 20,000)
Stabilizer B: "Stabaxol I" (manufactured by Rhein Chemie GmbH, weight average molecular weight: 363)
Stabilizer C: "CARBODILITE (registered trademark) LA-1" (manufactured by Nisshinbo Chemical Inc., weight average molecular weight: 2000)

The prepared raw material was placed in an extruder hopper, extruded from a T-die while being heated to 210°C, and brought into contact with a cast roll at 50°C for 0.3 minutes to form a preliminarily crystallized sheet having a thickness of 150 μm (preliminary crystallization step). The obtained preliminarily crystallized sheet was started to be stretched by a roll-to-roll method at a stretching speed of 10 m/min while being heated to 70°C, and uniaxially stretched up to 3.5 times in a sheet flow direction (MD: Machine Direction) (stretching step).

Thereafter, the uniaxially stretched film was brought into contact with a roll heated to 145°C for 15 seconds by the roll-to-roll method and annealed, and quenched to produce an organic piezoelectric film (annealing treatment step).

Then, the organic piezoelectric film was slit using a slit processing machine so that a slitting direction of the organic piezoelectric film and a stretching direction of the organic piezoelectric film were substantially parallel to obtain a slit ribbon (first elongated organic piezoelectric body 121) of the organic piezoelectric film having a width of 0.6 mm and an average thickness of 50 μm.

Whether or not the obtained slit ribbon has the "piezoelectric constant $d_{14}$" was determined by the above-described first determination method, and it was found that the slit ribbon has the piezoelectric constant $d_{14}$.

(Measurement of Orientation Degree F of First Elongated Organic Piezoelectric Body 121)

The first elongated organic piezoelectric body 121 was fixed to a holder using a wide-angle X-ray diffractometer (RINT2550 manufactured by Rigaku Corporation, attached device: rotating sample stage, X-ray source: CuKα, output: 40 kV 370 mA, detector: scintillation counter), and an azimuth angle distribution intensity of a crystal plane peak [(110) plane/(200) plane] was measured.

In the obtained azimuthal angle distribution curve (X-ray interference diagram), the orientation degree F (degree of C-axis orientation) of the first elongated organic piezoelectric body 121 was calculated and evaluated from a degree of crystallinity and a half-value width (α) of the peak by the following formula. The orientation degree F of the first elongated organic piezoelectric body 121 was 0.97.

$$\text{Orientation degree (F)} = (180° - \alpha)/180°$$

(α is a half-value width of the peak derived from orientation.)

The first piezoelectric sensor 10A in which the winding direction of the slit ribbon of the organic piezoelectric film was left winding and the second piezoelectric sensor 10B in which the winding direction of the slit ribbon of the organic piezoelectric film was right winding were prepared as follows.

As the first inner conductor 11A, a tinsel wire having a radius of 0.135 mm and a length of 850 mm was prepared. As the tinsel wire, a polyester thread around which a copper foil was wound right-handed was used. The slit ribbon of the obtained organic piezoelectric film was spirally wound around the outer peripheral surface of the tinsel wire left-handed at an angle of 45° (spiral angle) with respect to a long axis direction of the tinsel wire without a gap such that the tinsel wire was not exposed. As a result, a coaxial line structure including the tinsel wire and the slit ribbon (first piezoelectric body 12A) was obtained. The number of times of winding of the slit ribbon around the tinsel wire at this time was 17 times/cm.

Next, a copper foil ribbon slit-cut into a width of 0.6 mm was prepared as the first outer conductor 13A. The copper foil ribbon was wound on the outer peripheral surface of the coaxial line structure without any gap such that the slit ribbon (first piezoelectric body 12A) of the organic piezoelectric film was not exposed by the same method as the

slit ribbon of the organic piezoelectric film described above. In this way, the first piezoelectric sensor 10A having a length of 800 mm was obtained.

(Preparation of Second Piezoelectric Sensor)

The second piezoelectric sensor 10B having a length of 800 mm was obtained in a similar manner to the first piezoelectric sensor 10A except that the winding direction of the copper foil of the tinsel wire, the winding direction of the slit ribbon of the organic piezoelectric film, and the winding direction of the copper foil ribbon were reversed from those of the first piezoelectric sensor 10A. Specifically, in the second piezoelectric sensor 10B, the winding direction of the copper foil of the tinsel wire is left winding, the winding direction of the slit ribbon of the organic piezoelectric film is right winding, and the winding direction of the copper foil ribbon is left winding.

As described above, the first piezoelectric sensor 10A and the second piezoelectric sensor 10B were obtained.

In the first piezoelectric sensor 10A, the slit ribbon of the organic piezoelectric film is wound left-handed in the first direction D1. In the second piezoelectric sensor 10B, the slit ribbon of the organic piezoelectric film is wound right-handed in the first direction D1.

An average radius of the tinsel wire and an average thickness of the slit ribbon of each of the first piezoelectric sensor 10A and the second piezoelectric sensor 10B were measured by the method described above. In both the first piezoelectric sensor 10A and the second piezoelectric sensor 10B, the average radius of the tinsel wire was 0.135 mm, and the average thickness of the slit ribbon was 0.05 mm.

< Production of Measurement Device >

A measurement device 100 was manufactured using the first piezoelectric sensor 10A and the first piezoelectric sensor 10B. Fig. 12 is a top view of the measurement device 100 and a vibration generator 200 of Example 1. Fig. 13 is a cross-sectional view taken along line XIII in Fig. 12. Fig. 14 is a cross-sectional view taken along line XIV in Fig. 12. Fig. 15 is a side view of the measurement device 100 and the vibration generator 200 of Example 1 as viewed from a direction D4 in Fig. 12.

Hereinafter, a length in a front-back direction (see Figs. 12 and 15) is referred to as "length", a length in a left-right direction (see Figs. 12 to 14) is referred to as "width", and a length in an up-down direction (see Figs. 13 to 15) is referred to as "thickness".

As illustrated in Fig. 13, first, a lower buffer layer 120 made of a foamed resin having a length of 800 mm, a width of 20 mm, and a thickness of 5 mm, to which a double-sided tape 101 was attached, was attached to an upper surface of an aluminum support plate 110 having a length of 850 mm, a width of 30 mm, and a thickness of 5 mm. Next, an upper buffer layer 130 made of a foamed resin having a length of 800 mm, a width of 5 mm, and a thickness of 5 mm and having double-sided tapes 101 attached to both surfaces of an upper surface and a lower surface was attached to an upper surface of the lower buffer layer 120. Next, a lower insulating layer 140 made of a polyimide tape having a thickness of 25 $\mu$m was attached to an upper surface of the upper buffer layer 130 via the double-sided tape 101. The first outer conductor 13A of the first piezoelectric sensor 10A and the second outer conductor 13B of the second piezoelectric sensor 10B were laterally attached to a central part of the upper surface of the lower insulating layer 140 in a width direction (left-right direction in Fig. 13) in a state of being in contact with each other. Next, an upper insulating layer 150 made of a polyimide tape having a thickness of 25 $\mu$m was attached to the upper parts of the first piezoelectric sensor 10A, the second piezoelectric sensor 10B, and the lower insulating layer 140. As a result, the measurement device 100 was obtained.

The first piezoelectric sensor 10A was electrically connected to a 2 m first coaxial cable 20A with a jack connector. The second piezoelectric sensor 10B was electrically connected to a 2 m second coaxial cable 20B with a jack connector.

Specifically, a female connector on a central axis side of the first coaxial cable 20A and an end part on a back side (see Fig. 12) of the first inner conductor 11A of the first piezoelectric sensor 10A were soldered, and an outer line of the female connector of the first coaxial cable 20A and an end part on the back side (see Fig. 12) of the first outer conductor 13A of the first piezoelectric sensor 10A were soldered. Similarly, a female connector on a central axis side of the second coaxial cable and an end part on the back side (see Fig. 12) of the first inner conductor 11A of the second piezoelectric sensor 10B were soldered, and an outer line of the female connector of the second coaxial cable and an end part on the back side (see Fig. 12) of the second outer conductor 13B of the second piezoelectric sensor 10B were soldered.

Next, each of the first piezoelectric sensor 10A and the second piezoelectric sensor 10B was electrically connected to the instrumentation amplifier 30 housed in a metal housing (GND). "INA128" (manufactured by Texas Instruments Inc.) was used as the instrumentation amplifier 30. In "INA128", in Fig. 4, the resistors $R_2$ and $R_3$ are 25 kQ, and the resistors $R_4$ to $R_8$ are 40 kQ. A circuit having an amplification factor of 2 was formed by connecting 50 k$\Omega$ to the resistor $R_1$.

The first conductive wire 21A of the first coaxial cable 20A was connected to the first differential input terminal $V_{IN}^-$ of the instrumentation amplifier 30. The second conductive wire 21B of the second coaxial cable 20B was connected to the second differential input terminal $V_{IN}^+$ of the instrumentation amplifier 30. The first conductor layer 23A of the first coaxial cable 20A was connected to the reference terminal $V_{ref}$ of the instrumentation amplifier 30, and the second conductor layer 23B of the second coaxial cable 20B was connected to the metal housing (GND) accommodating the instrumentation amplifier 30.

That is, the first inner conductor 11A of the first piezoelectric sensor 10A is electrically connected to the first differential input terminal $V_{IN}^-$ of the instrumentation amplifier 30. The second inner conductor 11B of the second piezoelectric sensor 10B was electrically connected to the second differential input terminal $V_{IN}^+$ of the instrumentation amplifier 30. The first outer conductor 13A of the first piezoelectric sensor 10A and the second outer conductor 13B of the second piezoelectric sensor 10B were electrically connected to the reference terminal $V_{ref}$ of the instrumentation amplifier 30.

Next, the output terminal $V_{OUT}$ of the instrumentation amplifier 30 was connected to a voltmeter, and a differential voltage from the measurement device 100 was detected using the vibration generator 200.

Sensor sensitivity and noise measurement were performed by fixing a rotation shaft 211 of a rotary motor 210 at a position shifted from a central axis BX of a cylindrical rotor 220 made of brass, and applying vibration caused by eccentric rotation of the cylindrical rotor 220 to the measurement device 100.

Specifically, drilling was performed at a position shifted by 4.5 mm from the central axis BX of the cylindrical rotor 220 having an outer diameter of 15 mm and a length of 31.2 mm, and the rotation shaft 211 of the rotary motor 210 ("SYNCHRONOUS MOTER TYPED-5" manufactured by NIDEC CORPORATION) was inserted into this hole and fixed. The rotary motor 210 was screwed and fixed to a fixing jig 230.

The fixing jig 230 has a U-shape and is composed of three acrylic plates made of acrylic having a thickness of 10 m. The acrylic plates 231 of 75 mm × 75 mm were disposed above and below the fixing jig 230, the central part of the acrylic plate 232 of 75 mm × 55 mm used vertically was hollowed out, the rotary motor 210 was fixed, and the ends of the two upper and lower acrylic plates 231 and the ends of the acrylic plate 232 were screwed and fixed together in a U-shape. The cylindrical rotor 220 was placed in an upper part in the vicinity of the substantially central part in the left-right direction (see Fig. 12) of the measurement device 100 so that the longitudinal direction of the cylindrical rotor was perpendicular to each of the first piezoelectric sensor 10A and the second piezoelectric sensor 10B, placed along the first piezoelectric sensor 10A and the second piezoelectric sensor 10B of the measurement device 100 under the acrylic plate 231 on the side on which the rotary motor 210 of the fixing jig 230 was fixed, and a foot 233 was attached to the lower side of the lower acrylic plate 232 on the side on which the rotation motor 210 was not fixed so that the acrylic plate 231 was adjusted in height and fixed so as to be parallel to the lower surface. In addition, a metal weight 240 was placed on the upper side of the fixing jig 230, and a load was applied to perform the measurement. The metal weight 240 had a donut shape with an outer diameter of 100 mm, an inner diameter of 40 mm, and a thickness of 17 mm, and had a weight of 865 g.

After the setting was completed, since the commercial power supply was in an area of 60 Hz, the vibration at 1.2 Hz generated by rotating the rotary motor 210 was transmitted to the measurement device 100, the signal obtained from each of the first piezoelectric sensor 10A and the second piezoelectric sensor 10B was input to the instrumentation amplifier 30, the voltage of the output of the instrumentation amplifier 30 was measured using "USB-6001" manufactured by Nathional Instrument Inc., data was taken into a personal computer (PC), the voltage signal was analyzed by fast Fourier transform (FFT), and frequency characteristics were evaluated.

FFT analysis was performed using the spectrum measurement configuration ExpressVI of LabView under the conditions of amplitude measurement, a Hanning window, the number of samples: 32768, and a rate: 1 kHz. Fig. 16 illustrates a measurement result of FFT analysis of Example 1.

The noise level was evaluated as hum noise at a commercial power supply frequency (60 Hz). The signal voltage level at 60 Hz was 0.027 V as illustrated in Fig. 16.

The evaluation target of the sensor sensitivity was a voltage signal level of a peak in the vicinity of 1.2 Hz to which vibration was applied. The voltage signal level at the peak near 1.2 Hz was 0.021 V as illustrated in Fig. 16.

[Example 2]

A measurement device 100 was manufactured in the same manner as in Example 1 except that the first outer conductor 13A of the first piezoelectric sensor 10A and the second outer conductor 13B of the second piezoelectric sensor 10B were laterally attached to each other with a distance of about 2 mm in the central part of the upper surface of the lower insulating layer 140 in the width direction (left-right direction in Fig. 13).

In the same manner as in Example 1, hum noise and sensor sensitivity at 60 Hz were measured. The measurement results are shown in Table 1.

[Comparative Example 1]

A measurement device 100 was manufactured in a similar manner to Example 1 except that the second piezoelectric sensor 10B of Example 1 was used instead of the first piezoelectric sensor 10A. That is, in the measurement device 100 of Comparative Example 1, two second piezoelectric sensors 10B are disposed.

Fig. 17 illustrates a measurement result of FFT analysis in the same manner as in Example 1 in Comparative Example 1. The measurement results of Comparative Example 1 of hum noise and sensor sensitivity at 60 Hz are shown in Table 1.

[Comparative Example 2]

A measurement device 100 was manufactured in a similar manner to Example 1 except that the second inner conductor 11B of the second piezoelectric sensor 10B was not electrically connected to the second differential input terminal $V_{IN}^+$ of the instrumentation amplifier 30. Specifically, the first inner conductor 11A of the first piezoelectric sensor 10A was electrically connected to the first differential input terminal $V_{IN}^-$ of the instrumentation amplifier 30, and the metal housing (GND) housing the instrumentation amplifier 30 was connected to the second differential input terminal $V_{IN}^+$ of the instrumentation amplifier 30.

Fig. 18 illustrates a measurement result of FFT analysis in the same manner as in Example 1 in Comparative Example 2. The measurement results of Comparative Example 2 of hum noise and sensor sensitivity at 60 Hz are shown in Table 1.

[Comparative Example 3]

Hum noise and sensor sensitivity at 60 Hz were measured in a similar manner to Example 1 except that the configuration of the second piezoelectric sensor 10B was changed to the configuration of the first piezoelectric sensor 10A of Example 1, the first conductive wire 21A of the first coaxial cable 20A was connected to the metal housing (GND), and the first conductor layer 23A of the first coaxial cable 20A was connected to the first differential input terminal $V_{IN}^-$ of the instrumentation amplifier 30. The measurement results are shown in Table 1.

In Comparative Example 3, the first inner conductor 11A of the first piezoelectric sensor 10A is electrically connected to the metal housing (GND), and the first outer conductor 13A of the first piezoelectric sensor 10A is electrically connected to the first differential input terminal $V_{IN}^-$ of the instrumentation amplifier 30.

[Example 3]

< Preparation of First Elongated Organic Piezoelectric Body 121 >

As an organic piezoelectric material of the first elongated organic piezoelectric body 121, raw silk was prepared. Raw silk is a long fiber composed of an optically active polypeptide. The raw silk had a denier of 21. A thickness of the raw silk was 0.06 mm to 0.04 mm.

When whether or not the raw silk has the "piezoelectric constant $d_{14}$" was determined by the first determination method described above using a ribbon-shaped raw silk cured product, it was found that the raw silk has the piezoelectric constant $d_{14}$.

The cured product of raw silk was a ribbon-shaped product, and was obtained by impregnating four raw silk threads with a cyanoacrylate-based adhesive ("201" manufactured by Toagosei Co., Ltd.) and curing the cyanoacrylate-based adhesive. The four raw silk threads are arranged in parallel in a direction orthogonal to the length direction of the raw silk threads, and are arranged such that adjacent raw silk threads are in contact with each other.

(Measurement of Orientation Degree F of Optically Active Polypeptide Fiber)

Using a wide-angle X-ray diffractometer ("RINT2550" manufactured by Rigaku Corporation, attached device: rotating sample stage, X-ray source: CuK$\alpha$, output: 40 kV 370 mA, detector: scintillation counter), raw silk (optically active polypeptide fiber) was fixed to a holder, and an azimuth angle distribution intensity of a crystal plane peak [$2\theta$ = 20°] was measured.

In the obtained azimuth angle distribution curve (X-ray interference diagram), the orientation degree F (c-axis orientation degree) of raw silk (optically active polypeptide fiber) was calculated from the half-value width ($\alpha$) of the peak by the following Formula (a).

The orientation degree F of the optically active polypeptide fiber was 0.91.

$$\text{Orientation degree (F)} = (180° - \alpha)/180° \ldots (a)$$

($\alpha$ is a half-value width of the peak derived from orientation.)

< Production of Piezoelectric Base Material >

As the first inner conductor 11A, a tinsel wire "U24-01-00" (wire diameter: 0.26 mm, length: 200 mm) manufactured by Meisei Sangyo Co., Ltd. was prepared.

A raw silk (first elongated organic piezoelectric body 121) was wound on the outer peripheral surface of the first inner conductor 11A by left winding with as little gap as possible so that the spiral angle was about 45°. As a result, a layer (Hereinafter, the layer is referred to as a "raw silk layer".) was formed on the outer peripheral surface of the first inner conductor 11A to obtain a first piezoelectric precursor. The raw silk layer covered the entire outer peripheral surface of the first inner conductor 11A. That is, the outer peripheral surface of the first inner conductor 11A was not exposed.

Note that "left winding" indicates that the raw silk is wound in the left winding from the near side to the far side of the inner conductor when viewed from one end in the axial direction of the first inner conductor 11A (tinsel wire). The "spiral angle" indicates an angle formed by the length direction of the raw silk with respect to the axial direction of the first inner conductor 11A.

"201" (cyanoacrylate-based adhesive) manufactured by Toagosei Co., Ltd. was prepared as a convergence agent.

A convergence agent (cyanoacrylate-based adhesive) was dropped on the outer peripheral surface of the raw silk layer of the first piezoelectric precursor to impregnate the inside of the raw silk. Thereafter, an excess amount of the convergence agent was immediately wiped off the outer peripheral surface of the raw silk with a Kimwipe and left at room temperature to cure the convergence agent. Thus, the first piezoelectric body 12A was formed from the piezoelectric thread layer.

A 2 m first coaxial cable 20A with a jack connector was prepared. Parts of the piezoelectric layers at both ends of the first piezoelectric body 12A were removed to expose the first inner conductor 11A. In a state where the first inner conductor 11A was exposed, a female connector on a central axis side of the first coaxial cable 20A and an end part on a back side (see Fig. 12) of the first inner conductor 11A of the two-layer piezoelectric base material were soldered to obtain a two-layer piezoelectric base material.

A rolled copper foil ribbon having a rectangular cross section was prepared as an elongated conductor of the first outer conductor 13A. A width of the rolled copper foil ribbon was 0.3 mm. A thickness of the rolled copper foil ribbon was 30 $\mu$m.

The rolled copper foil ribbon was wound on the outer peripheral surface of the first piezoelectric body 12A right-handed without a gap so that the first piezoelectric body 12A was not exposed. Thus, the first outer conductor 13A disposed at the outer periphery of the first piezoelectric body 12A was formed.

An outer line of the female connector of the first coaxial cable 20A and an end part on the back side (see Fig. 12) of the first outer conductor 13A of the first piezoelectric sensor 10A were soldered.

As described above, the first piezoelectric sensor 10A having a length of 150 mm and electrically connected to the first coaxial cable 20A was manufactured.

Similarly to the first piezoelectric sensor 10A, the second piezoelectric sensor 10B having a length of 150 mm and electrically connected to the second coaxial cable 20B was manufactured. In the second piezoelectric sensor 10B, the winding direction of the raw silk is right winding, and the winding direction of the rolled copper foil ribbon is left winding.

The measurement device 100 was manufactured in the same manner as in Example 1 except that each of the first piezoelectric sensor 10A and the second piezoelectric sensor 10B having a length of 150 mm was placed on and stuck to the upper surface of the lower insulating layer 140 (Fig. 13), and the first outer conductor 13A of the first piezoelectric sensor 10A and the second outer conductor 13B of the second piezoelectric sensor 10B were laterally attached at a distance of about 1 mm from each other at the central part of the upper surface of the lower insulating layer 140 in the width direction (left-right direction in Fig. 13).

Hum noise and sensor sensitivity at 60 Hz were measured in the same manner as in Example 1 except that the first conductive wire 21A of the first coaxial cable 20A was connected to the first differential input terminal $V_{IN}^-$ of the instrumentation amplifier 30 via the buffer circuit using the operational amplifier and the 10 times amplification circuit in this order, and the second conductive wire 21B of the second coaxial cable 20B was connected to the second differential input terminal $V_{IN}^+$ of the instrumentation amplifier 30 via the buffer circuit using the operational amplifier and the 10 times amplification circuit in this order. The measurement results are shown in Table 2.

[Comparative Example 4]

A measurement device 100 was manufactured in a similar manner to Example 3 except that the first piezoelectric sensor 10A of Example 3 was not used (That is, only one second piezoelectric sensor 10B is used.), and a Kapton tape was attached for fixing between the support plate 110 (see Fig. 13) and the first double-sided tape 101 (see Fig. 13).

The first differential input terminal $V_{IN}^-$ of the instrumentation amplifier 30 was electrically connected to the metal housing (GND) accommodating the instrumentation amplifier 30.

In the same manner as in Example 3, hum noise and sensor sensitivity at 60 Hz were measured. The measurement results are shown in Table 1.

(Example 4)

As a sheet-shaped piezoelectric body, a PVDF tape processed into a tape shape by slitting a polarized sheet of PVDF into a width of about 0.6 mm was prepared.

The polarized sheet of PVDF is a "piezo film sheet" (thickness: 50 μm) manufactured by Waki Laboratories Co., Ltd.

Whether or not the PVDF tape had the piezoelectric constant $d_{33}$ and the piezoelectric constant dsi was determined by the second determination method described above, and it was found that the PVDF tape had the piezoelectric constant $d_{33}$ and the piezoelectric constant dsi.

As the first inner conductor 11A, the first outer conductor 13A, the second inner conductor 11B, and the second outer conductor 13B, a material similar to that of Example 1 was prepared except that the lengths of the first inner conductor 11A and the second outer conductor 13B were 150 mm.

A first piezoelectric sensor 10A having a length of 150 mm was manufactured in the same manner as in Example 1 except that a PVDF tape was used instead of the slit ribbon and a winding method of the PVDF tape was different. A second piezoelectric sensor 10B having a length of 150 mm was manufactured in the same manner as the method for manufacturing the first piezoelectric sensor 10A.

Here, a piezoelectric body $P^+$ (first piezoelectric body 12A) and a piezoelectric body $P^-$ (first piezoelectric body 12B) were produced so as to generate voltages having opposite characteristics when the first piezoelectric sensor 10A and the second piezoelectric sensor 10B were pressurized (That is, when the PVDF tape is wound, the polarization direction of the PVDF tape is reversed.).

Specifically, the PVDF tape was wound (S-wound) right-handed on the outer peripheral surface of the first inner conductor 11A such that the side on which a positive voltage was generated when the first piezoelectric sensor 10A was pressurized was on the inner side (first inner conductor 11A side) to form the piezoelectric body $P^+$ (first piezoelectric body 12A). The second piezoelectric sensor 10B was wound (S-wound) right-handed so that the side on which a positive voltage was generated when the second piezoelectric sensor 10B was pressurized in the same manner as described above was the outer side (the side opposite to the second inner conductor 11B side) to form a piezoelectric body P' (second piezoelectric body 12B).

In the same manner as in Example 3, hum noise and sensor sensitivity at 60 Hz were measured. The measurement results are shown in Table 2.

[Comparative Example 5]

A measurement device 100 was manufactured in a similar manner to Example 4 except that the first piezoelectric sensor 10A of Example 4 was not used (That is, only one second piezoelectric sensor 10B of Example 4 was used.), and a Kapton tape was attached between the support plate 110 (see Fig. 13) and the first double-sided tape 101 (see Fig. 13).

The first differential input terminal VIN- of the instrumentation amplifier 30 was electrically connected to the metal housing (GND) accommodating the instrumentation amplifier 30.

In the same manner as in Example 3, hum noise and sensor sensitivity at 60 Hz were measured. The measurement results are shown in Table 2.

[Table 1]

| | First piezoelectric sensor | | | | | Second piezoelectric sensor | | | | | Piezoelectric device | | Measurement results | |
|---|---|---|---|---|---|---|---|---|---|---|---|---|---|---|
| | Length | First piezoelectric body | | Connection point | | Length | Second piezoelectric body | | Connection point | | Positive/negative relationship between first voltage and second voltage | Sensor interval | Hum noise | Sensor sensitivity |
| | (mm) | Material | Winding direction | First inner conductor | First outer conductor | (mm) | Material | Winding direction | Second inner conductor | Second outer conductor | | (mm) | (V) | (V) |
| Example 1 | 800 | Organic piezoelectric film | Left winding | First differential input terminal | Metal housing (GND) | 800 | Organic piezoelectric film | Right winding | Second differential input terminal | Metal housing (GND) | Different | 0 | 0.027 | 0.021 |
| Example 2 | 800 | Organic piezoelectric film | Left winding | First differential input terminal | Metal housing (GND) | 800 | Organic piezoelectric film | Right winding | Second differential input terminal | Metal housing (GND) | Different | 2 | 0.030 | 0.019 |
| Comparative Example 1 | 800 | Organic piezoelectric film | Right winding | First differential input terminal | Metal housing (GND) | 800 | Organic piezoelectric film | Right winding | Second differential input terminal | Metal housing (GND) | Identical | 0 | 0.028 | 0.002 |
| Comparative Example 2 | 800 | Organic piezoelectric film | Left winding | First differential input terminal | Metal housing (GND) | 800 | Organic piezoelectric film | Right winding | Metal housing (GND) | Metal housing (GND) | Different | 0 | 0.710 | 0.006 |
| Comparative Example 3 | 800 | Organic piezoelectric film | Left winding | Metal housing (GND) | First differential input terminal | 800 | Organic piezoelectric film | Left winding | Second differential input terminal | Metal housing (GND) | Identical | 0 | 3.300 | 0.021 |

[Table 2]

| | First piezoelectric sensor | | | | | Second piezoelectric sensor | | | | | Piezoelectric device | | Measurement results (10 times amplification circuit used) | |
|---|---|---|---|---|---|---|---|---|---|---|---|---|---|---|
| | Length | First piezoelectric body | | Connection point | | Length | Second piezoelectric body | | Connection point | | Positive/negative relationship between first voltage and second voltage | Sensor interval | Hum noise | Sensor sensitivity |
| | (mm) | Material | Winding direction | First inner conductor | First outer conductor | (mm) | Material | Winding direction | Second inner conductor | Second outer conductor | | (mm) | (V) | (V) |
| Example 3 | 150 | Raw silk Silk | Left winding | First differential input terminal | Metal housing (GND) | 150 | Raw silk Silk | Right winding | Second differential input terminal | Metal housing (GND) | Different | 1 | 0.003 | 0.104 |
| Comparative Example 4 | - | - | - | - | - | 150 | Raw silk Silk | Right winding | Second differential input terminal | Metal housing (GND) | - | - | 0.009 | 0.061 |
| Example 4 | 150 | PVDF | - | First differential input terminal | Metal housing (GND) | 150 | PVDF | - | Second differential input terminal | Metal housing (GND) | Different | 1 | 0.012 | 0.124 |
| Comparative Example 5 | - | - | - | - | - | 150 | PVDF | - | Second differential input terminal | Metal housing (GND) | - | - | 0.035 | 0.064 |

The lengths of the sensors of Example 1, Example 2, and Comparative Examples 1 to 3 were 800 mm as shown in Table 1. The lengths of the sensors of Example 3, Example 4, Comparative Example 4, and Comparative Example 5 were 200 mm as shown in Table 2.

The piezoelectric device of Comparative Example 1 includes the first piezoelectric sensor 10A, the second piezoelectric sensor 10B, and the instrumentation amplifier 30. The first inner conductor 11A was electrically connected to the first differential input terminal $V_{IN}^-$ of the instrumentation amplifier 30. The second inner conductor 11B is electrically connected to the second differential input terminal $V_{IN}^+$ of the instrumentation amplifier 30.

However, the polarity of the second voltage are the same as those of the first voltage. Therefore, as shown in Table 1, the sensor sensitivity was low.

From the above, it has been found that the piezoelectric device of Comparative Example 1 cannot detect an external force with high sensitivity.

The piezoelectric device of Comparative Example 2 includes the first piezoelectric sensor 10A, the second piezoelectric sensor 10B, and the instrumentation amplifier 30. The positive or negative of the second voltage is different from that of the first voltage. The first inner conductor 11A was electrically connected to the first differential input terminal $V_{IN}^-$ of the instrumentation amplifier 30.

However, in Comparative Example 2, the second inner conductor 11B was electrically connected to the metal housing (GND). Therefore, as shown in Table 1, the sensor sensitivity was low, and the hum noise of 60 Hz was very high.

From the above, it has been found that the piezoelectric device of Comparative Example 2 cannot detect an external force with high sensitivity.

The piezoelectric device of Comparative Example 3 includes the first piezoelectric sensor 10A, the second piezoelectric sensor 10B, and the instrumentation amplifier 30. The positive or negative of the second voltage is different from that of the first voltage. The second inner conductor 11B is electrically connected to the second differential input terminal $V_{IN}^+$ of the instrumentation amplifier 30.

However, in Comparative Example 3, the first inner conductor 11A was electrically connected to the metal housing (GND). Therefore, as shown in Table 1, the hum noise of 60 Hz was very high.

From the above, it has been found that the piezoelectric device of Comparative Example 3 cannot detect an external force with high sensitivity.

On the other hand, the piezoelectric devices of Examples 1 and 2 include the first piezoelectric sensor 10A, the second piezoelectric sensor 10B, and the instrumentation amplifier 30. The positive or negative of the second voltage is different from that of the first voltage. The first inner conductor 11A was electrically connected to the first differential input terminal $V_{IN}^-$ of the instrumentation amplifier 30. The second inner conductor 11B is electrically connected to the second differential input terminal $V_{IN}^+$ of the instrumentation amplifier 30.

Therefore, as shown in Table 1, the level of hum noise at 60 Hz was lower than that in Comparative Example 2 and Comparative Example 3. That is, it was possible to confirm the effect that the piezoelectric devices of Examples 1 and 2 can reduce external electromagnetic noise.

Further, by differentially amplifying the output voltages of the first piezoelectric sensor 10A and the second piezoelectric sensor 10B, the external electromagnetic noise was reduced, and the sensor sensitivity was higher than that of Comparative Example 1 and Comparative Example 2. That is, it was found that the piezoelectric devices of Examples 1 and 2 can perform sensing with a high SN ratio with high sensitivity and low noise.

From the above, it was found that the piezoelectric devices of Examples 1 and 2 can detect an external force with high sensitivity.

The piezoelectric device of Comparative Example 4 includes the second piezoelectric sensor 10B and the instrumentation amplifier 30. The second inner conductor 11B is electrically connected to the second differential input terminal $V_{IN}^+$ of the instrumentation amplifier 30.

However, in Comparative Example 4, the piezoelectric device did not include the first piezoelectric sensor 10A. Therefore, as shown in Table 2, the sensor sensitivity was low, and the hum noise at 60 Hz was high.

From the above, it has been found that the piezoelectric device of Comparative Example 4 cannot detect an external force with high sensitivity.

On the other hand, the piezoelectric device of Example 3 includes the first piezoelectric sensor 10A, the second piezoelectric sensor 10B, and the instrumentation amplifier 30. The positive or negative of the second voltage is different from that of the first voltage. The first inner conductor 11A was electrically connected to the first differential input terminal $V_{IN}^-$ of the instrumentation amplifier 30. The second inner conductor 11B is electrically connected to the second differential input terminal $V_{IN}^+$ of the instrumentation amplifier 30.

Therefore, as shown in Table 2, the level of hum noise at 60 Hz was lower than that in Comparative Example 4, and the sensor sensitivity was higher than that in Comparative Example 4.

From the above, it was found that the piezoelectric device of Example 3 can detect an external force with high sensitivity.

The piezoelectric device of Comparative Example 5 includes the second piezoelectric sensor 10B and the

instrumentation amplifier 30. The second inner conductor 11B is electrically connected to the second differential input terminal $V_{IN}^+$ of the instrumentation amplifier 30.

However, in Comparative Example 5, the piezoelectric device did not include the first piezoelectric sensor 10A. Therefore, as shown in Table 2, the sensor sensitivity was low, and the hum noise at 60 Hz was high.

From the above, it has been found that the piezoelectric device of Comparative Example 5 cannot detect an external force with high sensitivity.

On the other hand, the piezoelectric device of Example 4 includes the first piezoelectric sensor 10A, the second piezoelectric sensor 10B, and the instrumentation amplifier 30. The positive or negative of the second voltage is different from that of the first voltage. The first inner conductor 11A was electrically connected to the first differential input terminal $V_{IN}^-$ of the instrumentation amplifier 30. The second inner conductor 11B is electrically connected to the second differential input terminal $V_{IN}^+$ of the instrumentation amplifier 30.

Therefore, as shown in Table 2, the level of hum noise at 60 Hz was lower than that in Comparative Example 5, and the sensor sensitivity was higher than that in Comparative Example 5.

From the above, it was found that the piezoelectric device of Example 4 can detect an external force with high sensitivity.

The disclosure of Japanese Patent Application No. 2020-161350 filed on September 25, 2020 is incorporated herein by reference in its entirety.

All documents, patent applications, and technical standards described in this specification are incorporated herein by reference to the same extent as if each document, patent application, and technical standard were specifically and individually indicated to be incorporated by reference.

## Claims

**1.** A piezoelectric device comprising:

a first piezoelectric sensor including a first inner conductor extending in a first direction, a first piezoelectric body covering at least a part of the first inner conductor, and a first outer conductor disposed at an outer periphery of the first piezoelectric body, the first piezoelectric sensor generating a first voltage in the first inner conductor with respect to the first outer conductor according to displacement of the first piezoelectric body due to an external force when the external force acts on the first piezoelectric body;

a second piezoelectric sensor including a second inner conductor extending in a second direction, a second piezoelectric body covering at least a part of the second inner conductor, and a second outer conductor disposed at an outer periphery of the second piezoelectric body, the second piezoelectric sensor generating a second voltage having a voltage different in polarity from the first voltage in the second inner conductor with respect to the second outer conductor according to displacement of the second piezoelectric body due to the external force when the external force acts on the second piezoelectric body; and

a differential signal forming unit including one differential input terminal to which the first inner conductor is electrically connected and another differential input terminal to which the second inner conductor is electrically connected, the differential signal forming unit forming a differential signal based on a first signal input to the one differential input terminal and a second signal input to the other differential input terminal.

**2.** The piezoelectric device according to claim 1, wherein the differential signal forming unit includes a differential amplifier circuit.

**3.** The piezoelectric device according to claim 1 or 2, wherein the first direction and the second direction are substantially parallel.

**4.** The piezoelectric device according to claim 3, wherein the first outer conductor and the second outer conductor are in physical contact with each other.

**5.** The piezoelectric device according to any one of claims 1 to 4, wherein:

the first piezoelectric body is formed by spirally winding an elongated organic piezoelectric body including an organic piezoelectric material having a piezoelectric constant $d_{14}$ in a first spiral direction toward the first direction, and

the second piezoelectric body is formed by spirally winding the elongated organic piezoelectric body in a second spiral direction different from the first spiral direction toward the second direction.

6. The piezoelectric device according to claim 5, wherein the organic piezoelectric material contains a helical chiral polymer having optical activity.

7. The piezoelectric device according to claim 6, wherein the helical chiral polymer contains polylactic acid.

8. The piezoelectric device according to claim 5, wherein the organic piezoelectric material contains long fibers composed of an optically active polypeptide.

9. The piezoelectric device according to any one of claims 1 to 4, wherein:

   the first piezoelectric body is formed by winding a sheet-shaped piezoelectric body containing a piezoelectric material having a piezoelectric constant $d_{33}$ and a piezoelectric constant $d_{31}$ and not having a piezoelectric constant $d_{14}$ such that one surface of the sheet-shaped piezoelectric body is on a side of the first inner conductor, and
   the second piezoelectric body is formed by winding the sheet-shaped piezoelectric body such that another surface of the sheet-shaped piezoelectric body is on a side of the second inner conductor.

10. The piezoelectric device according to claim 9, wherein the sheet-shaped piezoelectric body contains polyvinylidene fluoride.

11. A piezoelectric device comprising:

   a first piezoelectric sensor including a first inner conductor extending in a first direction, a first piezoelectric body covering at least a part of the first inner conductor, and a first outer conductor disposed at an outer periphery of the first piezoelectric body, the first piezoelectric sensor generating a first voltage in the first inner conductor with respect to the first outer conductor according to displacement of the first piezoelectric body due to an external force when the external force acts on the first piezoelectric body;
   a second piezoelectric sensor including a second inner conductor extending in a second direction, a second piezoelectric body covering at least a part of the second inner conductor, and a second outer conductor disposed at an outer periphery of the second piezoelectric body, the second piezoelectric sensor generating a second voltage having a voltage different in polarity from the first voltage in the second inner conductor with respect to the second outer conductor according to displacement of the second piezoelectric body due to the external force when the external force acts on the second piezoelectric body; and
   an instrumentation amplifier including one differential input terminal to which the first inner conductor is electrically connected, another differential input terminal to which the second inner conductor is electrically connected, and a reference terminal electrically connected to each of the first outer conductor and the second outer conductor.

12. A force sensor comprising the piezoelectric device according to any one of claims 1 to 11.

13. A biological information acquisition device comprising the piezoelectric device according to any one of claims 1 to 11.

FIG.1

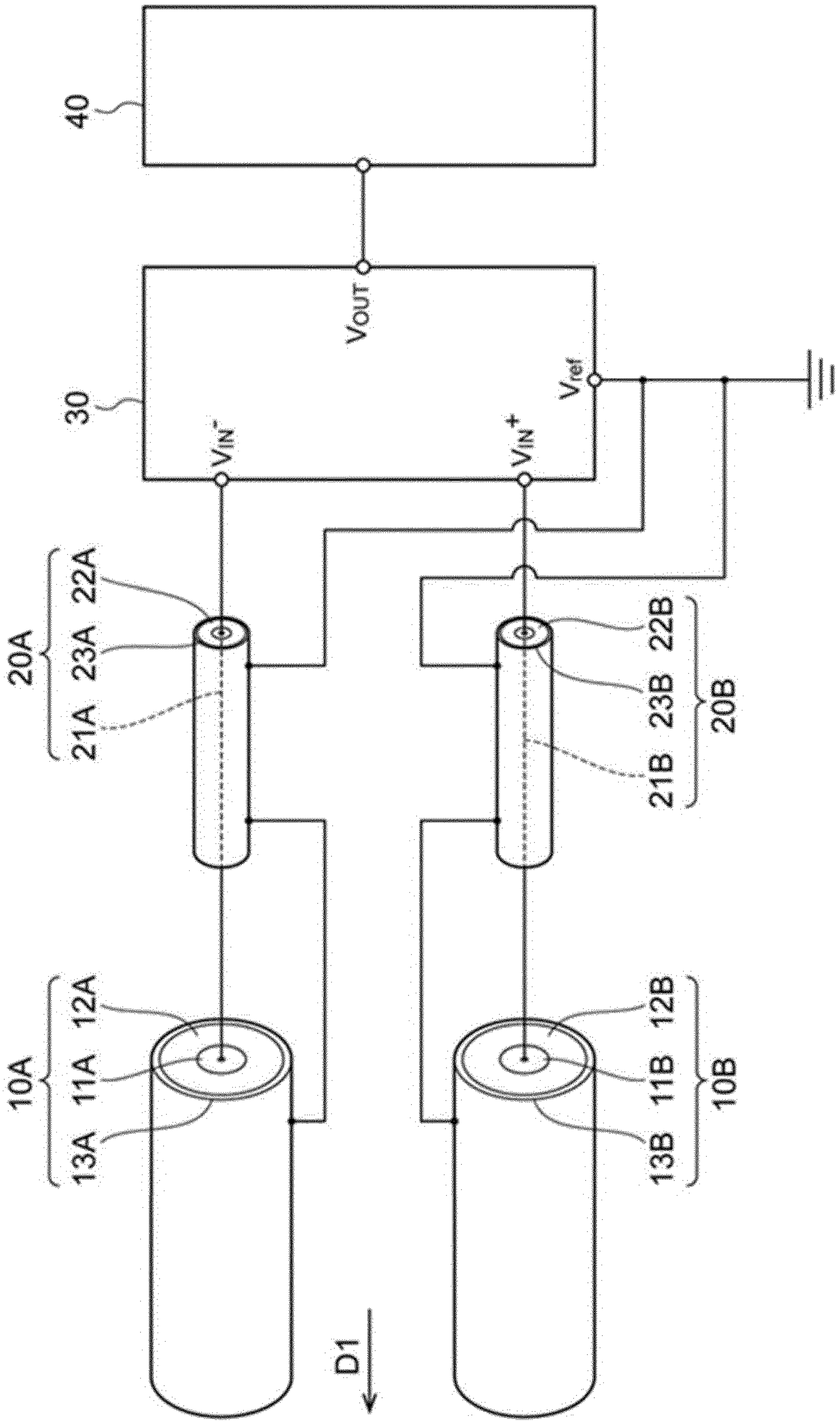

40
30
VOUT
VIN-
VIN+
Vref
20A
22A
23A
21A
20B
22B
23B
21B
10A
11A
12A
13A
10B
11B
12B
13B
D1

FIG.2

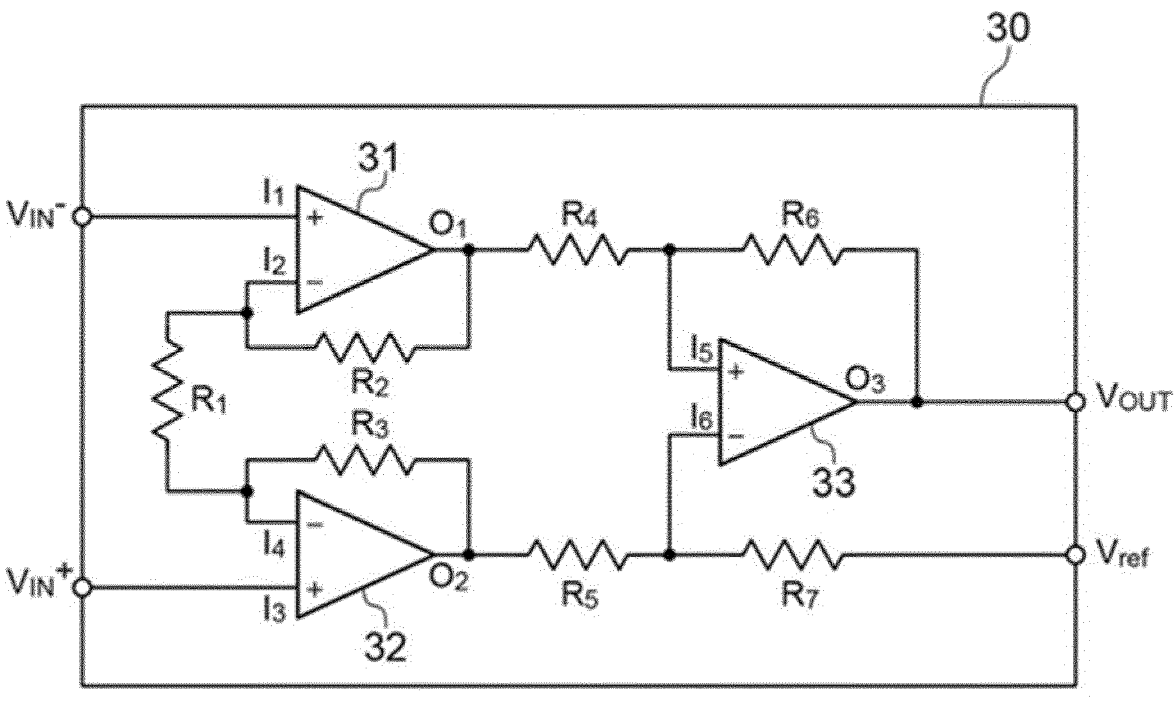
30
31
32
33
VIN-
VIN+
I1
I2
I3
I4
I5
I6
O1
O2
O3
R1
R2
R3
R4
R5
R6
R7
VOUT
Vref

FIG.3

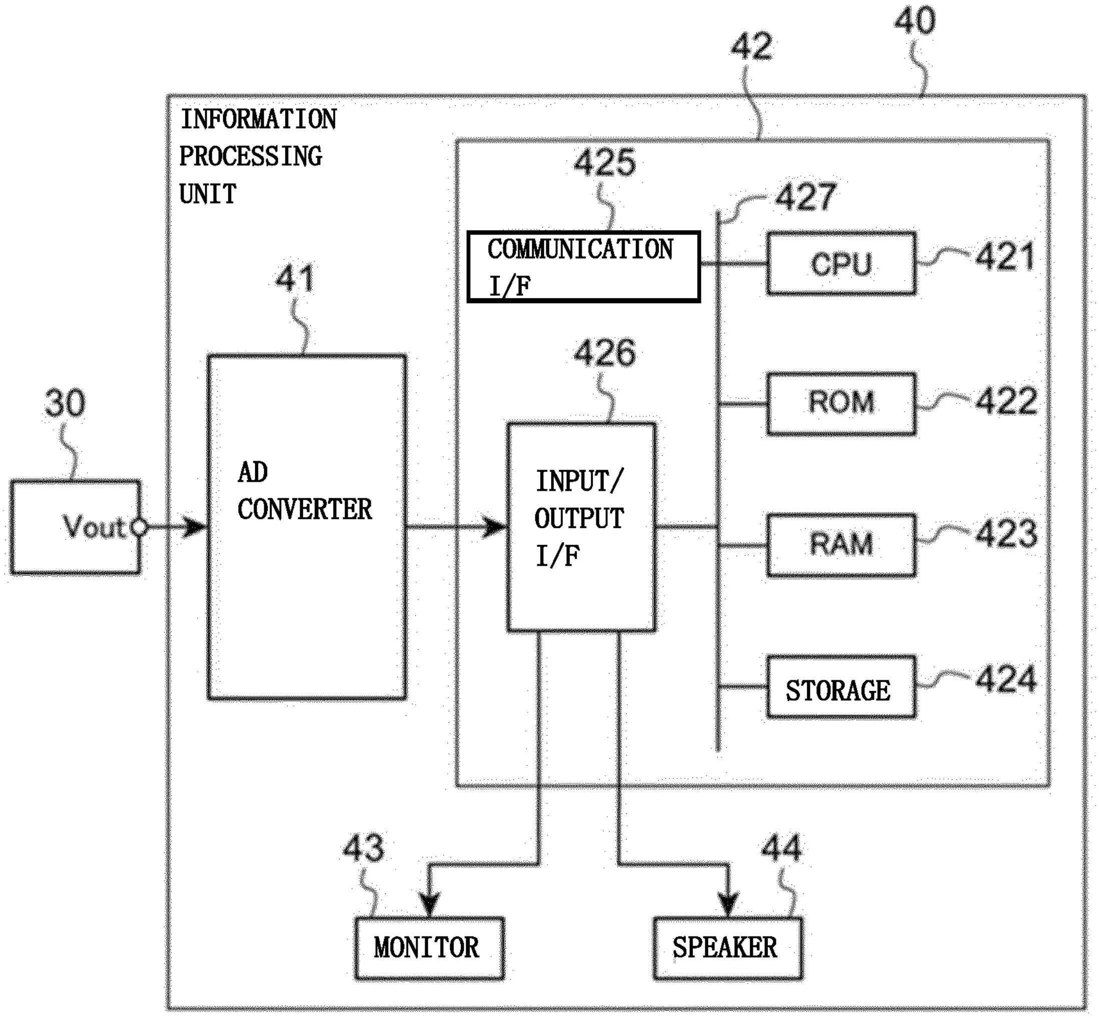
40
42
INFORMATION PROCESSING UNIT
425
427
COMMUNICATION I/F
CPU
421
41
426
ROM
422
30
AD CONVERTER
Vout
INPUT/ OUTPUT I/F
RAM
423
STORAGE
424
43
44
MONITOR
SPEAKER

FIG.4

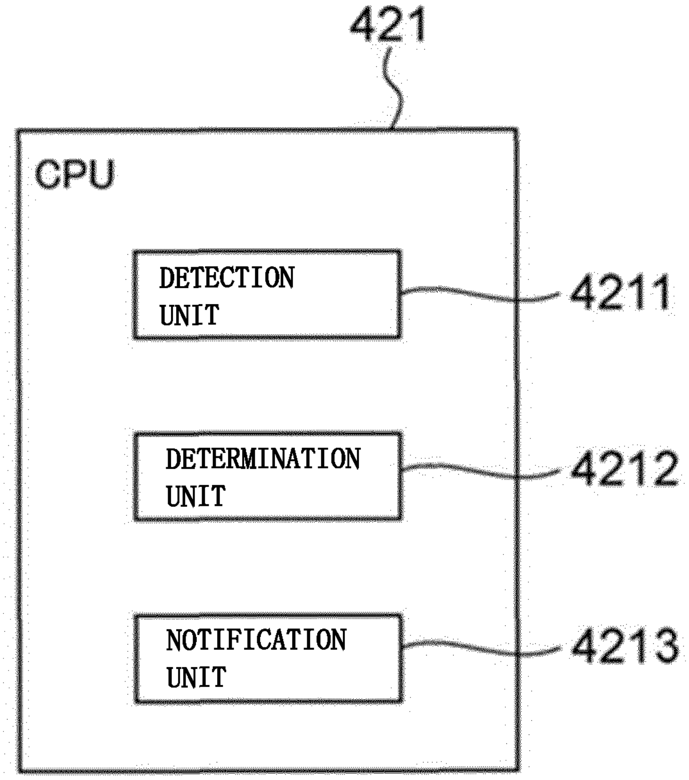
421
CPU
DETECTION UNIT
4211
DETERMINATION UNIT
4212
NOTIFICATION UNIT
4213

FIG.5

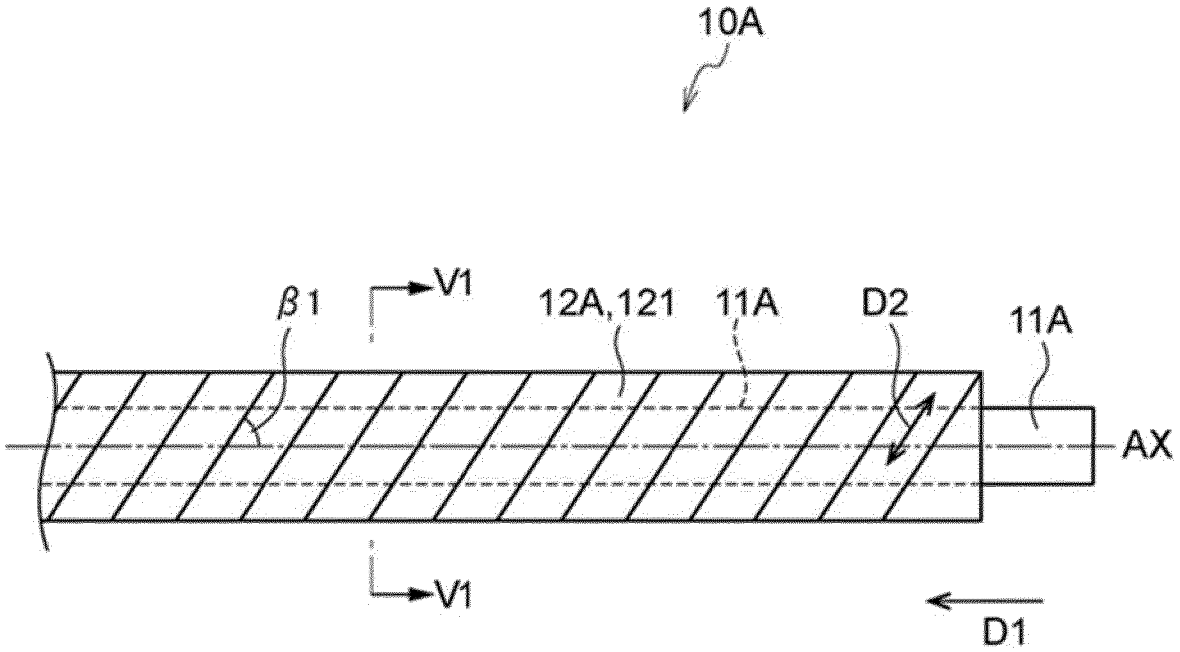


FIG.6

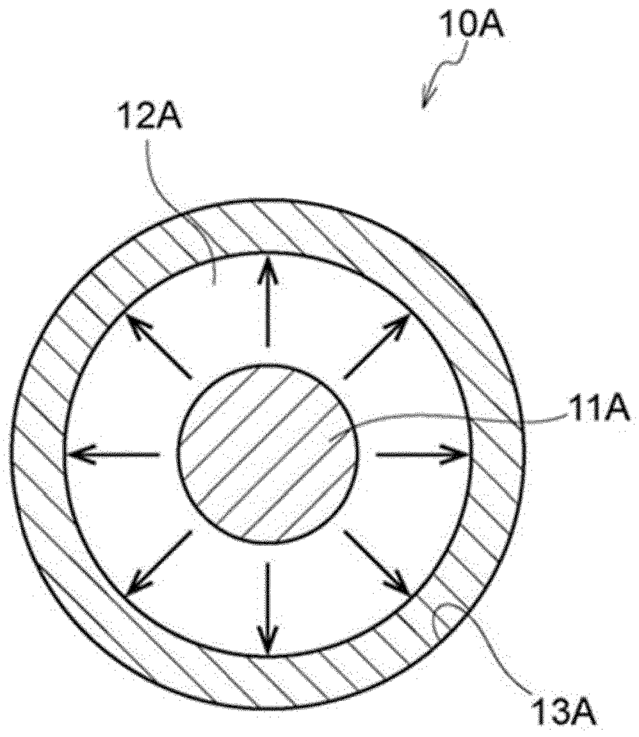

FIG.7

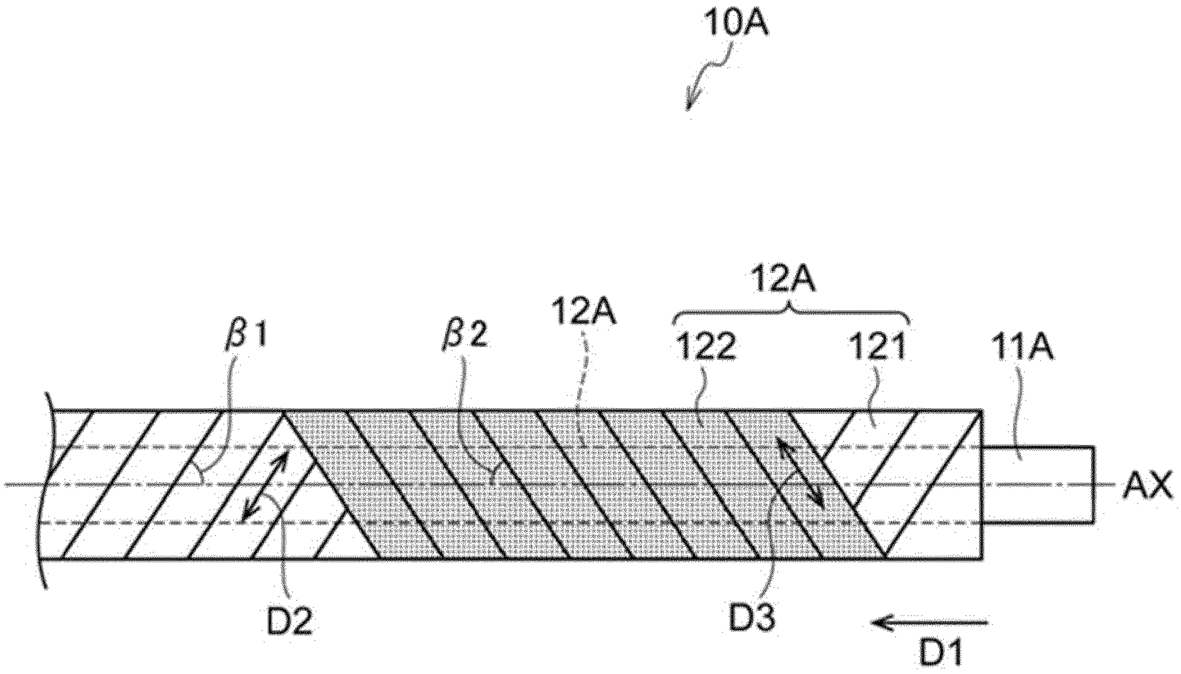
10A
12A
β1
β2
12A
122
121
11A
AX
D2
D3
D1

FIG.8

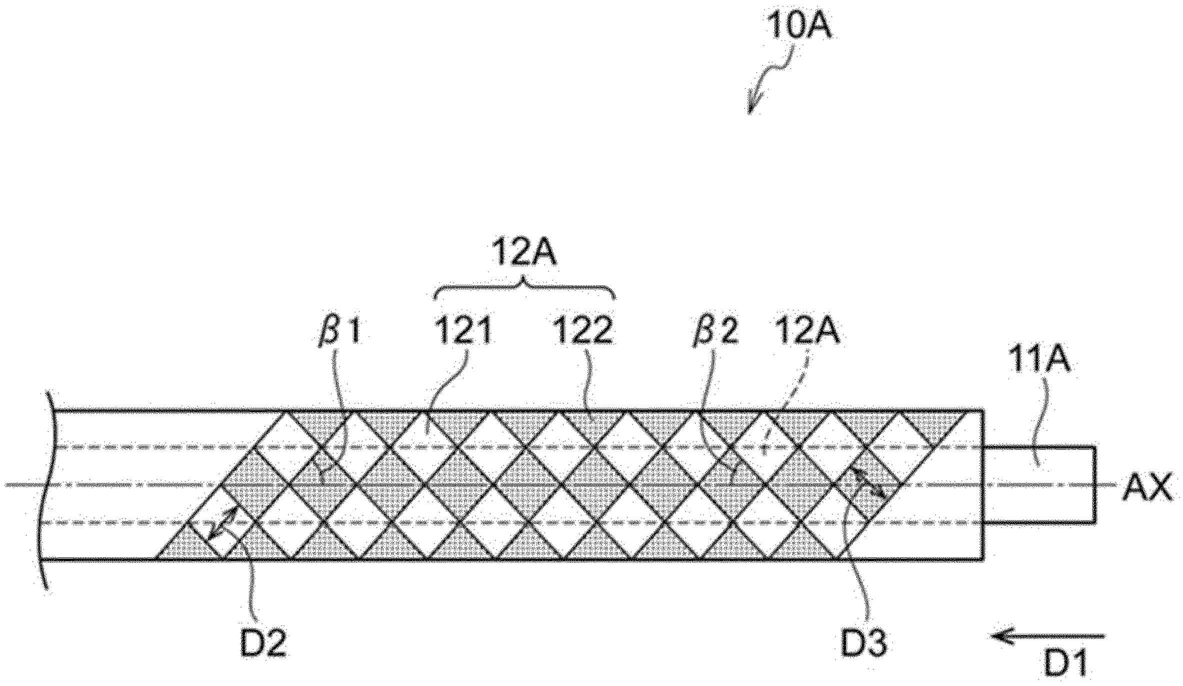
10A
12A
β1
121
122
β2
12A
11A
AX
D2
D3
D1

FIG.9

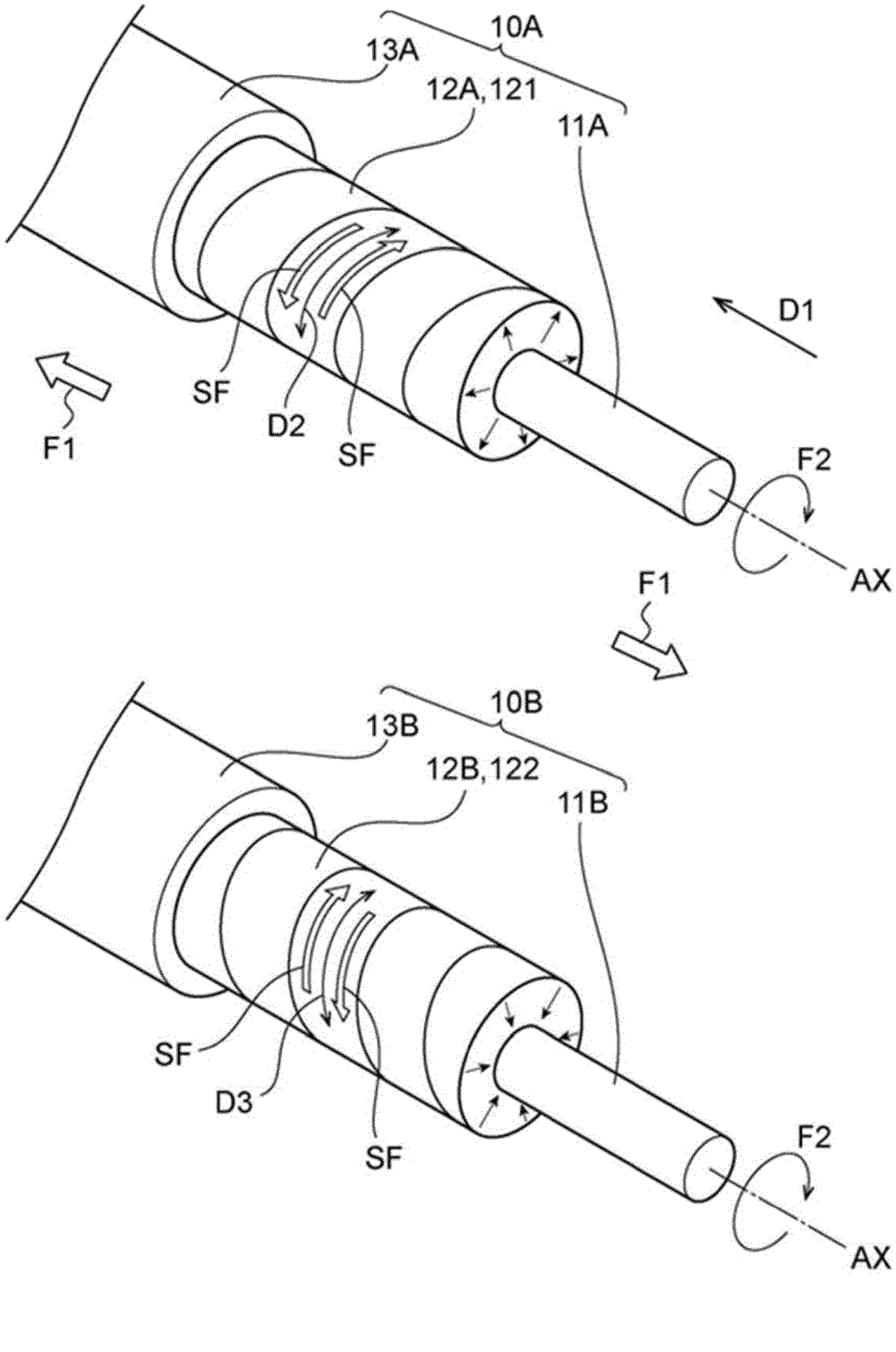
10A
13A
12A,121
11A
D1
SF
D2
SF
F1
F2
AX
F1
10B
13B
12B,122
11B
SF
D3
SF
F2
AX

FIG.10

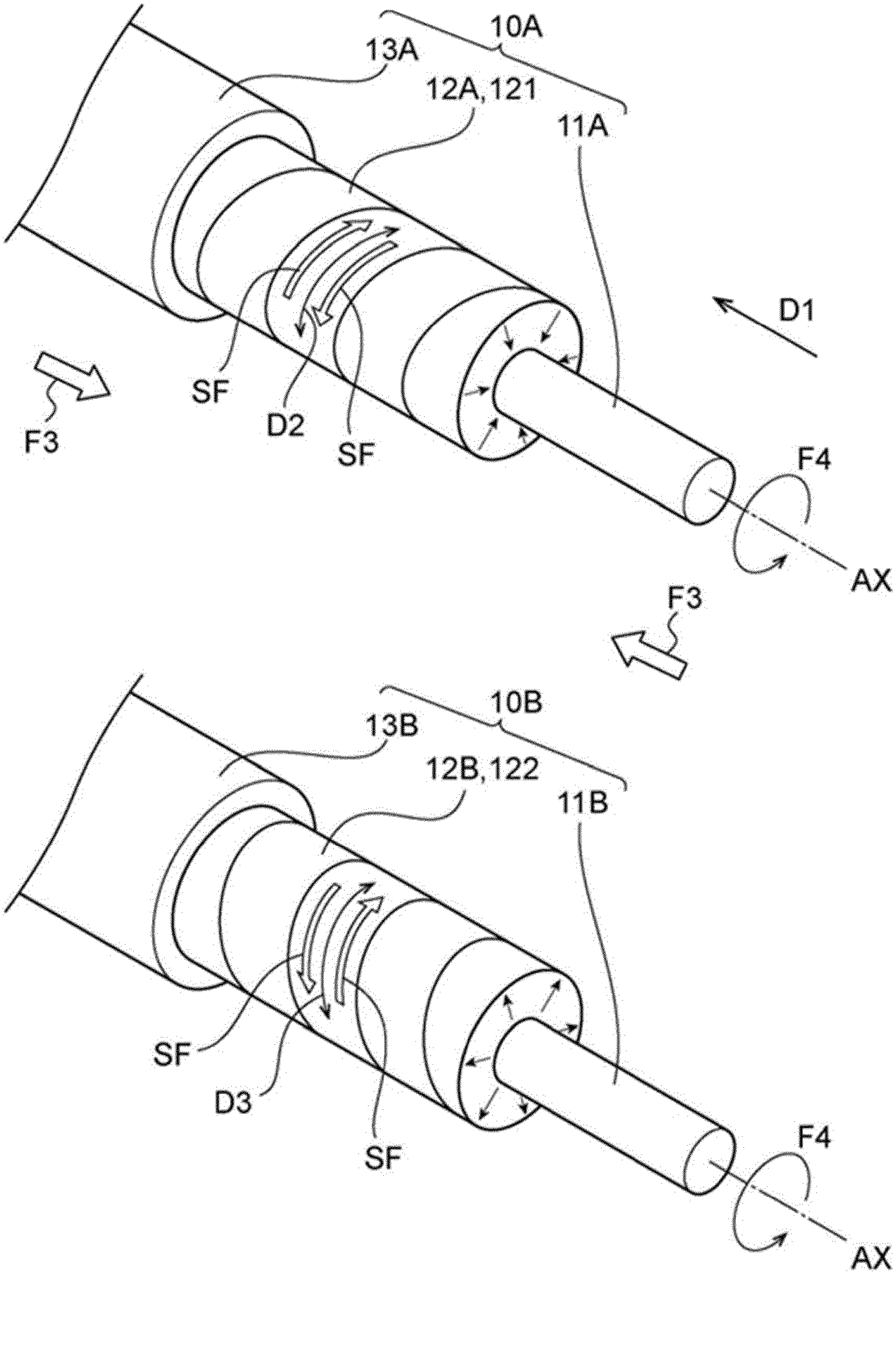
10A
13A
12A,121
11A
D1
SF
D2
SF
F3
F4
AX
F3
10B
13B
12B,122
11B
SF
D3
SF
F4
AX

FIG.11

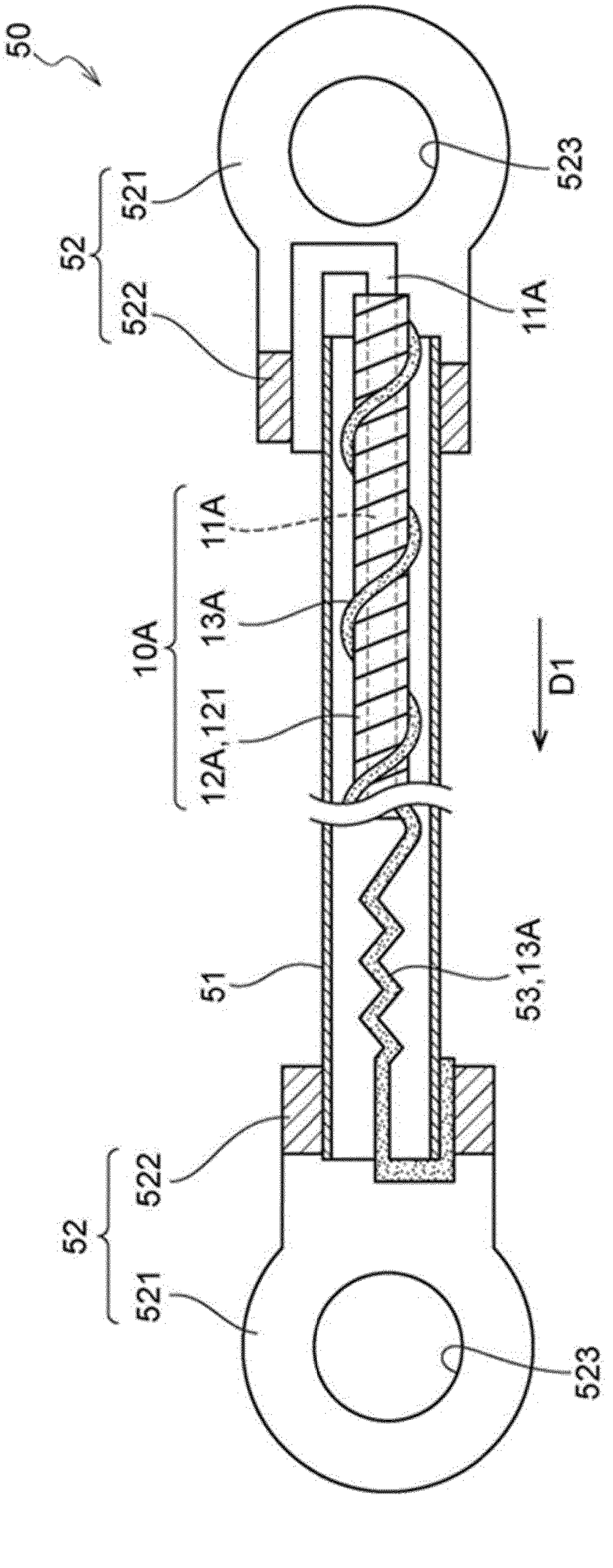
50
52
521
522
523
11A
10A
11A
13A
12A,121
D1
51
53,13A
52
522
521
523

FIG.12

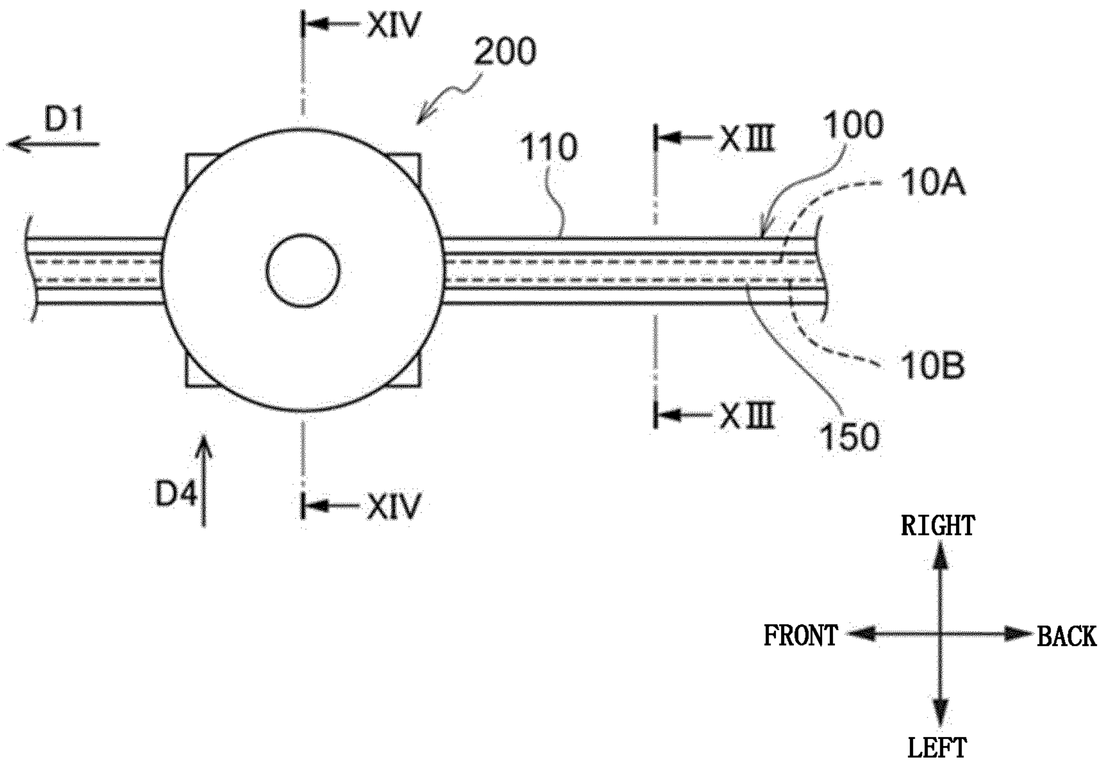
XIV
200
D1
110
XIII
100
10A
10B
XIII
150
D4
XIV
RIGHT
FRONT
BACK
LEFT

FIG.13

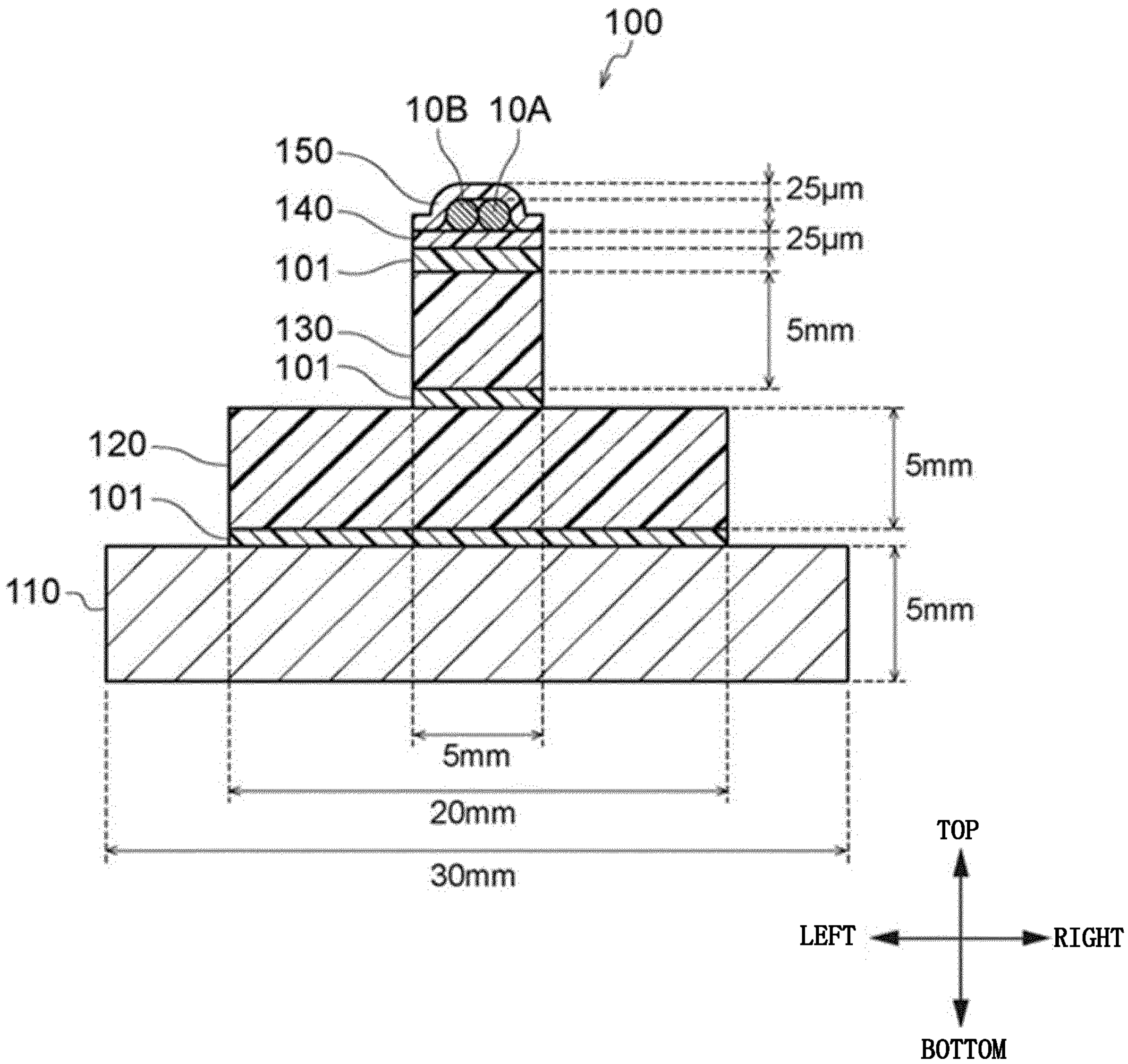
100
10B 10A
150
140
101
130
101
120
101
110
25μm
25μm
5mm
5mm
5mm
5mm
20mm
30mm
TOP
LEFT
RIGHT
BOTTOM

FIG.14

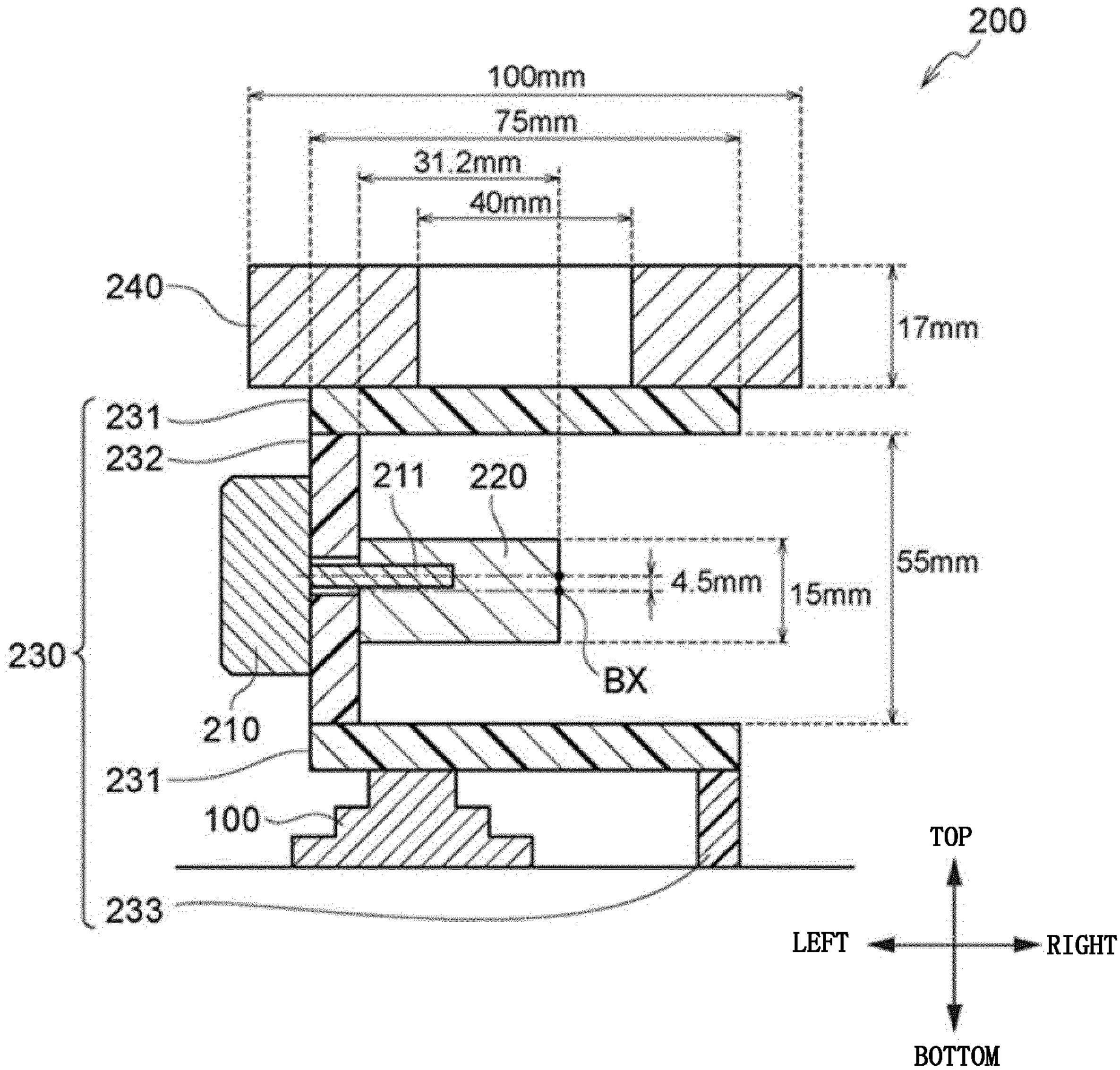
200
100mm
75mm
31.2mm
40mm
240
17mm
231
232
211
220
55mm
4.5mm
15mm
230
BX
210
231
100
233
TOP
LEFT
RIGHT
BOTTOM

FIG.15

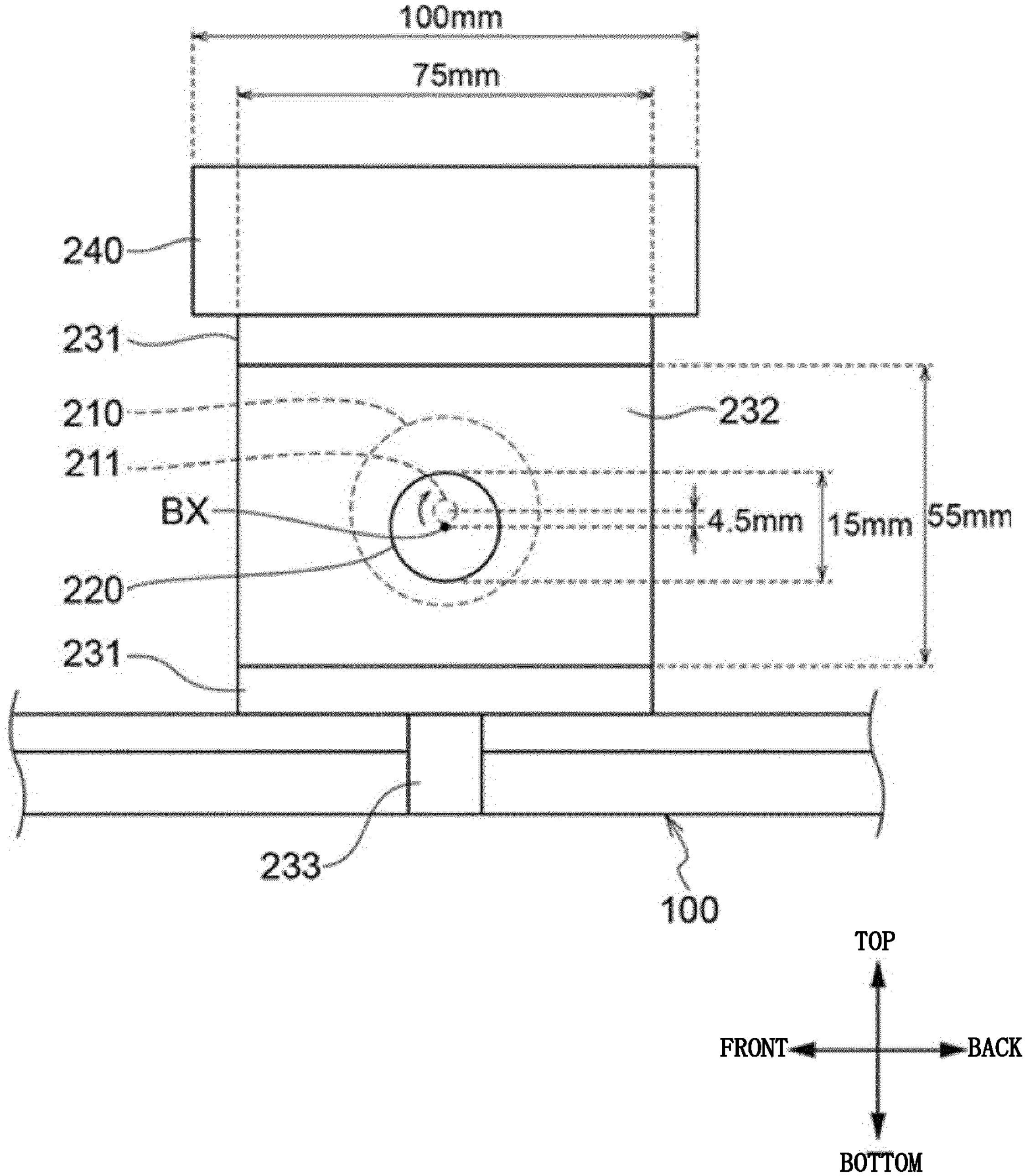
100mm
75mm
240
231
210
211
BX
220
231
232
4.5mm
15mm
55mm
233
100
TOP
FRONT
BACK
BOTTOM

FIG.16

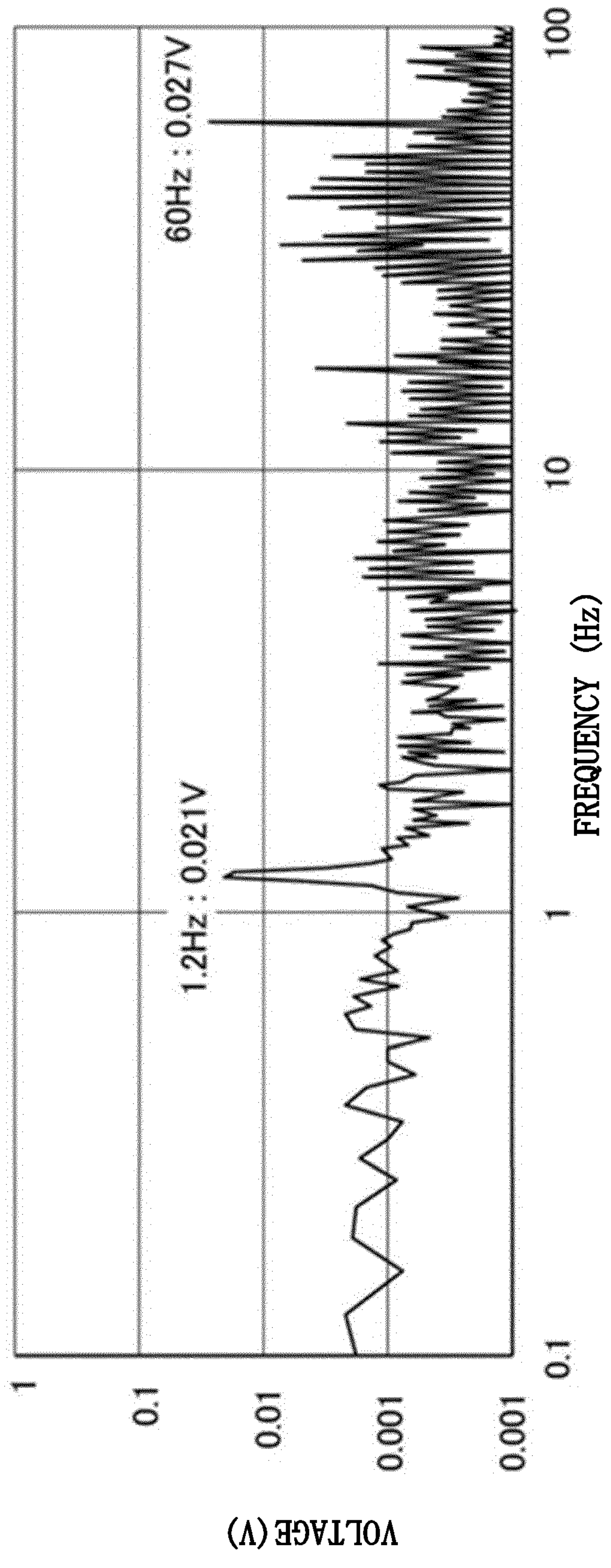
60Hz : 0.027V
1.2Hz : 0.021V
FREQUENCY (Hz)
VOLTAGE (V)
0.1
1
10
100
1
0.1
0.01
0.001
0.001

FIG.17

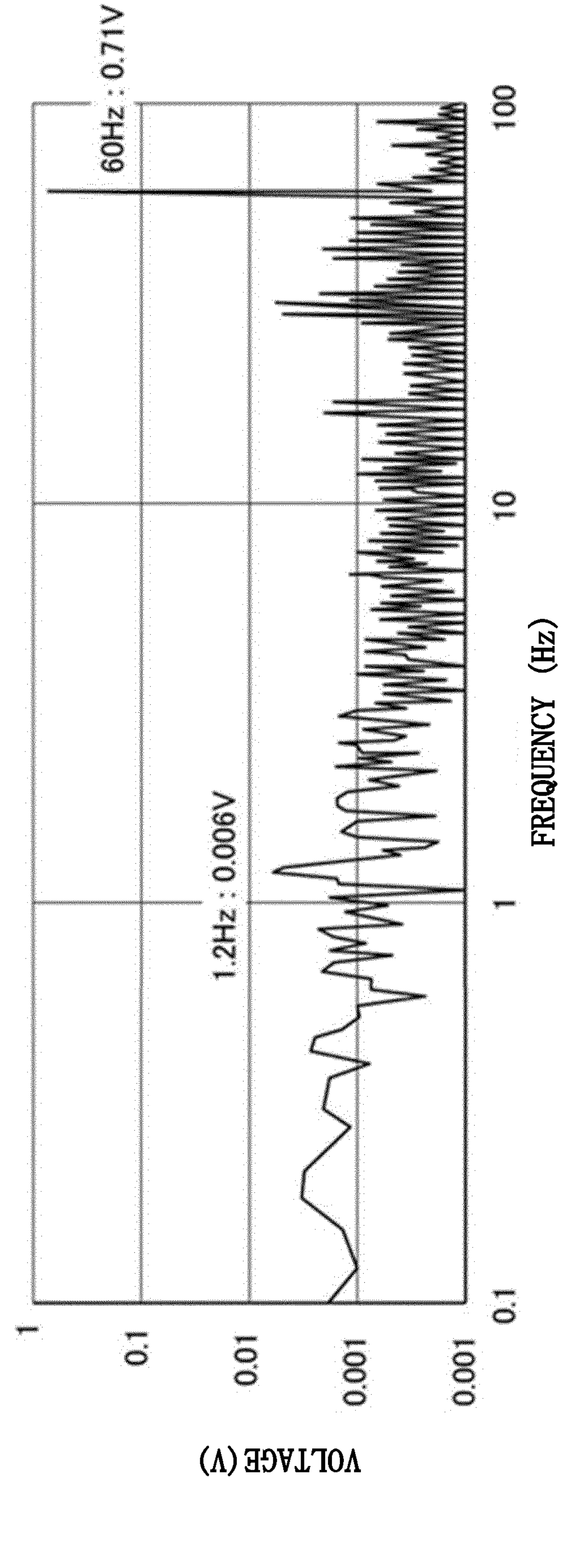
60Hz : 0.71V
1.2Hz : 0.006V
FREQUENCY (Hz)
VOLTAGE (V)
0.1
1
10
100
1
0.1
0.01
0.001
0.001

FIG.18

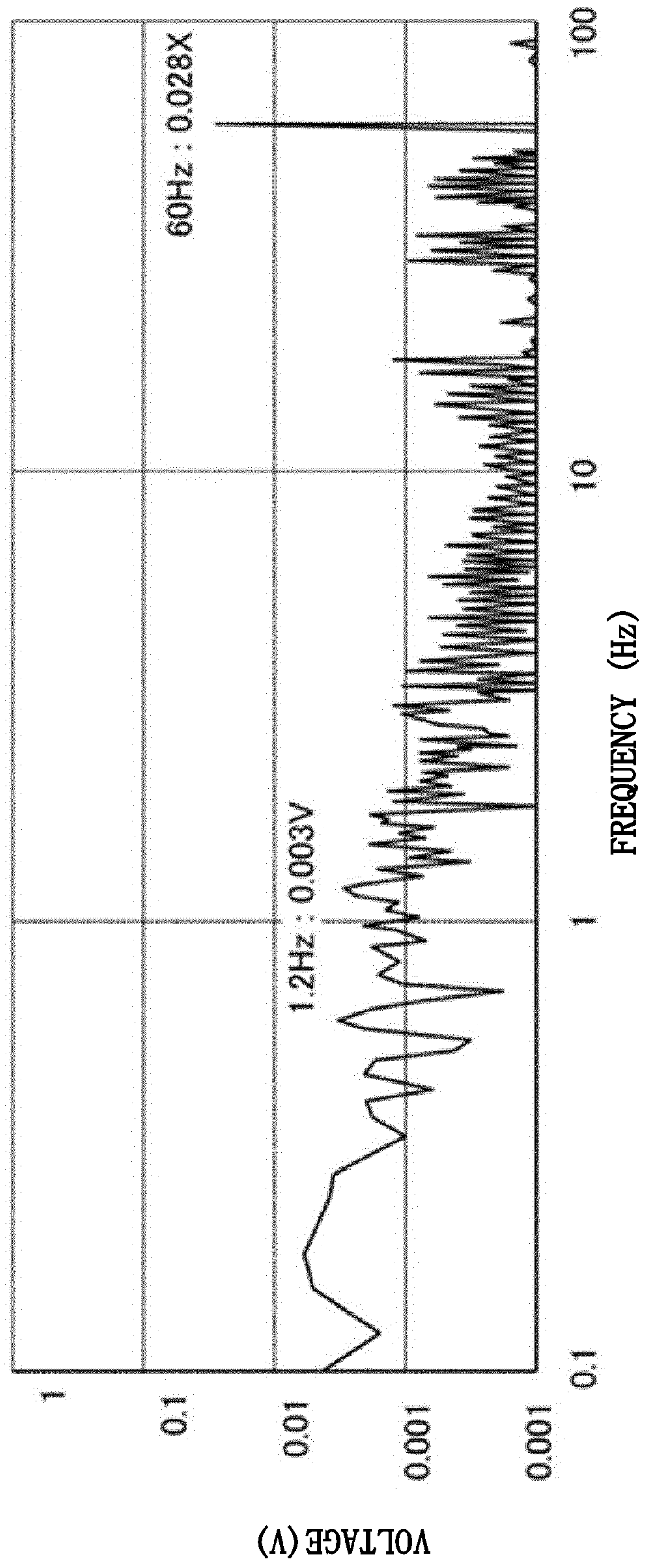
60Hz : 0.028X
1.2Hz : 0.003V
FREQUENCY (Hz)
VOLTAGE (V)
0.1
1
10
100
1
0.1
0.01
0.001
0.001

**INTERNATIONAL SEARCH REPORT**

International application No.
**PCT/JP2021/035189**

**A. CLASSIFICATION OF SUBJECT MATTER**

***G01L 1/16***(2006.01)i; ***H01L 41/087***(2006.01)i; ***H01L 41/113***(2006.01)i; ***H01L 41/193***(2006.01)i
FI: H01L41/113; H01L41/193; G01L1/16 G; H01L41/087

According to International Patent Classification (IPC) or to both national classification and IPC

**B. FIELDS SEARCHED**

Minimum documentation searched (classification system followed by classification symbols)

G01L1/16; H01L41/087; H01L41/113; H01L41/193

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 2019/126477 A2 (FISHMAN, Lawrence et al.) 27 June 2019 (2019-06-27) paragraphs [0052], [0057], [0059], [0074], [0078], fig. 2, 13, 15 | 1-4, 11 |
| Y | | 5-8, 12, 13 |
| A | | 9, 10 |
| Y | WO 2017/111108 A1 (MITSUI CHEMICALS, INC.) 29 June 2017 (2017-06-29) paragraphs [0016], [0050], [0073], [0168], [0171], fig. 2, 7 | 5-8, 12, 13 |
| A | | 1-4, 9-11 |
| A | JP 56-2800 A (THOMSON CSF) 13 January 1981 (1981-01-13) entire text, all drawings | 1-13 |

☐ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **02 November 2021** | **16 November 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**
**Information on patent family members**

International application No.
**PCT/JP2021/035189**

| Patent document cited in search report | Publication date (day/month/year) | Patent family member(s) | Publication date (day/month/year) |
|---|---|---|---|
| WO 2019/126477 A2 | 27 June 2019 | JP 2021-508941 A | |
| | | US 2020/0343438 A1 | |
| WO 2017/111108 A1 | 29 June 2017 | JP 2020-36027 A | |
| | | US 2019/0003905 A1 paragraphs [0134], [0238], [0309], [0598], [0613], fig. 2, 7 | |
| | | EP 3376549 A1 | |
| | | KR 10-2018-0082499 A | |
| | | CN 108701753 A | |
| JP 56-2800 A | 13 January 1981 | US 4369391 A entire text, all drawings | |
| | | EP 21879 A1 | |
| | | FR 2458909 A1 | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP H10132669 A
- JP 4934235 B
- WO 2010104196 A
- WO 2013054918 A
- WO 2013089148 A
- JP S59096123 A
- JP H07033861 A
- US 2668182 A
- US 2941956 A
- JP S4733279 B
- JP 4084953 B
- JP 2011256337 A
- JP 2020161350 A

### Non-patent literature cited in the description

- *J.0rg.Chem.,* 1963, vol. 28, 2069-2075
- *Chemical Review,* 1981, vol. 81 (4), 619-621